# EUROPEAN PATENT APPLICATION

(11) **EP 4 745 136 A1**
(43) Date of publication of application: **20.05.2026**
(21) Application number: 24838822.5
(22) Date of filing: 10.07.2024
(51) Int. Cl.: C07D 403/12, C07D 417/04, C07D 285/10, A61K 31/433, A61K 31/4439, A61P 35/00, A61P 3/00

(54) **PROTEIN TYROSINE PHOSPHATASE INHIBITOR, COMPOSITION COMPRISING SAME, AND MEDICAL USE THEREOF**

(30) Priority: 11.07.2023 CN 202310857115; 12.09.2023 CN 202311171624
(71) Applicant: Shenzhen Zhongge Biological Technology Co., Ltd., Futian District Shenzhen, Guangdong 518017 (CN)
(72) Inventor: MOU, Jianfeng, Shenzhen, Guangdong 518017 (CN); DUAN, Xinli, Shenzhen, Guangdong 518017 (CN); YU, Qingfang, Shenzhen, Guangdong 518017 (CN); WANG, Tong, Shenzhen, Guangdong 518017 (CN); DONG, Guangping, Shenzhen, Guangdong 518017 (CN); HU, Chenming, Shenzhen, Guangdong 518017 (CN); GU, Zhengsong, Shenzhen, Guangdong 518017 (CN); E, Jingwen, Shenzhen, Guangdong 518017 (CN); WANG, Shaohui, Shenzhen, Guangdong 518017 (CN); CHEN, Bin, Shenzhen, Guangdong 518017 (CN); ZHANG, Peiyu, Shenzhen, Guangdong 518017 (CN)
(74) Representative: Maiwald GmbH
(86) International application number: PCT/CN2024/104645
(87) International publication number: WO 2025/011570

(57) **Abstract**

The present invention provides a compound or an enantiomer, diastereomer, racemate, tautomer, stereoisomer, geometric isomer, nitrogen oxide, metabolite or pharmaceutically acceptable salt, ester, solvate, hydrate, isotope labeled compound or prodrug thereof, and also provides a composition containing the compound and a medical use. The compound has good pharmaceutical prospects in the aspect of treating or preventing diseases or disorders related to PTPN2.

## Description

### Cross-Reference to Related Applications

This application claims the priority to Chinese Patent Applications No. CN202310857115.1, filed on July 11, 2023, and No. CN202311171624.5, filed on September 12, 2023, the content of which is hereby incorporated by reference into this application in their entireties and for all purposes.

### Technical field

The present invention relates to the field of medicine, and specifically, to a protein tyrosine phosphatase inhibitor and a composition thereof, and pharmaceutical use.

### Background

Immune checkpoint inhibitors, represented by PD-1 antibodies, have changed the treatment of many cancers, but the majority of patients do not benefit from such PD-1 blocking therapies. This state of affairs urge some research teams to work on finding new immunotherapy targets to develop novel immunotherapeutic agents that can be used for cancer patients who do not respond to PD-1 antibodies, or therapies that can synergize with PD-1 antibodies to enhance their anti-cancer efficacy.

PTPN2, the full name of which is protein tyrosine phosphatase non-receptor type 2, also known as TC-PTP, is a member of the protein tyrosine phosphatase (PTP) family. PTPN2 possesses 74% sequence homology and 86% structural similarity to its cognate family protein, PTPN1 (also known as PTP1B). As signaling factors, members of this family participate in regulating a variety of signaling pathways and cellular processes, including cell growth, differentiation, mitotic cycle, and oncogenic transformation. The PTPN2 protein contains an N-terminal kinase domain and a C-terminal non-catalytic domain, and a nuclear localization signal (NLS) in the C-terminus participates in the autoregulation of catalytic activity and the determination of subtype localization. PTPN2 possesses two isoforms, TC45 and TC48, due to selective splicing, and the differences in the C-terminal region cause different subtypes to be located differently, thereby affecting their substrate selection. PTPN2 negatively regulates signals of some receptor protein tyrosine kinases (including INSR, EGFR, CSF1R, and PDGFR), non-receptor protein tyrosine kinases (such as JAK1, JAK2, and JAK3), transcription factors (STAT1, STAT3, and STAT6), and Src family kinases (Fyn and Lck). PTPN2 can negatively regulate the signal transduction mediated by inflammatory cytokines (IL-2 and interferon) through the dephosphorylation of JAK1, JAK3 and their substrates signal transducer and activator of transcription 1 (STATI).

Related studies have shown that inhibiting the PTPN2 immunomodulatory factor can promote anti-tumor immunity, thereby clearing tumors. Specifically, deleting the gene expressing PTPN2 from the immune system (CD8+ T cells) of cancer-affected mice can stimulate the production and adaptability of killer T cells that fight infection and cancer. In one of the experiments, deleting PTPN2 eliminated colon cancer in all mice. In addition, another experiment showed that deletion of PTPN2 in combination with PD-1 blockade therapy successfully eliminated a quarter of melanomas in mice harboring highly aggressive and treatment-resistant cancers.

### Summary of the invention

In order to solve the above technical problems existing in the prior art, the present invention provides a novel PTPN2 inhibitor, which has good drug development prospects in the treatment or prevention of a disease or disorder related to PTPN2.

Specifically,
On the one hand, the present invention provides a compound having a structure represented by formula (I-1) or (I-2) or an enantiomer, diastereomer, racemate, tautomer, stereoisomer, geometrical isomer, N-oxide, metabolite or pharmaceutically acceptable salt, ester, solvate, hydrate, isotope-labeled compound or prodrug thereof; 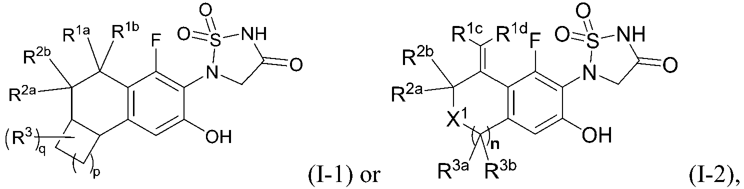
wherein R^{1a} and R^{1b} are each independently selected from H, D, halogen, -OH, C₁₋₆ alkyl, C₁₋₆ alkoxy, C₁₋₆ haloalkyl, and C₁₋₆ haloalkoxy;
R^{1c} and R^{1d} are each independently selected from H, D, halogen, C₁₋₆ alkyl, and C₁₋₆ haloalkyl;
R^{2a} is selected from H, D, -OH, -NH₂, C₁₋₈ alkyl, C₂₋₈ alkenyl, C₃₋₆ cycloalkyl, 5-6 membered heteroaryl, 4-8 membered heterocyclyl, -O-C₁₋₈ alkyl, -O-C₁₋₆ alkylene-C₃₋₆ cycloalkyl, -O-C₁₋₆ alkylene-(4-8 membered heterocyclyl), -O-C(=O)-N(R^{a})-C₁₋₈ alkyl, -O-C(=O)-N(R^{a})-phenyl, -N(R^{a})-C₁₋₈ alkyl, -N(R^{a})-C₃₋₆ cycloalkyl, -N(R^{a})-(4-8 membered heterocyclyl), -N(R^{a})-C(=O)-C₁₋₈ alkyl, -N(R^{a})-C(=O)-O-C₁₋₈ alkyl, -N(R^{a})-C₁₋₆ alkylene-C₃₋₆ cycloalkyl, -N(R^{a})-C₁₋₆ alkylene-Si(R^{c})₃, -N(R^{a})-(C=N(R^{b}))-C₁₋₈ alkyl, -N(R^{a})-S(=O)_{w}-C₁₋₈ alkyl, -N(R^{a})-C₁₋₆ alkylene-(4-8 membered heterocyclyl), - N(R^{a})-C₁₋₆ alkylene-(5-6 membered heteroaryl), -N(R^{a})-C₁₋₆ alkylene-phenyl, -C₁₋₆ alkylene-C₃₋₆ cycloalkyl, -C₁₋₆ alkylene-(4-8 membered heterocyclyl), -C₁₋₆ alkylene-N(R^{a})(R^{b}), -C₁₋₆ alkylene-N(R^{a})-C(=O)-O-C₁₋₈ alkyl, -C₁₋₆ alkylene-N(R^{a})-C₁₋₈ alkyl, - C₁₋₆ alkylene-N(R^{a})-C₁₋₆ alkylene-C₃₋₆ cycloalkyl, -C₁₋₆ alkylene-N(R^{a})-C(=O)-C₁₋₈ alkyl, -C₁₋₆ alkylene-N(R^{a})-C₁₋₆ alkylene-(4-8 membered heterocyclyl), -C₁₋₆ alkylene-N(R^{a})-C₁₋₆ alkylene-(5-6 membered heteroaryl), -C₁₋₆ alkylene-N(R^{a})-C₁₋₆ alkylene-phenyl, -C₁₋₆ alkylene-O-C₁₋₈ alkyl, -C₁₋₆ alkylene-O-C₁₋₆ alkylene-C₃₋₆ cycloalkyl, -C₁₋₆ alkylene-O-C₁₋₆ alkylene-(4-8 membered heterocyclyl), -C₁₋₆ alkylene-O-C₁₋₆ alkylene-N(R^{a})(R^{b}), -S(=O)_{w}-C₁₋₈ alkyl, and -C(=O)-N(R^{a})-C₁₋₈ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h};
R^{2b} is selected from H, D, -OH, halogen, -N(R^{a})(R^{b}), and -N(R^{a})-N(R^{b})-C(O)-phenyl;
R³, R^{3a}, and R^{3b} are each independently selected from H, D, halogen, -OH, C₁₋₆ alkyl, C₁₋₆ haloalkyl, C₁₋₆ alkoxy, C₁₋₆ haloalkoxy, C₃₋₆ cycloalkyl, C₃₋₆ halocycloalkyl, -NH₂, -NH(C₁₋₆ alkyl), and -N(C₁₋₆ alkyl)(C₁₋₆ alkyl);
X¹ is selected from O, S, NR^{f}, and C(R^{e})(R^{d});
R^{a} and R^{b} are each independently selected from H and C₁₋₆ alkyl; optionally, the C₁₋₆ alkyl is substituted by one or more substituents selected from halogen, -CN, oxo, and -OH;
R^{c} is selected from -OH, C₁₋₆ alkyl, and phenyl;
R^{e} and R^{d} are each independently selected from H, D, halogen, -OH, C₁₋₆ alkyl, C₁₋₆ haloalkyl, C₁₋₆ alkoxy, C₁₋₆ haloalkoxy, C₃₋₆ cycloalkyl, C₃₋₆ halocycloalkyl, -NH₂, -NH(C₁₋₆ alkyl), and -N(C₁₋₆ alkyl)(C₁₋₆ alkyl); optionally, R^{e} and R^{3a} together with the atoms to which they connect form a 3-7 membered carbocyclic ring; or R^{e} and R^{2b} together with the atoms to which they connect form a 3-7 membered carbocyclic ring;
R^{f} is selected from H, C₁₋₆ alkyl, and C₁₋₆ haloalkyl; optionally, R^{f} and R^{3a} together with the atoms to which they connect form a 3-7 membered heterocyclic ring; or R^{f} and R^{2b} together with the atoms to which they connect form a 3-7 membered heterocyclic ring;
each R^{g} is independently selected from H, D, halogen, -OH, -CN, nitro, oxo, -NH₂, -NH(C₁₋₆ alkyl), -N(C₁₋₆ alkyl)(C₁₋₆ alkyl), C₁₋₆ alkyl, and C₁₋₆ haloalkyl;
R^{h} is selected from H, D, C₁₋₆ alkyl, and C₁₋₆ haloalkyl;
n is selected from 0 or 1;
p is selected from 0, 1, 2, or 3;
q is selected from 0, 1, 2, 3, or 4;
w is selected from 1 or 2.

On the other hand, the present invention provides a pharmaceutical composition including the compound or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof according to the present invention and at least one pharmaceutically acceptable carrier.

On the other hand, the present invention provides the compound or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof according to the present invention, or the composition according to the present invention, for treating or preventing a disease or disorder related to PTPN2.

On the other hand, the present invention provides use of the compound or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof according to the present invention, or the composition according to the present invention, in a preparation/manufacture of a medicament for treating or preventing a disease or disorder related to PTPN2.

On the other hand, the present invention provides a method for treating or preventing a disease or disorder related to PTPN2, including administering to a person in need a therapeutically effective amount of the compound or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof according to the present invention, or the composition according to the present invention.

On the other hand, the present invention relates to methods for preparing, separating and purifying compounds represented by formulae (I-1), (I-2), (II-0), (II-1), (II-2), (III-0), (III-1), (III-2), (IV-1), (IV-2), (V), (V-1), (VI-0), and (VI-1).

Any embodiment of any aspect of the present invention can be combined with another embodiment, as long as they do not conflict. Furthermore, any technical feature in any embodiment of any aspect of the present invention can be applied to the technical feature in another embodiment, as long as they do not conflict.

The foregoing description only summarizes certain aspects of the present invention but is not limited to these aspects. Content of these aspects and other aspects will be more specifically and completely described below. All references in this specification are incorporated herein by reference in their entireties.

### Detailed description

In order to make the purposes, technical solutions, and advantages of the present invention clearer and more understandable, the present invention is described in further detail hereinafter in conjunction with embodiments. The specific embodiments described herein are only used to explain the present invention and are not intended to constitute any limitation on the present invention. Furthermore, in the following description, descriptions of well-known structures and techniques are omitted to avoid unnecessary confusion of the concepts of the present disclosure. Such structures and techniques are also described in numerous publications.

### Definitions

Certain embodiments of the present invention are now described in detail, and examples thereof are illustrated by the accompanying structural and chemical formulae. The present invention is intended to encompass all substitutions, modifications, and equivalent technical solutions, which all fall within the scope of the present invention as defined by the claims. Those skilled in the art should recognize that many methods and materials similar or equivalent to those described herein can be used in practicing the present invention. The present invention is by no means limited to the methods and materials described herein. In the event that one or more of the combined literatures, patents, and similar materials differ from or contradict the present application (including, but not limited to, defined terms, applications of terms, described technologies, etc.), the present application shall prevail.

It should be further recognized that certain features of the present invention, which are described in a plurality of separate embodiments for the sake of clarity, may alternatively be provided in combination in a single embodiment. Conversely, various features of the present invention, for brevity, are described in a single embodiment, but may alternatively be provided individually or in any suitable sub-combination.

Unless otherwise specified, all technical terms used in the present invention have the same meanings as those commonly understood by those skilled in the art to which the present invention belongs. All patents and publications related to the present invention are incorporated herein by reference in their entireties.

Unless otherwise indicated, the following definitions used herein shall apply. For the purposes of the present invention, the chemical elements are consistent with the CAS version of the Periodic Table, and the Handbook of Chemistry and Physics, 75th Edition, 1994. In addition, general principles of organic chemistry can be found in Organic Chemistry, Thomas Sorrell, University Science Books, Sausalito: 1999, and March's Advanced Organic Chemistry by Michael B. Smith and Jerry March, John Wiley & Sons, New York: 2007, the entire content of which are incorporated herein by reference.

Unless otherwise indicated or unless there is an apparent conflict in the context, the articles "a", "an", and "the" as used herein are intended to include "at least one" or "one or more". Therefore, these articles as used herein refer to articles that refer to one or more than one (i.e., at least one) of the objects. For example, "a component" refers to one or more components, that is, there may be more than one component contemplated for adoption or use in the implementation of the described embodiment.

The term "subject" refers to an animal. Typically, the animal is a mammal. Subjects, for example, also refer to primates (e.g., humans, male or female), cattle, sheep, goats, horses, dogs, cats, rabbits, rats, mice, fish, birds, etc. In certain embodiments, the subject is a primate. In another embodiment, the subject is a human.

The term "patient" refers to humans (including adults and children) or other animals. In some embodiments, "patient" refers to humans.

The term "comprise(s)", "comprising", "include(s)", "included", "including", "encompass", "contain(s)", or "containing" is an open expression, namely including the content specified in the present invention but not excluding other content.

When substituents are described by conventional chemical formulae written from left to right, the substituents also include chemically equivalent substituents that would result if the formulae were written from right to left. For example, -CH₂O- is equivalent to -OCH₂-.

The term "enantiomer" refers to two non-superimposable isomers of a compound that are mirror images of each other.

The term "diastereomer" refers to stereoisomers that have two or more chiral centers and whose molecules are not mirror images of each other. Diastereomers have different physical properties, such as melting points, boiling points, spectral properties, and reactivity. Diastereomeric mixtures can be separated by high resolution analytical procedures such as electrophoresis and chromatography, for example, HPLC.

The terms "racemate" "raceme" or "racemic mixture" refer to an equimolar mixture of two enantiomers that lack optical activity.

The term "tautomer" or "tautomeric form" refers to structural isomers that have different energies and that can be converted into each other through a low energy barrier. If tautomerism is possible (such as in a solution), a chemical equilibrium of tautomers can be achieved. For example, proton tautomers (also called prototropic tautomers) involve interconversion that is performed by proton migration, such as keto-enol isomerization and imine-enamine isomerization. For another example, 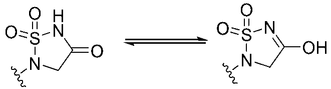 is provided. Valence tautomers involve interconversion that is performed by reconfiguration of some of the bonding electrons. A specific example of keto-enol tautomerism is the interconversion of pentane-2,4-dione and 4-hydroxypent-3-en-2-one tautomers. Another example of tautomerism is phenol-keto tautomerism. A specific example of phenol-keto tautomerism is the interconversion of pyridin-4-ol and pyridin-4(1H)-one tautomers. Unless otherwise indicated, all tautomeric forms of the compounds of the present invention are within the scope of the present invention.

The term "stereoisomer" refers to compounds that have the same chemical constitution, but differ in the way the atoms or groups are arranged in space. Stereoisomers include enantiomers, diastereomers, conformers (rotamers), geometric isomers (cis/trans isomers), atropisomers, and the like.

The term "geometric isomer" is also called "cis-trans isomer", which is an isomer caused by double bond (including olefin double bond, C=N double bond, and N=N double bond) or a single bond of ring carbon atom that cannot rotate freely.

The stereochemical definitions and rules used in the present invention generally follow S. P. Parker, Ed., McGraw-Hill Dictionary of Chemical Terms (1984) McGraw-Hill Book Company, New York; and Eliel, E. and Wilen, S, Stereochemistry of Organic Compounds, John Wiley & Sons, Inc, New York, 1994. Many organic compounds exist in optically active forms, that is, they have the ability to rotate the plane of plane polarized light. When describing optically active compounds, the prefixes D and L or R and S are used to indicate the absolute configuration of the molecule about one or more of its chiral centers. The prefixes d and l or (+) and (-) are the symbols used to specify the rotation of plane polarized light caused by the compound, where (-) or l indicates that the compound is left-handed, and the prefix (+) or d indicates that the compound is right-handed. A specific stereoisomer is an enantiomer, and a mixture of such isomers is called an enantiomeric mixture. A mixture of enantiomers at ratio of 50:50 is called a racemic mixture or racemate, which can occur when there is no stereoselectivity or stereospecificity in a chemical reaction or process.

Any asymmetric atom (e.g., carbon) of the compounds disclosed by the present invention can exist in a racemic or enantiomerically enriched form, such as in form of (R)-, (S)-, or (R,S)-configuration. In certain embodiments, each asymmetric atom has at least 50% enantiomeric excess, at least 60% enantiomeric excess, at least 70% enantiomeric excess, at least 80% enantiomeric excess, at least 90% enantiomeric excess, at least 95% enantiomeric excess, or at least 99% enantiomeric excess, in terms of the (R)- or (S)-configuration.

Depending on the choice of starting materials and process, the compound according to the present invention can exist in the form of one of its possible isomers or a mixture thereof, such as a racemate and a diastereomeric mixture (which depends on the number of asymmetric carbon atoms). Optically active (R)- or (S)-isomers may be prepared using chiral synthons or chiral reagents, or resolved using conventional techniques. If the compound contains a double bond, the substituents may be in the E or Z configuration; and if the compound contains a disubstituted cycloalkyl group, the cycloalkyl substituents may be in the cis or trans configuration.

Any mixture of stereoisomers obtained may be separated into pure or substantially pure geometric isomers, enantiomers, or diastereomers, based on differences in the physicochemical properties of components, for example, by chromatography and/or fractional crystallization.

Any racemate of the resulting final product or intermediate may be resolved into optical enantiomers by methods familiar to those skilled in the art, for example, by separation of the diastereomeric salt thereof as obtained. The racemic products may alternatively be separated by chiral chromatography, for example, by high performance liquid chromatography (HPLC) using a chiral adsorbent. In particular, enantiomers can be prepared by asymmetric synthesis, for example, see Jacques, et al., Enantiomers, Racemates and Resolutions (Wiley Interscience, New York, 1981); Principles of Asymmetric Synthesis (2nd Ed. Robert E. Gawley, Jeffrey Aube, Elsevier, Oxford, UK, 2012); Eliel, E. L. Stereochemistry of Carbon Compounds (McGraw-Hill, NY, 1962); Wilen, S. H. Tables of Resolving Agents and Optical Resolution sp. 268 (E. L. Eliel, Ed., Univ. of Notre Dame Press, Notre Dame, IN 1972); Chiral Separation Techniques: A Practical Approach (Subramanian, G. Ed., Wiley-VCH Verlag GmbH & Co. KGaA, Weinheim, Germany, 2007).

The term "nitrogen oxide" or "N-oxide" refers to that when a compound contains several amine functional groups, one or more nitrogen atoms can be oxidized to form an N-oxide. Specific examples of N-oxides are N-oxides of tertiary amines or N-oxides of nitrogen atoms of nitrogen-containing heterocyclic rings. The corresponding amines can be treated with an oxidant such as hydrogen peroxide or a peracid (e.g., peroxycarboxylic acid) to form an N-oxide (see Advanced Organic Chemistry, Wiley Interscience, 4th edition, Jerry March, pages). In particular, the N-oxides can be prepared by the method of L.W. Deady (Syn. Comm. 1977, 7, 509-514), wherein for example, the amine compounds are reacted with m-chloroperoxybenzoic acid (MCPBA), e.g., in an inert solvent such as dichloromethane.

The term "metabolite" refers to a product obtained by the metabolism of a specific compound or its salt in vivo. The metabolite of a compound can be identified by techniques well known in the art, and its activity can be characterized by experimental methods as described in the present invention. Such products can be obtained by oxidation, reduction, hydrolysis, amidation, deamidation, esterification, defatting, enzymatic cleavage, and the like of the administered compound. Correspondingly, the present invention includes metabolites of compounds, including metabolites produced by fully contacting the compounds of the present invention with mammals for a period of time.

The term "pharmaceutically acceptable" means that a substance or composition must be chemically and/or toxicologically compatible with the other ingredients of the formulation and/or the mammals treated therewith. Preferably, the "pharmaceutically acceptable" of the present invention refers to those approved by federal regulatory agencies or national governments or listed in the U.S. Pharmacopoeia or other generally recognized pharmacopeias for use in animals, particularly humans.

The term "pharmaceutically acceptable salt" refers to organic and inorganic salts of the compounds of the present invention. Pharmaceutically acceptable salts are well known in the art, as described in the literature: S.M. Berge et al., J. Pharmaceutical Sciences, 66: 1-19, 1977. Pharmaceutically acceptable salts include salts formed by compounds with acids, including but not limited to inorganic acid salts (such as hydrochlorides, hydrobromides, phosphates, sulfates, nitrates, and perchlorates) and organic acid salts (such as acetates, glycolates, oxalates, maleates, tartrates, citrates, succinates, fumarates, mandelates, and sulfosalicylate), or these salts obtained by other methods described in books and literature, such as ion exchange methods. More pharmaceutically acceptable salts include adipate, alginate, ascorbate, aspartate, benzenesulfonate, benzoate, bisulfate, borate, butyrate, camphorate, camphorsulfonate, cyclopentylpropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, malonate, methanesulfonate, 2-naphthalenesulfonate, nicotinate, oleate, palmitate, pamoate, pectinate, persulfate, 3-phenylpropionate, picrate, pivalate, propionate, stearate, thiocyanate, p-toluenesulfonate, undecanoate, valerate, and the like. Pharmaceutically acceptable salts also include salts formed by compounds with bases, including but not limited to, inorganic base salts (such as alkali metal salts, alkaline earth metal salts, ammonium salts, and N⁺(C₁₋₄ alkyl)₄ salts), and alkali metal or alkaline earth metal salts include sodium, lithium, potassium, calcium, magnesium, and the like. The present invention also contemplates quaternary ammonium salts formed by any compounds including N groups. Water-soluble or oil-soluble or dispersible products can be obtained by quaternization. Pharmaceutically acceptable salts further include appropriate and non-toxic ammonium, quaternary ammonium salts and amine cations formed by counter ions, such as halides, hydroxides, carboxylates, sulfates, phosphates, nitrates, C₁₋₈ sulfonates, and aromatic sulfonates. Organic base salts (e.g., primary, secondary, and tertiary amine salts, substituted amines (including naturally occurring substituted amines, cyclic amines, basic ion exchange resins) salts), and certain organic amine salts include, for example, isopropylamine salts, benzathine salts, cholinate salts, diethanolamine salts, diethylamine salts, lysine salts, meglumine salts, piperazine salts, and tromethamine salts.

Pharmaceutically acceptable acid addition salts can be formed by the compounds of the present invention with inorganic or organic acid, and pharmaceutically acceptable base addition salts can be formed by the compounds of the present invention with inorganic or organic base. Pharmaceutically acceptable salts of the present invention can be synthesized by conventional chemical methods from parent compounds, alkaline or acidic moieties. Generally, such salts can be prepared by reacting the free acid forms of these compounds with a stoichiometric amount of a suitable base (such as hydroxides, carbonates, bicarbonates, etc. of Na, Ca, Mg or K), or by reacting the free base forms of these compounds with a stoichiometric amount of a suitable acid. Such reactions are usually carried out in water or an organic solvent or mixture of the two. Generally, in appropriate cases, it is necessary to use a non-aqueous medium such as ether, ethyl acetate, ethanol, isopropanol or acetonitrile. Additional lists of suitable salts can be found in, for example, Remington's Pharmaceutical Sciences, 20th edition, Mack Publishing Company, Easton, Pa., (1985); and Handbook of Pharmaceutical Salts: Properties, Selection, and Use, Stahland Wermuth (Wiley-VCH, Weinheim, Germany, 2002).

The term "solvate" refers to an association formed by one or more solvent molecules and the compound of the present invention. The solvent may be water, acetic acid, ether, isopropyl ether, petroleum ether, ethyl formate, ethyl acetate, isopropyl acetate, n-propyl acetate, isobutyl acetate, n-butyl acetate, methyl tert-butyl ether (MTBE), n-heptane, a mixed solvent of ethanol and water at a volume ratio of 10:90 to 90:10, acetone, methyl isobutyl ketone, acetonitrile, benzene, chloroform, carbon tetrachloride, dichloromethane, dimethyl sulfoxide, 1,4-dioxane, ethanol, ethyl acetate, ethylene glycol, n-butanol, tert-butanol, sec-butyl alcohol, N,N-dimethylacetamide, N,N-dimethylformamide, formamide, formic acid, n-hexane, cyclohexane, n-heptane, a mixed solvent of n-heptane and ethyl acetate at a volume ratio of 1:5 to 5:1, isopropanol, methanol, butanone, 1-methyl-2-pyrrolidone, mesitylene, nitromethane, polyethylene glycol, n-propanol, isopropanol, 2-acetone, 4-methyl-2-pentanone, pyridine, tetrahydrofuran, methyl ethyl ketone, toluene, xylene, isopropylbenzene or a mixture thereof, etc.

The term "hydrate" refers to an associated matter formed by one or more water molecules and the compound of the present invention.

In addition, the compounds disclosed in the present invention, including their salts, can alternatively be obtained in the form of their hydrates or in the form of containing their solvents (such as ethanol or DMSO) for their crystallization. The compounds disclosed in the present invention can form solvates with pharmaceutically acceptable solvents (including water), either inherently or by design. Therefore, the present invention is intended to include both solvated and unsolvated forms.

The term "ester" is represented by the formula -OC(O)R or -C(O)OR, wherein R may be an alkyl, cycloalkyl, alkenyl, cycloalkenyl, alkynyl, cycloalkynyl, aryl or heteroaryl group as described herein.

The term "isotope-labeled compound" refers to a compound of the present invention being labeled with an isotope. The compound is identical to those compounds described herein except for the fact that one or more atoms are replaced by atoms having an atomic mass or mass number different from the natural common atomic mass or mass number. Exemplary isotopes that may also be introduced into the compounds of the present invention include isotopes of hydrogen, carbon, nitrogen, oxygen, phosphorus, sulfur, fluorine, and chlorine, such as ²H, ³H, ¹³C, ¹⁴C, ¹⁵N, ¹⁶O, ¹⁷O, ³¹P, ³²P, ³⁶S, ¹⁸F, and ³⁷Cl.

The compounds of the present invention including the aforementioned isotope labels and/or other isotope labels of other atoms and pharmaceutically acceptable salts of the compounds all fall within the scope of the present invention. Isotope-labeled compounds of the present invention, e.g., radioactive isotope-labeled compounds such as ³H and ¹⁴C, are incorporated into the compounds of the present invention and can be used for drug and/or substrate tissue distribution analysis. Due to ease of preparation as well as detection, deuterated, i.e., ³H, and carbon-14, i.e., ¹⁴C, isotopes are particularly preferred. In addition, substitution with isotopes of larger mass numbers, such as deuterium, i.e., ²H, can provide some greater therapeutic advantages of metabolic stability, such as increased in vivo half-life or reduced dosage requirements. Therefore, it may be preferred in some cases.

In addition, the substitution of heavier isotopes, particularly deuterium (i.e., ²H or D) can provide certain therapeutic advantages, which are brought about by higher metabolic stability. For example, an increased in vivo half-life or a reduction in dosage requirements or an improved therapeutic index. It should be understood that deuterium in the present invention is regarded as a substituent of compounds of formulae I to VI. The concentration of such heavier isotopes, particularly deuterium, can be defined by an isotopic enrichment factor. The term "isotopic enrichment factor" used in the present invention refers to the ratio between the isotopic abundance and the natural abundance of a specified isotope. Where a substituent of a compound of the present invention is designated as deuterium, the compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation). Pharmaceutically acceptable solvates according to the present invention include those wherein the solvent of crystallization may be isotopically substituted, for example D₂O, acetone-d6, and DMSO-d6.

The term "prodrug", as used in the present invention, represents the in vivo conversion of a compound to a compound of formula I. Such conversion is affected by the hydrolysis of the prodrug in the blood or the enzymatic conversion of the prodrug in the blood or tissues into the parent structure. The prodrug compound of the present invention may be an ester, and among the existing invention, esters that can be used as prodrugs include phenyl esters, aliphatic (C1-24) esters, acyloxymethyl esters, carbonates, carbamates, and amino acid esters. For example, a compound in the present invention contains a hydroxyl group, which can be acylated to obtain a compound in the form of a prodrug. Other prodrug forms include phosphate esters, such as these phosphate ester compounds obtained by phosphorylation of the hydroxyl group on the parent. For a complete discussion of prodrugs, please refer to the following literature: Higuchi et al., Pro-drugs as Novel Delivery Systems, Vol. 14, A.C.S. Symposium Series; Roche et al., Bioreversible Carriers in Drug Design, American Pharmaceutical Association and Pergamon Press, 1987; Rautio et al., Prodrugs: Design and Clinical Applications, Nature Reviews Drug Discovery, 2008, 7, 255-270, and Hecker et al., Prodrugs of Phosphates and Phosphonates, J. Med. Chem., 2008, 51, 2328-2345.

Solid lines ( 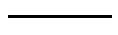 ), solid wedges ( 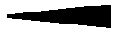 ), and dashed wedges ( 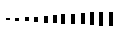 ) may be used herein to depict chemical bonds of the compounds of the present invention. The use of solid lines to depict bonds attached to asymmetric carbon atoms is intended to indicate that all possible stereoisomers (e.g., specific enantiomers and racemic mixtures) at that carbon atom are included. The use of solid or dashed wedges to depict bonds attached to asymmetric carbon atoms is intended to indicate the presence of the stereoisomers shown. When present in a racemic mixture, solid and dashed wedges are used to define relative stereochemistry, rather than absolute stereochemistry.

When a bond of the substituent is shown as passing through a bond that connecting two ring atoms ("floating bond"), such a substituent may be bonded to any ring atom in the substitutable ring, unless otherwise indicated. In the case where an available ring member is shown to carry a substitutable hydrogen atom, the substitutable hydrogen atom is substantially substituted (i.e., not present) when the floating bond is bonded to the available ring member.

Unless otherwise explicitly stated, when a substituent is described by a conventional chemical formula written from left to right, the substituent includes only its form written from left to right, respectively, connected to the left and right sides of the corresponding group in the structure of the compound of general formula.

Unless otherwise explicitly stated, the descriptions used in the present invention such as "each...independently", "...each independently", and "...independently" are interchangeable and should be understood broadly, which may mean that the specific options expressed by the same symbols in different groups do not affect each other, and may also mean that the specific options expressed by the same symbols in the same group do not affect each other.

The term "optional", "optionally" or "arbitrarily" means that an event or situation described subsequently may but need not occur, and the description includes instances where the event or situation occurs and instances where it does not occur. For example, "optionally substituted by ..." means that the substitution may or may not be present.

When the term "each independently" is used in combination with "optionally", for example, "each independently and optionally substituted by ..." means that the specific options are either substituted by or not substituted by ... independently of each other.

The term "unsaturation" or "unsaturated" refers to the moiety contains one or more degrees of unsaturation.

In various parts of this specification, the substituents of the compounds of the present invention are disclosed in terms of group types or ranges. It is particularly noted that the present invention includes each independent secondary combination of the individual members of these group types and ranges. For example, the term "C₁₋₆ alkyl" specifically refers to independently disclosed methyl, ethyl, C₃ alkyl, C₄ alkyl, C₅ alkyl, and C₆ alkyl.

In various parts of the present invention, linking substituents are described. When the structure clearly requires a linking group, the Markush variable listed for the group should be understood as a linking group. For example, if the structure requires a linking group and the Markush group definition for the variable lists "alkyl" or "aryl", it should be understood that the "alkyl" or "aryl" represents a linked alkylene group or arylene group, respectively.

The term "heteroatom" refers to O, S, N, P, and Si, including any oxidation state of S, N, and P; primary, secondary, tertiary amines and quaternary ammonium salts; or that the hydrogen on the nitrogen atom in the heterocyclic ring is substituted, for example, N (such as N in 3,4-dihydro-2H-pyrrolyl), NH (such as NH in pyrrolidinyl) or NRT (such as NRT in N-substituted pyrrolidinyl, RT is a substituent on N). Among the compounds involved in the present invention, when containing multiple heteroatoms, the compounds composed of the multiple heteroatoms conform to the covalent rules and composition rules of organic compounds, that is, the compounds containing multiple heteroatoms should exclude compounds that do not conform to the covalent rules and composition rules of organic compounds.

The term "heterocyclyl" or "heterocyclic" refers to optionally substituted partially or fully saturated non-aromatic cyclic group, for example, a 4-7-membered monocyclic, 7-12-membered bicyclic or 10-15-membered tricyclic system, which has at least one heteroatom in at least one of the rings containing carbon atoms. Each ring of the heterocyclyl containing heteroatoms may have 1, 2 or 3 heteroatoms selected from nitrogen, oxygen and sulfur atoms, wherein nitrogen and sulfur heteroatoms may also optionally be oxidized. The heterocyclyl may be connected at the heteroatom or carbon atom. In some embodiments, the heterocyclyl is selected from: monocyclic heterocyclyl, bicyclic heterocyclyl, and tricyclic heterocyclyl. A 4-8-membered heterocyclic group indicates that the ring atoms are composed of 4-8 carbon atoms and heteroatoms. The heterocyclic group includes fused rings, spirocycles, bridged rings, and combinations thereof. In some embodiments, the monocyclic heterocyclyl is selected from oxetanyl, pyrrolidinyl, tetrahydrofuranyl, piperazinyl, tetrahydropyranyl, morpholinyl, thiomorpholinyl, thiomorpholinyl sulfoxide, thiomorpholinyl sulfone, 1,3-dioxolane and tetrahydro-1,1-dioxothiophenyl, 1,1,4-trioxo-1,2,5-thiadiazolidin-2-yl, etc. In some embodiments, the bicyclic heterocyclyl is selected from 7-oxabicyclo[2.2.1]heptyl.

The term "carbocycle" refers to a saturated (i.e., "cycloalkyl" and "cycloalkylene") or partially unsaturated (i.e., having one or more double bonds (i.e., "cycloalkenyl" and "cycloalkenylene") and/or triple bonds within the ring) monocyclic or polycyclic hydrocarbon ring having, for example, 3-10 (suitably 3-8, more suitably 3-7, 3-6, 4-6 or 5-6) ring carbon atoms, including, but not limited to, cyclopropyl(ene) (ring), cyclobutyl(ene) (ring), cyclopentyl(ene) (ring), cyclohexyl(ene) (ring), cycloheptyl(ene) (ring), cyclooctyl(ene) (ring), cyclononyl(ene) (ring), cyclobutenyl(ene) (ring), cyclopentenyl(ene) (ring), cyclohexenyl(ene) (ring), cycloheptenyl(ene) (ring), cyclooctenyl(ene) (ring), cyclononenyl(ene) (ring), etc. The carbocycle includes fused rings, spiro rings, bridged rings, and combinations thereof.

The term "cycloalkyl" refers to a monovalent or polyvalent saturated or partially unsaturated monocyclic, bicyclic or tricyclic non-aromatic system containing carbon atoms. C₃₋₆ cycloalkyl refers to a cycloalkyl having 3-6 ring carbon atoms. In some embodiments, the cycloalkyl is selected from: monocyclic cycloalkyl, bicyclic cycloalkyl, and tricyclic cycloalkyl. In some embodiments, monocyclic cycloalkyl includes, but is not limited to, cyclopropyl, cyclobutyl, cyclopentyl, cyclopentenyl, cyclohexyl, and cyclohexenyl. In some embodiments, bicyclic cycloalkyl includes, but is not limited to, bornyl, indolyl, hexahydroindolyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl, etc. In some embodiments, tricyclic hydrocarbon groups include adamantly, etc.

The term "heteroaryl" or "heteroaromatic ring" refers to a monocyclic, bicyclic and tricyclic aromatic system containing heteroatoms. The term "heteroaryl" can be used interchangeably with the term "heteroaromatic ring" or "heteroaromatic compound". The heteroatoms have the definition described in the present invention. In some embodiments, the heteroaryl is a heteroaryl consisting of 5-10 atoms containing 1, 2, 3 or 4 heteroatoms independently selected from O, S, and N, i.e., 5-10 membered heteroaryl; the heteroaryl is a heteroaryl consisting of 5-8 atoms containing 1, 2, 3 or 4 heteroatoms independently selected from O, S, and N, i.e., 5-8 membered heteroaryl; in some embodiments, the heteroaryl is a heteroaryl consisting of 5-7 atoms containing 1, 2, 3 or 4 heteroatoms independently selected from O, S, and N, i.e., 5-7-membered heteroaryl; in some embodiments, the heteroaryl is a heteroaryl consisting of 5-6 atoms containing 1, 2, 3 or 4 heteroatoms independently selected from O, S, and N, i.e., 5-6 membered heteroaryl; in some embodiments, the heteroaryl is a heteroaryl consisting of 5 atoms containing 1, 2, 3 or 4 heteroatoms independently selected from O, S, and N, i.e., 5-membered heteroaryl; and in some embodiments, the heteroaryl is a heteroaryl consisting of 6 atoms containing 1, 2, 3 or 4 heteroatoms independently selected from O, S, and N, i.e., 6-membered heteroaryl.

The term "aryl" or "aryl ring" refers to a monocyclic, bicyclic, or tricyclic aromatic carbocyclic system. The term "aryl" can be used interchangeably with the term "aryl ring" or "aromatic ring". 6-10 membered aryl refers to an aromatic group containing 6-10 ring atoms. Examples include, but are not limited to, phenyl, naphthyl, etc.

The term "hydrogen" refers to ¹H; and "deuterium" refers to ²H.

The terms "halogen" and "halo" refer to fluorine (F), chlorine (Cl), bromine (Br) or iodine (I).

The term "amino" refers to -NH₂.

The term "hydroxyl" refers to -OH.

The term "cyano" refers to -CN.

The term "nitro" refers to -NO₂.

The term "carboxyl" refers to HO(C=O)-.

The term "oxo" refers to O=, that is, when the substituent is O=, O is connected to the substituted group by a double bond.

The term "alkyl" or "alkyl group" refers to a saturated, straight or branched-chain hydrocarbon group containing carbon atoms. In one embodiment, the alkyl group contains 1-6 carbon atoms, i.e., C₁₋₆ alkyl; in another embodiment, the alkyl group contains 1-4 carbon atoms, i.e., C₁₋₄ alkyl; and in another embodiment, the alkyl group contains 1-3 carbon atoms, i.e., C₁₋₃ alkyl. Examples of alkyl groups include, but are not limited to, methyl, ethyl, n-propyl, isopropyl, n-butyl, tert-butyl, isobutyl, sec-butyl, n-pentyl, n-hexyl, and the like.

The term "alkylene" means a saturated, straight or branched-chain, divalent hydrocarbon group containing carbon atoms. In one embodiment, the alkylene group contains 1-6 carbon atoms, i.e., C₁-C₆ alkylene; in another embodiment, the alkylene group contains 1-4 carbon atoms, i.e., C₁-C₄ alkylene; and in another embodiment, the alkylene group contains 1-3 carbon atoms, i.e., C₁-C₃ alkylene.

The term "alkenyl" refers to a straight or branched-chain monovalent hydrocarbon group containing carbon atoms with at least one unsaturated site, i.e., a carbon-carbon sp² double bond, including the positioning of "cis" and "tans", or "E" and "Z". In one embodiment, the alkenyl group contains 2-6 carbon atoms, i.e., C₂-C₆ alkenyl; and in another embodiment, the alkenyl group contains 2-4 carbon atoms, i.e., C₂-C₄ alkenyl. Examples of alkenyl groups include, but are not limited to, vinyl (-CH=CH₂), allyl (-CH₂CH=CH₂), and the like.

The term "alkoxy" refers to alkyl group that is connected to the rest moiety of a molecule via an oxygen atom, wherein the alkyl group has the meaning as described in the present invention. In one embodiment, the alkoxy group contains 1-6 carbon atoms, i.e., C₁₋₆ alkoxy; in another embodiment, the alkoxy group contains 1-4 carbon atoms, i.e., C₁₋₄ alkoxy; and in yet another embodiment, the alkoxy group contains 1-3 carbon atoms, i.e., C₁₋₃ alkoxy.

The term "comprising" is synonymous with "including", "containing", or "characterized by", which is inclusive or open-ended, and does not exclude additional, unmentioned elements or ingredients from the drug (or, step in the case of a method). The phrase "consisting of......" does not include any element, step, or ingredient not specified in the drug (or step in the case of a method). The phrase "consisting essentially of ..." refers to the specified materials and those materials that do not materially affect the basic and novel characteristics of the drug (or step in the case of a method).

As described herein, the ring system formed by substituent R connected to the center of the ring by a bond (as shown in the figure below) represents the substitution of substituent R at any substitutable or any reasonable position on ring A. For example, formula f represents any possible substituted position on ring A, as shown in formulae f1-f4:

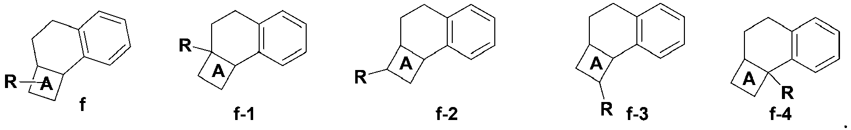

As described herein, the ring system formed by a substituent connected to the center of the ring by a bond, such as (R^{x})ₙ, which represents n substituents R^{x}, can be substituted at any substitutable position on the ring. For example, formula a represents a benzene ring that can be substituted by n R^{x}.

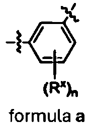

The term "substituted" refers to one or more hydrogen atoms on a specific group being replaced by a specific substituent. The specific substituent is a substituent described above, or a substituent appearing in the embodiments. Unless otherwise specified, a substituted group may have a substituent selected from a specific group at any substitutable site of the group, and the substituent may be the same or different at each position, that is, each substitution is independent of each other. It should be understood by those skilled in the art that the combinations of substituents contemplated in the present invention are those that are stable or chemically feasible.

Unless otherwise stated, substituents or group combinations involved herein for Markush structures are those that are stable or chemically feasible.

The term "tumor" as used herein refers to all neoplastic cell growth and proliferation, whether malignant or benign, as well as all precancerous and cancerous cells and tissues. The terms "cancer", "cancerous", "cell proliferative disorders", "proliferative disorders", and "tumor" are not mutually exclusive when referred to herein.

The terms "cancer" and "cancerous" as used herein refer to or describe the physiological condition in a subject that is typically characterized by unregulated cell growth and/or proliferation. Some cancers consist of cells that divide rapidly, while other cancers consist of cells that divide more slowly than normal cells. The types of cancer examples may include or exclude, for example, carcinoma, lymphoma (e.g., Hodgkin and non-Hodgkin lymphoma), blastoma, sarcoma, and leukemia. More specific examples of such cancers may include or exclude, for example, squamous cell carcinoma, small cell lung cancer, non-small cell lung cancer, lung adenocarcinoma, lung squamous carcinoma, peritoneal carcinoma, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, endometrial cancer or uterine cancer, salivary gland cancer, kidney cancer, liver cancer, prostate cancer, vulvar cancer, thyroid cancer, hepatic carcinoma, leukemia and other lymphoproliferative disorders, and various types of head and neck cancer.

### Description of the compounds of the present invention

The present invention provides a compound or an enantiomer, diastereomer, racemate, tautomer, stereoisomer, geometric isomer, N-oxide, metabolite or pharmaceutically acceptable salt, ester, solvate, hydrate, isotope-labeled compound or prodrug thereof, which plays an active role in treating or preventing PTPN2-related diseases or disorders.

Specifically, the present invention provides a compound having a structure represented by formula (I-1) or (I-2) or an enantiomer, diastereomer, racemate, tautomer, stereoisomer, geometric isomer, N-oxide, metabolite or pharmaceutically acceptable salt, ester, solvate, hydrate, isotope-labeled compound or prodrug thereof; 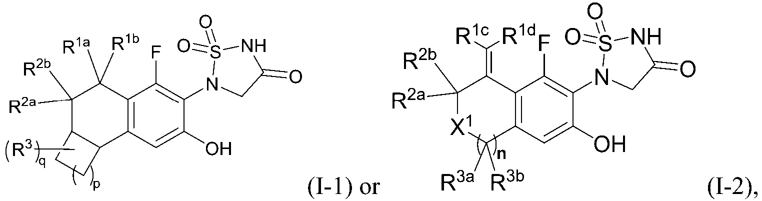 wherein R¹², R^{1b}, R^{1c}, R^{1d}, R^{2a}, R^{2b}, R³, R^{3a}, R^{3b}, X¹, n, p, and q have the definitions described in the present invention.

In some embodiments, the compounds of the present invention are not 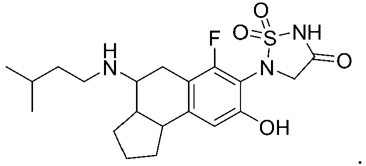 In some embodiments, R^{1a} and R^{1b} are each independently selected from H, D, halogen, -OH, C₁₋₆ alkyl, C₁₋₆ alkoxy, C₁₋₆ haloalkyl, and C₁₋₆ haloalkoxy.

In some embodiments, R^{1a} and R^{1b} are each independently selected from H, D, halogen, -OH, C₁₋₄ alkyl, C₁₋₄ alkoxy, C₁₋₄ haloalkyl and C₁₋₄ haloalkoxy.

In some embodiments, R^{1a} and R^{1b} are each independently selected from H, D, F, Cl, Br, -OH, methyl, ethyl, methoxy, ethoxy, trifluoromethyl, difluoromethyl, 2,2-difluoroethyl, trifluoromethoxy, and 2,2-difluoroethoxy.

In some embodiments, R^{1a} and R^{1b} are each independently selected from H, D, F, and -OH.

In some embodiments, R^{1a} and R^{1b} are both H.

In some embodiments, R^{1c} and R^{1d} are each independently selected from H, D, halogen, C₁₋₆ alkyl, and C₁₋₆ haloalkyl.

In some embodiments, R^{1c} and R^{1d} are each independently selected from H, D, halogen, C₁₋₄ alkyl, and C₁₋₄ haloalkyl.

In some embodiments, R^{1c} and R^{1d} are each independently selected from H, D, F, Cl, Br, methyl, ethyl, trifluoromethyl, and difluoromethyl.

In some embodiments, R^{1c} and R^{1d} are each independently selected from H, D, and F.

In some embodiments, R^{1c} and R^{1d} are each independently selected from H and F.

In some embodiments, R^{2a} is selected from H, D, -OH, -NH₂, C₁₋₈ alkyl, C₂₋₈ alkenyl, C₃₋₆ cycloalkyl, 5-6 membered heteroaryl, 4-8 membered heterocyclyl, -O-C₁₋₈ alkyl, -O-C₁₋₆ alkylene-C₃₋₆ cycloalkyl, -O-C₁₋₆ alkylene-(4-8 membered heterocyclyl), -O-C(=O)-N(R^{a})-C₁₋₈ alkyl, -O-C(=O)-N(R^{a})-phenyl, -N(R^{a})-C₁₋₈ alkyl, -N(R^{a})-C₃₋₆ cycloalkyl, -N(R^{a})-(4-8 membered heterocyclyl), -N(R^{a})-C(=O)-C₁₋₈ alkyl, -N(R^{a})-C(=O)-O-C₁₋₈ alkyl, -N(R^{a})-C₁₋₆ alkylene-C₃₋₆ cycloalkyl, -N(R^{a})-C₁₋₆ alkylene-Si(R^{c})₃, -N(R^{a})-(C=N(R^{b}))-C₁₋₈ alkyl, -N(R^{a})-S(=O)_{w}-C₁₋₈ alkyl, -N(R^{a})-C₁₋₆ alkylene-(4-8 membered heterocyclyl), -N(R^{a})-C₁₋₆ alkylene-(5-6 membered heteroaryl), -N(R^{a})-C₁₋₆ alkylene-phenyl, -C₁₋₆ alkylene-C₃₋₆ cycloalkyl, -C₁₋₆ alkylene-(4-8 membered heterocyclyl), -C₁₋₆ alkylene-N(R^{a})(R^{b}), -C₁₋₆ alkylene-N(R^{a})-C(=O)-O-C₁₋₈ alkyl, -C₁₋₆ alkylene-N(R^{a})-C₁₋₈ alkyl, -C₁₋₆ alkylene-N(R^{a})-C₁₋₆ alkylene-C₃₋₆ cycloalkyl, -C₁₋₆ alkylene-N(R^{a})-C(=O)-C₁₋₈ alkyl, -C₁₋₆ alkylene-N(R^{a})-C₁₋₆ alkylene-(4-8 membered heterocyclyl), -C₁₋₆ alkylene-N(R^{a})-C₁₋₆ alkylene-(5-6 membered heteroaryl), -C₁₋₆ alkylene-N(R^{a})-C₁₋₆ alkylene-phenyl, -C₁₋₆ alkylene-O-C₁₋₈ alkyl, -C₁₋₆ alkylene-O-C₁₋₆ alkylene-C₃₋₆ cycloalkyl, -C₁₋₆ alkylene-O-C₁₋₆ alkylene-(4-8 membered heterocyclyl), -C₁₋₆ alkylene-O-C₁₋₆ alkylene-N(R^{a})(R^{b}), -S(=O)_{w}-C₁₋₈ alkyl, and -C(=O)-N(R^{a})-C₁₋₈ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}.

In some embodiments, R^{2a} is selected from H, D, -OH, -NH₂, C₁₋₆ alkyl, C₂₋₆ alkenyl, C₃₋₆ cycloalkyl, 5-6 membered heteroaryl, 4-8 membered heterocyclyl, -O-C₁₋₆ alkyl, -O-C₁₋₄ alkylene-C₃₋₆ cycloalkyl, -O-C₁₋₄ alkylene-(4-8 membered heterocyclyl), -O-C(=O)-N(R^{a})-C₁₋₆ alkyl, -O-C(=O)-N(R^{a})-phenyl, -N(R^{a})-C₁₋₆ alkyl, -N(R^{a})-C₃₋₆ cycloalkyl, -N(R^{a})-(4-8 membered heterocyclyl), -N(R^{a})-C(=O)-C₁₋₆ alkyl, -N(R^{a})-C(=O)-O-C₁₋₈ alkyl, -N(R^{a})-C₁₋₄ alkylene-C₃₋₆ cycloalkyl, -N(R^{a})-C₁₋₄ alkylene-Si(R^{c})₃, -N(R^{a})-(C=N(R^{b}))-C₁₋₆ alkyl, -N(R^{a})-S(=O)_{w}-C₁₋₆ alkyl, -N(R^{a})-C₁₋₄ alkylene-(4-8 membered heterocyclyl), -N(R^{a})-C₁₋₄ alkylene-(5-6 membered heteroaryl), -N(R^{a})-C₁₋₄ alkylene-phenyl, -C₁₋₄ alkylene-C₃₋₆ cycloalkyl, -C₁₋₄ alkylene-(4-8 membered heterocyclyl), -C₁₋₄ alkylene-N(R^{a})(R^{b}), -C₁₋₄ alkylene-N(R^{a})-C(=O)-O-C₁₋₆ alkyl, -C₁₋₄ alkylene-N(R^{a})-C₁₋₆ alkyl, -C₁₋₄ alkylene-N(R^{a})-C₁₋₄ alkylene-C₃₋₆ cycloalkyl, -C₁₋₄ alkylene-N(R^{a})-C(=O)-C₁₋₆ alkyl, -C₁₋₄ alkylene-N(R^{a})-C₁₋₄ alkylene-(4-8 membered heterocyclyl), -C₁₋₄ alkylene-N(R^{a})-C₁₋₄ alkylene-(5-6 membered heteroaryl), -C₁₋₄ alkylene-N(R^{a})-C₁₋₄ alkylene-phenyl, -C₁₋₄ alkylene-O-C₁₋₆ alkyl, -C₁₋₄ alkylene-O-C₁₋₄ alkylene-C₃₋₆ cycloalkyl, -C₁₋₄ alkylene-O-C₁₋₄ alkylene-(4-8 membered heterocyclyl), -C₁₋₄ alkylene-O-C₁₋₄ alkylene-N(R^{a})(R^{b}), -S(=O)_{w}-C₁₋₆ alkyl, and -C(=O)-N(R^{a})-C₁₋₆ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}.

In some embodiments, R^{2a} is selected from -O-C₁₋₆ alkyl, -O-C₁₋₄ alkylene-C₃₋₆ cycloalkyl, -O-C₁₋₄ alkylene-(4-8 membered heterocyclyl), -N(R^{a})-C₁₋₆ alkyl, -N(R^{a})-C₃₋₆ cycloalkyl, -N(R^{a})-(4-8 membered heterocyclyl), -N(R^{a})-C(=O)-C₁₋₆ alkyl, -N(R^{a})-C₁₋₄ alkylene-C₃₋₆ cycloalkyl, -N(R^{a})-C₁₋₄ alkylene-(4-8 membered heterocyclyl), - N(R^{a})-C₁₋₄ alkylene-(5-6 membered heteroaryl), -N(R^{a})-C₁₋₄ alkylene-phenyl, -C₁₋₄ alkylene-C₃₋₆ cycloalkyl, -C₁₋₄ alkylene-(4-8 membered heterocyclyl), -C₁₋₄ alkylene-N(R^{a})(R^{b}), -C₁₋₄ alkylene-N(R^{a})-C₁₋₆ alkyl, -C₁₋₄ alkylene-N(R^{a})-C₁₋₄ alkylene-C₃₋₆ cycloalkyl, -C₁₋₄ alkylene-N(R^{a})-C(=O)-C₁₋₆ alkyl, -C₁₋₄ alkylene-N(R^{a})-C₁₋₄ alkylene-(4-8 membered heterocyclyl), -C₁₋₄ alkylene-N(R^{a})-C₁₋₄ alkylene-(5-6 membered heteroaryl), -C₁₋₄ alkylene-N(R^{a})-C₁₋₄ alkylene-phenyl, -C₁₋₄ alkylene-O-C₁₋₆ alkyl, -C₁₋₄ alkylene-O-C₁₋₄ alkylene-C₃₋₆ cycloalkyl, -C₁₋₄ alkylene-O-C₁₋₄ alkylene-(4-8 membered heterocyclyl), -C₁₋₄ alkylene-O-C₁₋₄ alkylene-N(R^{a})(R^{b}), -S(=O)_{w}-C₁₋₆ alkyl, and -C(=O)-N(R^{a})-C₁₋₆ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}.

In some embodiments, R^{2a} is selected from -N(R^{a})-C₁₋₆ alkyl, -N(R^{a})-C₃₋₆ cycloalkyl, -N(R^{a})-(4-8 membered heterocyclyl), -N(R^{a})-C(=O)-C₁₋₆ alkyl, -N(R^{a})-C₁₋₄ alkylene-C₃₋₆ cycloalkyl, -N(R^{a})-C₁₋₄ alkylene-(4-8 membered heterocyclyl), - N(R^{a})-C₁₋₄ alkylene-(5-6 membered heteroaryl), and -N(R^{a})-C₁₋₄ alkylene-phenyl, wherein the substitutable carbon atoms of R^{2a} are each independently and optionally substituted by one or more substituents each independently selected from R^{g}.

In some embodiments, R^{2a} is selected from -N(R^{a})-C₁₋₆ alkyl, -N(R^{a})-C₁₋₆ haloalkyl, and -N(R^{a})-C₁₋₄ alkylene-C₃₋₆ cycloalkyl.

In some embodiments, R^{2a} is selected from the following groups:

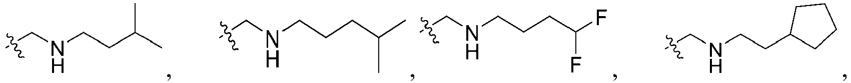

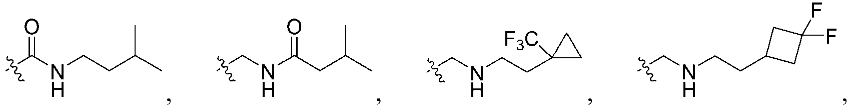

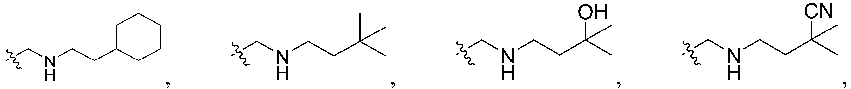

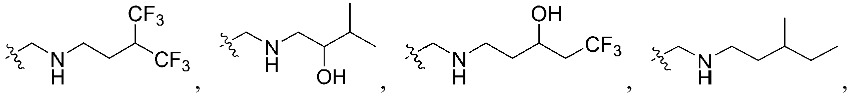

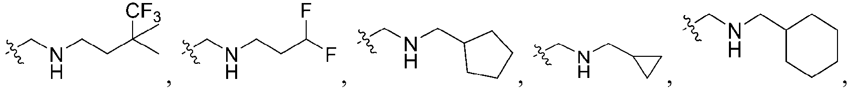

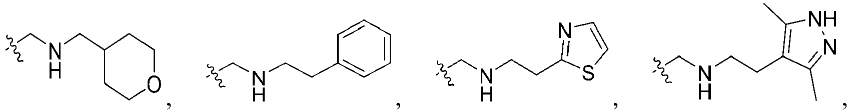

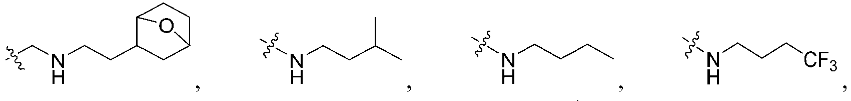

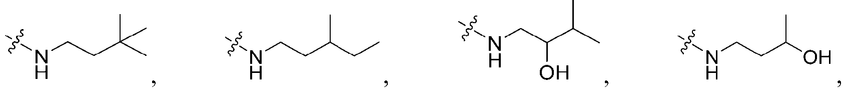

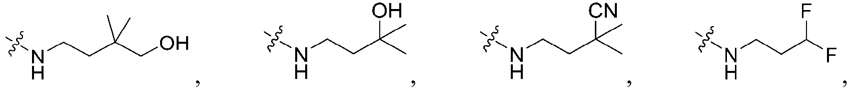

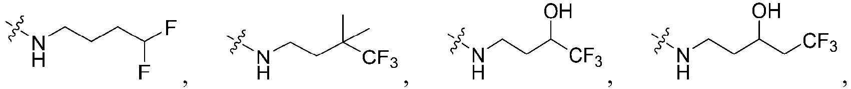

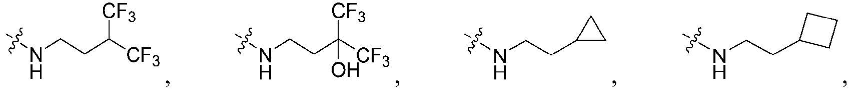

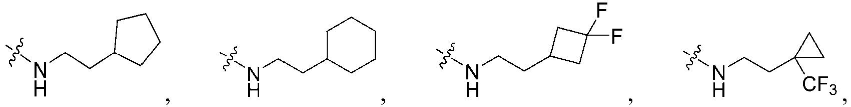

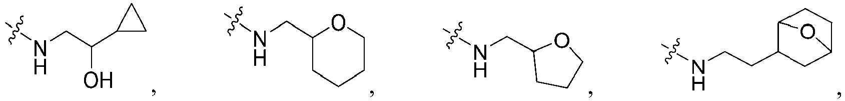

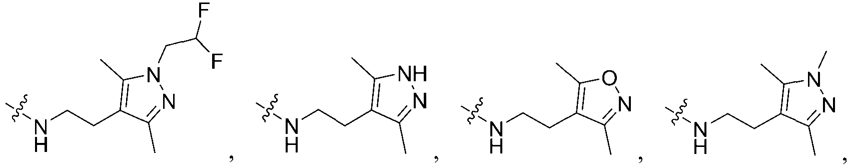

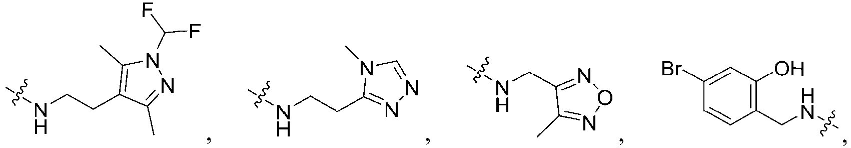

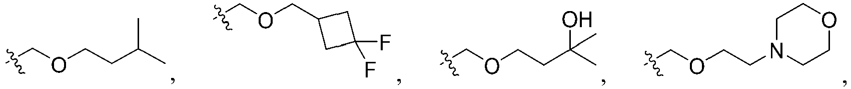

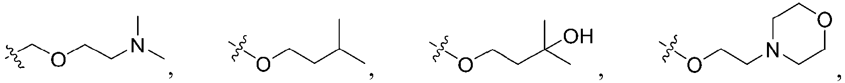

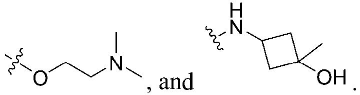

In some embodiments, R^{2a} is selected from the following groups:

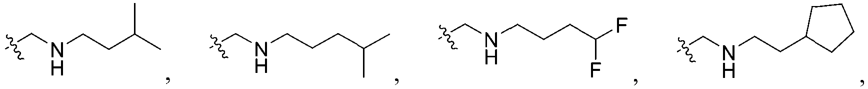

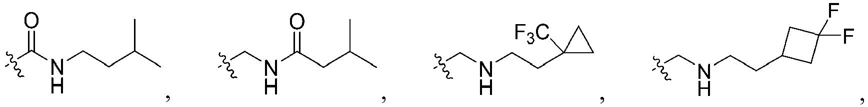

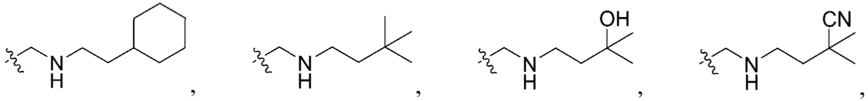

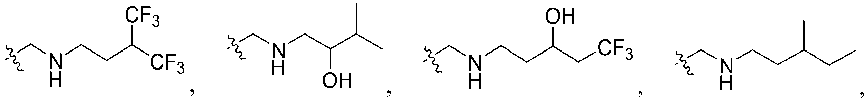

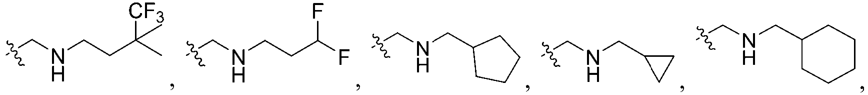

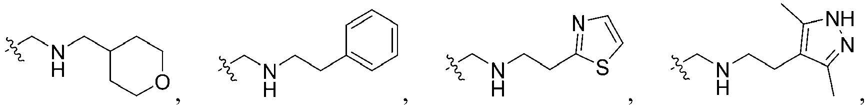

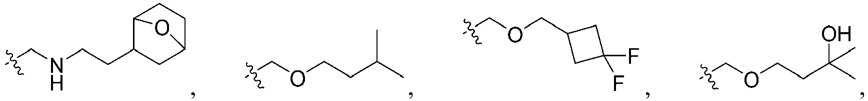

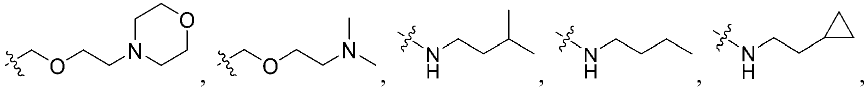

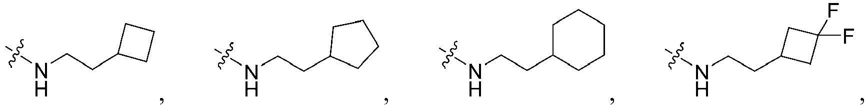

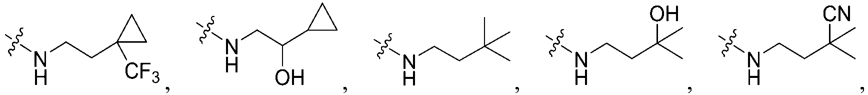

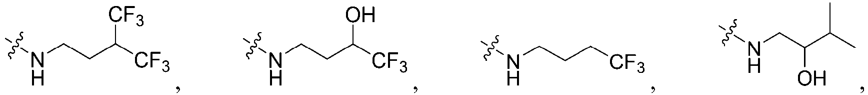

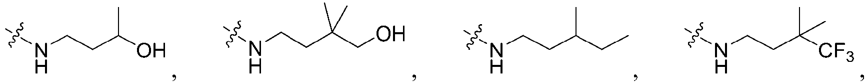

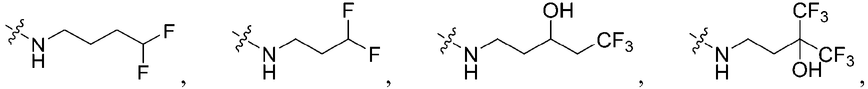

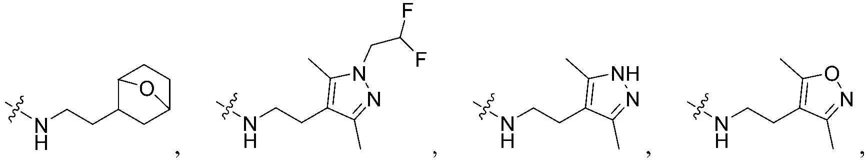

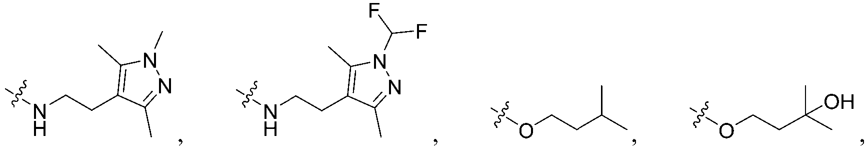

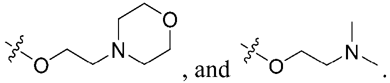

In some embodiments, R^{2a} is selected from the following groups:

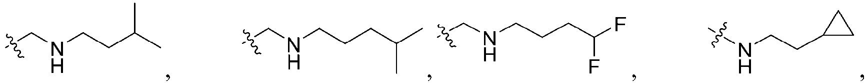

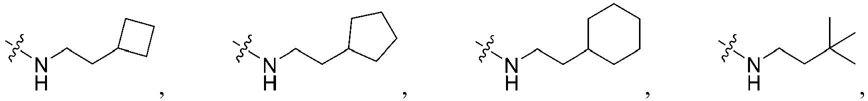

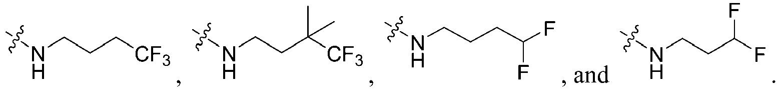

In some embodiments, R^{2a} is selected from the following groups:

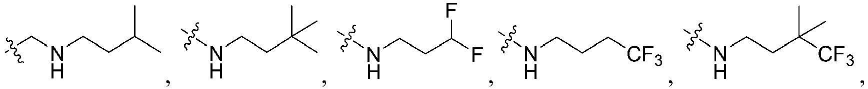

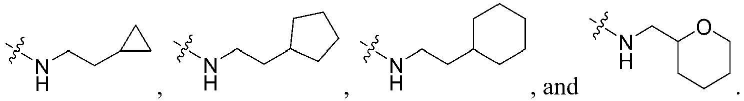

In some embodiments, R^{2a} is selected from the following groups:

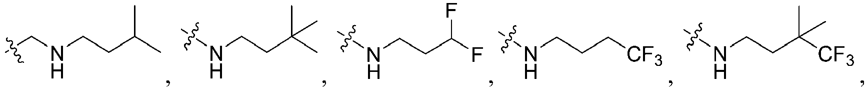

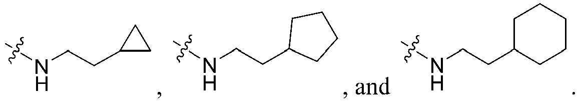

In some embodiments, R^{2a} is

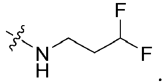

In some embodiments, R^{2b} is selected from H, D, -OH, halogen, -N(R^{a})(R^{b}), and - N(R^{a})-N(R^{b})-C(O)-phenyl.

In some embodiments, R^{2b} is selected from H, D, -OH, F, Cl, and Br.

In some embodiments, R^{2b} is selected from H and D.

In some embodiments, R^{2b} is H.

In some embodiments, R³, R^{3a}, and R^{3b} are each independently selected from H, D, halogen, -OH, C₁₋₆ alkyl, C₁₋₆ haloalkyl, C₁₋₆ alkoxy, C₁₋₆ haloalkoxy, C₃₋₆ cycloalkyl, C₃₋₆ halocycloalkyl, -NH₂, -NH(C₁₋₆ alkyl), and -N(C₁₋₆ alkyl)(C₁₋₆ alkyl).

In some embodiments, R³, R^{3a}, and R^{3b} are each independently selected from H, D, halogen, -OH, C₁₋₄ alkyl, C₁₋₄ haloalkyl, C₁₋₄ alkoxy, C₁₋₄ haloalkoxy, C₃₋₆ cycloalkyl, C₃₋₆ halocycloalkyl, -NH₂, -NH(C₁₋₄ alkyl), and -N(C₁₋₄ alkyl)(C₁₋₄ alkyl).

In some embodiments, R³, R^{3a}, and R^{3b} are each independently selected from H, D, F, Cl, Br, -OH, C₁₋₄ alkyl, and C₁₋₄ haloalky.

In some embodiments, R³, R^{3a}, and R^{3b} are each independently selected from H, D, F, Cl, Br, -OH, methyl, ethyl, trifluoromethyl, and difluoromethyl.

In some embodiments, R³, R^{3a}, and R^{3b} are each independently selected from H, D, F, and -OH.

In some embodiments, R³, R^{3a}, and R^{3b} are each independently selected from H, F, and -OH.

In some embodiments, X¹ is selected from O, S, NR^{f}, and C(R^{e})(R^{d}).

In some embodiments, R^{a} and R^{b} are each independently selected from H and C₁₋₆ alkyl; and optionally, the C₁₋₆ alkyl is substituted by one or more substituents selected from halogen, -CN, oxo, and -OH.

In some embodiments, R^{a} and R^{b} are each independently selected from H and C₁₋₄ alkyl; and optionally, the C₁₋₄ alkyl is substituted by one or more substituents selected from halogen, -CN, oxo, and -OH.

In some embodiments, R^{a} and R^{b} are each independently selected from H, and methyl.

In some embodiments, R^{c} is selected from -OH, C₁₋₆ alkyl, and phenyl.

In some embodiments, R^{c} is selected from -OH, methyl, ethyl, n-propyl, isopropyl, n-butyl, tert-butyl, and phenyl.

In some embodiments, R^{e} and R^{d} are each independently selected from H, D, halogen, -OH, C₁₋₆ alkyl, C₁₋₆ haloalkyl, C₁₋₆ alkoxy, C₁₋₆ haloalkoxy, C₃₋₆ cycloalkyl, C₃₋₆ halocycloalkyl, -NH₂, -NH(C₁₋₆ alkyl), and -N(C₁₋₆ alkyl)(C₁₋₆ alkyl); optionally, R^{e} and R^{3a} together with the atoms to which they connect form a 3-7 membered carbocyclic ring; or R^{e} and R^{2b} together with the atoms to which they connect form a 3-7 membered carbocyclic ring.

In some embodiments, R^{e} and R^{d} are each independently selected from H, D, F, Cl, Br, -OH, C₁₋₄ alkyl, C₁₋₄ haloalkyl, C₁₋₄ alkoxy, C₁₋₄ haloalkoxy, C₃₋₆ cycloalkyl, C₃₋₆ halocycloalkyl, -NH₂, -NH(C₁₋₄ alkyl), and -N(C₁₋₄ alkyl)(C₁₋₄ alkyl); optionally, R^{e} and R^{3a} together with the atoms to which they connect form a 3-6 membered carbocyclic ring; or R^{e} and R^{2b} together with the atoms to which they connect form a 3-6 membered carbocyclic ring.

In some embodiments, R^{f} is selected from H, C₁₋₆ alkyl, and C₁₋₆ haloalkyl; optionally, R^{f} and R^{3a} together with the atoms to which they connect form a 3-7 membered heterocyclic ring; or R^{f} and R^{2b} together with the atoms to which they connect form a 3-7 membered heterocyclic ring.

In some embodiments, R^{f} is selected from H, C₁₋₄ alkyl, and C₁₋₄ haloalkyl; optionally, R^{f} and R^{3a} together with the atoms to which they connect form a 3-6 membered heterocyclic ring; or R^{f} and R^{2b} together with the atoms to which they connect form a 3-6 membered heterocyclic ring.

In some embodiments, R^{g} is independently selected from H, D, halogen, -OH, - CN, nitro, oxo, -NH₂, -NH(C₁₋₆ alkyl), -N(C₁₋₆ alkyl)(C₁₋₆ alkyl), C₁₋₆ alkyl, and C₁₋₆ haloalkyl.

In some embodiments, R^{g} is independently selected from H, D, F, Cl, Br, -OH, - CN, nitro, oxo, -NH₂, -NH(C₁₋₄ alkyl), -N(C₁₋₄ alkyl)(C₁₋₄ alkyl), C₁₋₄ alkyl, and C₁₋₄ haloalkyl.

In some embodiments, R⁸ is independently selected from H, D, F, Cl, Br, -OH, - CN, nitro, oxo, -NH₂, -NH-CH₃, -N(CH₃)(CH₃), methyl, ethyl, trifluoromethyl, and difluoromethyl.

In some embodiments, R^{h} is selected from H, D, C₁₋₆ alkyl, and C₁₋₆ haloalkyl. In some embodiments, R^{h} is selected from H, D, C₁₋₄ alkyl, and C₁₋₄ haloalkyl.

In some embodiments, R^{h} is selected from H, D, methyl, trifluoromethyl, difluoromethyl, and 2,2-difluoroethyl.

In some embodiments, R^{h} is selected from H, methyl, trifluoromethyl, difluoromethyl, and 2,2-difluoroethyl.

In some embodiments, n is selected from 0 or 1.

In some embodiments, p is selected from 0, 1, 2, or 3.

In some embodiments, q is selected from 0, 1, 2, 3, or 4.

In some embodiments, w is selected from 1 or 2.

In some embodiments, the compound having a structure represented by formula (1-1) is not

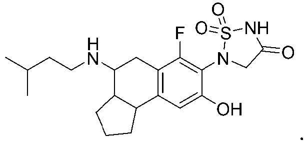

In some embodiments, R^{1a} and R^{1b} are each independently selected from H and D, and preferably, R^{1a} and R^{1b} are selected from H.

In some embodiments, R^{1c} and R^{1d} are each independently selected from H, D, and halogen, preferably, R^{1c} and R^{1d} are each independently selected from H and halogen, more preferably, one of R^{1c} and R^{1d} is selected from H, and the other is selected from halogen.

In some embodiments, p is selected from 0 or 1, and preferably, p is selected from 0.

In some embodiments, n is selected from 0.

In some embodiments, R³ is selected from H, D, and halogen, preferably, R³ is selected from H and halogen, and more preferably, R³ is selected from H.

In some embodiments, q is selected from 0, 1, or 2, and preferably, q is selected from 0.

In some embodiments, X¹ is selected from O, S, NR^{f}, and C(R^{e})(R^{d}), and preferably, X¹ is selected from C(R^{e})(R^{d}).

In some embodiments, R^{e} and R^{d} are each independently selected from H, D, halogen, and -OH, or R^{e} and R^{2b} together with the atoms to which they connect form a 3-7 membered carbocyclic ring; preferably, R^{e} and R^{d} are each independently selected from H, halogen, and OH, or R^{e} and R^{2b} together with the atoms to which they connect form a 3-6 membered carbocyclic ring (such as C₃₋₆ cycloalkyl), and more preferably, R^{e} and R^{d} are selected from H.

In some embodiments, R^{2b} is selected from H or R^{2b} and R^{e} together with the atoms to which they connect form a 3-6 membered carbocyclic ring (such as C₃₋₆ cycloalkyl), and more preferably, R^{2b} is selected from H.

In some embodiments, R^{2a} is selected from -N(R^{a})-C₁₋₈ alkyl, -N(R^{a})-C₃₋₆ cycloalkyl, -N(R^{a})-C₁₋₆ alkylene-C₃₋₆ cycloalkyl, -N(R^{a})-C₁₋₆ alkylene-(4-8 membered heterocyclyl), -N(R^{a})-C₁₋₆ alkylene-(5-6 membered heteroaryl), -N(R^{a})-C₁₋₆ alkylene-phenyl, -O-C₁₋₈ alkyl, -O-C₁₋₆ alkylene-(4-8 membered heterocyclyl), -O-C₁₋₆ alkylene-C₃₋₆ cycloalkyl, -N(R^{a})-(4-8 membered heterocyclyl), -C₁₋₆ alkylene-N(R^{a})-C₁₋₈ alkyl, -C₁₋₆ alkylene-N(R^{a})-C₁₋₆ alkylene-C₃₋₆ cycloalkyl, -C₁₋₆ alkylene-N(R^{a})-C₁₋₆ alkylene-(4-8 membered heterocyclyl), -C₁₋₆ alkylene-N(R^{a})-C₁₋₆ alkylene-(5-6 membered heteroaryl), -C₁₋₆ alkylene-N(R^{a})-C₁₋₆ alkylene-phenyl, -C₁₋₆ alkylene-O-C₁₋₈ alkyl, -C₁₋₆ alkylene-O-C₁₋₆ alkylene-C₃₋₆ cycloalkyl, -C₁₋₆ alkylene-O-C₁₋₆ alkylene-(4-8 membered heterocyclyl), -C(=O)-N(R^{a})-C₁₋₈ alkyl, -C₁₋₆ alkylene-N(R^{a})-C(=O)-C₁₋₈ alkyl, -C₁₋₆ alkylene-N(R^{a})(R^{b}), -C₁₋₆ alkylene-O-C₁₋₆ alkylene-N(R^{a})(R^{b}), and -N(R^{a})-C(=O)-C₁₋₈ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}.

In some embodiments, R^{2a} is selected from -N(R^{a})-C₁₋₆ alkyl, -N(R^{a})-C₃₋₆ cycloalkyl, -N(R^{a})-C₁₋₄ alkylene-C₃₋₆ cycloalkyl, -N(R^{a})-C₁₋₄ alkylene-(4-7 membered heterocyclyl), -N(R^{a})-C₁₋₄ alkylene-(5-6 membered heteroaryl), -N(R^{a})-C₁₋₄ alkylene-phenyl, -O-C₁₋₆ alkyl, -O-C₁₋₄ alkylene-(4-7 membered heterocyclyl), -O-C₁₋₄ alkylene-C₃₋₆ cycloalkyl, -N(R^{a})-(4-7 membered heterocyclyl), -C₁₋₄ alkylene-N(R^{a})-C₁₋₆ alkyl, -C₁₋₄ alkylene-N(R^{a})-C₁₋₄ alkylene-C₃₋₆ cycloalkyl, -C₁₋₄ alkylene-N(R^{a})-C₁₋₄ alkylene-(4-7 membered heterocyclyl), -C₁₋₄ alkylene-N(R^{a})-C₁₋₄ alkylene-(5-6 membered heteroaryl), -C₁₋₄ alkylene-N(R^{a})-C₁₋₄ alkylene-phenyl, -C₁₋₄ alkylene-O-C₁₋₆ alkyl, -C₁₋₄ alkylene-O-C₁₋₄ alkylene-C₃₋₆ cycloalkyl, -C₁₋₄ alkylene-O-C₁₋₄ alkylene-(4-7 membered heterocyclyl), -C(=O)-N(R^{a})-C₁₋₆ alkyl, -C₁₋₄ alkylene-N(R^{a})-C(=O)-C₁₋₆ alkyl, -C₁₋₄ alkylene-N(R^{a})(R^{b}), -C₁₋₄ alkylene-O-C₁₋₄ alkylene-N(R^{a})(R^{b}), and -N(R^{a})-C(=O)-C₁₋₆ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}.

In some embodiments, R^{2a} is selected from -N(R^{a})-C₁₋₈ alkyl, -N(R^{a})-C₃₋₆ cycloalkyl, -N(R^{a})-C₁₋₆ alkylene-C₃₋₆ cycloalkyl, -N(R^{a})-C₁₋₆ alkylene-(4-8 membered heterocyclyl), -N(R^{a})-C₁₋₆ alkylene-(5-6 membered heteroaryl), -N(R^{a})-C₁₋₆ alkylene-phenyl, -O-C₁₋₈ alkyl, -O-C₁₋₆ alkylene-(4-8 membered heterocyclyl), -C₁₋₆ alkylene-N(R^{a})-C₁₋₈ alkyl, -C₁₋₆ alkylene-N(R^{a})-C₁₋₆ alkylene-C₃₋₆ cycloalkyl, -C₁₋₆ alkylene-N(R^{a})-C₁₋₆ alkylene-(4-8 membered heterocyclyl), -C₁₋₆ alkylene-N(R^{a})-C₁₋₆ alkylene-(5-6 membered heteroaryl), -C₁₋₆ alkylene-N(R^{a})-C₁₋₆ alkylene-phenyl, -C₁₋₆ alkylene-O-C₁₋₈ alkyl, -C₁₋₆ alkylene-O-C₁₋₆ alkylene-C₃₋₆ cycloalkyl, -C₁₋₆ alkylene-O-C₁₋₆ alkylene-(4-8 membered heterocyclyl), -C(=O)-N(R^{a})-C₁₋₈ alkyl, and -C₁₋₆ alkylene-N(R^{a})-C(=O)-C₁₋₈ alkyl, wherein the substitutable carbon atoms of R^{2a} are each independently and optionally substituted by one or more substituents each independently selected from R^{g}, and/or the substitutable nitrogen atoms on the ring of R^{2a} are each independently and optionally substituted by one or more substituents each independently selected from R^{h}.

In some embodiments, R^{2a} is selected from -N(R^{a})-C₁₋₆ alkyl, -N(R^{a})-C₃₋₆ cycloalkyl, -N(R^{a})-C₁₋₄ alkylene-C₃₋₆ cycloalkyl, -N(R^{a})-C₁₋₄ alkylene-(4-7 membered heterocyclyl), -N(R^{a})-C₁₋₄ alkylene-(5-6 membered heteroaryl), -N(R^{a})-C₁₋₄ alkylene-phenyl, -O-C₁₋₆ alkyl, -O-C₁₋₄ alkylene-(4-7 membered heterocyclyl), -C₁₋₄ alkylene-N(R^{a})-C₁₋₆ alkyl, -C₁₋₄ alkylene-N(R^{a})-C₁₋₄ alkylene-C₃₋₆ cycloalkyl, -C₁₋₄ alkylene-N(R^{a})-C₁₋₄ alkylene-(4-7 membered heterocyclyl), -C₁₋₄ alkylene-N(R^{a})-C₁₋₄ alkylene-(5-6 membered heteroaryl), -C₁₋₄ alkylene-N(R^{a})-C₁₋₄ alkylene-phenyl, -C₁₋₄ alkylene-O-C₁₋₆ alkyl, -C₁₋₄ alkylene-O-C₁₋₄ alkylene-C₃₋₆ cycloalkyl, -C₁₋₄ alkylene-O-C₁₋₄ alkylene-(4-7 membered heterocyclyl), -C(=O)-N(R^{a})-C₁₋₆ alkyl, and -C₁₋₄ alkylene-N(R^{a})-C(=O)-C₁₋₆ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}.

In some embodiments, R^{2a} is selected from -N(R^{a})-C₁₋₈ alkyl, -N(R^{a})-C₁₋₆ alkylene-C₃₋₆ cycloalkyl, -N(R^{a})-C₁₋₆ alkylene-(4-8 membered heterocyclyl), -N(R^{a})-C₁₋₆ alkylene-phenyl, and -C₁₋₆ alkylene-N(R^{a})-C₁₋₈ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}.

In some embodiments, R^{2a} is selected from -N(R^{a})-C₁₋₆ alkyl, -N(R^{a})-C₁₋₄ alkylene-C₃₋₆ cycloalkyl, -N(R^{a})-C₁₋₄ alkylene-(4-7 membered heterocyclyl), -N(R^{a})-C₁₋₄ alkylene-phenyl, and -C₁₋₄ alkylene-N(R^{a})-C₁₋₆ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}.

In some embodiments, R^{2a} is selected from -N(R^{a})-C₁₋₈ alkyl, -N(R^{a})-C₁₋₆ alkylene-C₃₋₆ cycloalkyl, -N(R^{a})-C₁₋₆ alkylene-(4-8 membered heterocyclyl), and -C₁₋₆ alkylene-N(R^{a})-C₁₋₈ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}.

In some embodiments, R^{2a} is selected from -N(R^{a})-C₁₋₆ alkyl, -N(R^{a})-C₁₋₄ alkylene-C₃₋₆ cycloalkyl, -N(R^{a})-C₁₋₄ alkylene-(4-7 membered heterocyclyl), and - C₁₋₄ alkylene-N(R^{a})-C₁₋₆ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}.

In some embodiments, R^{g} is independently selected from H, D, halogen, -OH, - CN, -NH₂, -NH(C₁₋₆ alkyl), -N(C₁₋₆ alkyl)(C₁₋₆ alkyl), C₁₋₆ alkyl, and C₁₋₆ haloalkyl.

In some embodiments, R^{g} is independently selected from H, D, halogen, -OH, - CN, -NH₂, -NH(C₁₋₄ alkyl), -N(C₁₋₄ alkyl)(C₁₋₄ alkyl), C₁₋₄ alkyl, and C₁₋₄ haloalkyl.

In some embodiments, R^{h} is selected from H, D, C₁₋₆ alkyl, and C₁₋₆ haloalkyl.

In some embodiments, R^{h} is selected from H, D, C₁₋₄ alkyl, and C₁₋₄ haloalkyl.

In some embodiments, R^{a} and R^{b} are each independently selected from H and C₁₋₆ alkyl; and optionally, the C₁₋₆ alkyl is substituted by one or more substituents selected from halogen.

In some embodiments, R^{a} and R^{b} are each independently selected from H and C₁₋₄ alkyl; and optionally, the C₁₋₄ alkyl is substituted by one or more substituents selected from halogen.

In some embodiments, the compound has a structure represented by formula (II-0):

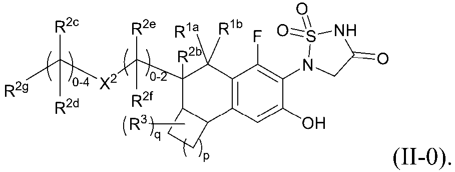

In some embodiments, the compound has a structure represented by formula (II-1) or (II-2): 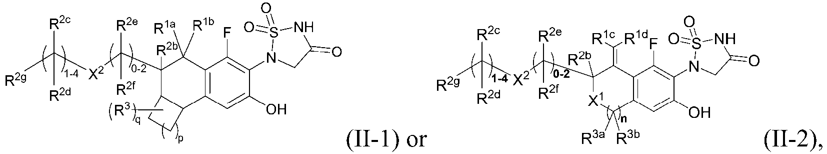 wherein R^{1a}, R^{1b}, R^{1c}, R^{1d}, R^{2b}, R^{2c}, R^{2d}, R^{2e}, R^{2f}, R^{2g}, R³, R^{3a}, R^{3b}, X¹, X², n, p, and q have the definitions described in the present invention.

In some embodiments, R^{2c}, R^{2d}, R^{2e} and R^{2f} are each independently selected from H, D, F, Cl, -CN, -OH, C₁₋₆ alkyl, and C₁₋₆ haloalkyl; optionally R^{2c} and R^{2d} form an oxo, or R^{2e} and R^{2f} form an oxo.

In some embodiments, R^{2c}, R^{2d}, R^{2e}, and R^{2f} are each independently selected from H, D, F, Cl, -CN, -OH, C₁₋₄ alkyl, and C₁₋₄ haloalkyl; and optionally, R^{2c} and R^{2d} form an oxo, or R^{2e} and R^{2f} form an oxo.

In some embodiments, R^{2c}, R^{2d}, R^{2e}, and R^{2f} are each independently selected from H, D, F, Cl, -CN, -OH, methyl, ethyl, and trifluoromethyl; and optionally, R^{2c} and R^{2d} form an oxo, or R^{2e} and R^{2f} form an oxo.

In some embodiments, R^{2g} is selected from H, C₁₋₄ alkyl, C₃₋₆ cycloalkyl, 4-8 membered heterocyclyl, 5-6 membered heteroaryl, phenyl, -NH₂, -NH(C₁₋₆ alkyl), and -N(C₁₋₆ alkyl)(C₁₋₆ alkyl); and the C₁₋₄ alkyl, C₃₋₆ cycloalkyl, 4-8 membered heterocyclyl, 5-6 membered heteroaryl, and phenyl are each independently and optionally substituted by one or more substituents selected from halogen, -OH, -CN, C₁₋₄ alkyl, and C₁₋₄ haloalkyl.

In some embodiments, R^{2g} is selected from H, C₁₋₄ alkyl, C₃₋₆ cycloalkyl, 4-8 membered heterocyclyl, 5-6 membered heteroaryl, phenyl, -NH₂, -NH(C₁₋₆ alkyl), and -N(C₁₋₆ alkyl)(C₁₋₆ alkyl); and the C₁₋₄ alkyl, C₃₋₆ cycloalkyl, 4-8 membered heterocyclyl, 5-6 membered heteroaryl, and phenyl are each independently and optionally substituted by one or more substituents selected from F, Cl, Br, -OH, -CN, methyl, trifluoromethyl, difluoromethyl, and 2,2-difluoroethyl.

In some embodiments, R^{2g} is selected from H, C₁₋₄ alkyl, C₃₋₆ cycloalkyl, 4-8 membered heterocyclyl, 5-6 membered heteroaryl, -NH₂, -NH(C₁₋₆ alkyl), and -N(C₁₋₆ alkyl)(C₁₋₆ alkyl); and the C₁₋₄ alkyl, C₃₋₆ cycloalkyl, 4-8 membered heterocyclyl, and 5-6 membered heteroaryl are each independently and optionally substituted by one or more substituents selected from F, Cl, Br, -OH, -CN, methyl, trifluoromethyl, difluoromethyl, and 2,2-difluoroethyl.

In some embodiments, R^{2g} is selected from H, C₁₋₄ alkyl, C₃₋₆ cycloalkyl, 4-7 membered heterocyclyl, 5-6 membered heteroaryl, and phenyl; and the C₁₋₄ alkyl, C₃₋₆ cycloalkyl, 4-7 membered heterocyclyl, 5-6 membered heteroaryl, and phenyl are each independently and optionally substituted by one or more substituents selected from halogen, -OH, -CN, C₁₋₄ alkyl, and C₁₋₄ haloalkyl.

In some embodiments, R^{2g} is selected from H, C₁₋₄ alkyl, C₃₋₆ cycloalkyl, and 4-7 membered heterocyclyl; and the C₁₋₄ alkyl, C₃₋₆ cycloalkyl, and 4-7 membered heterocyclyl are each independently and optionally substituted by one or more substituents selected from halogen.

In some embodiments, R^{2g} is selected from H, methyl, trifluoromethyl, difluoromethyl, tert-butyl, -CH₂OH, -N(CH₃)₂, or the following groups:

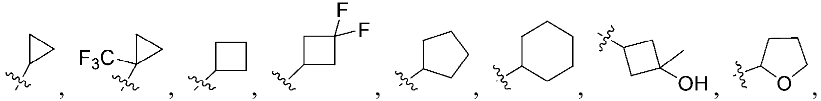

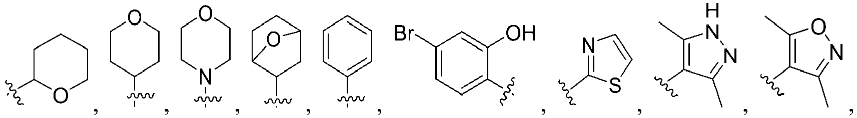

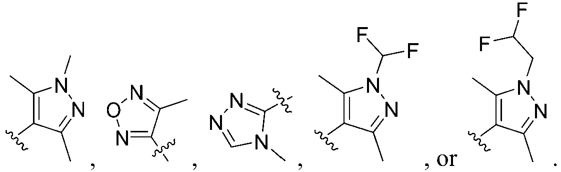

In some embodiments, R^{2g} is selected from H, methyl, trifluoromethyl, difluoromethyl, tert-butyl, -CH₂OH, -N(CH₃)₂, or the following groups:

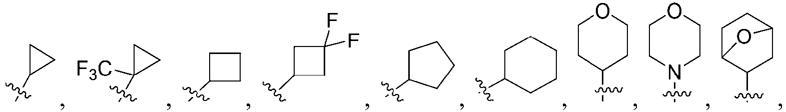

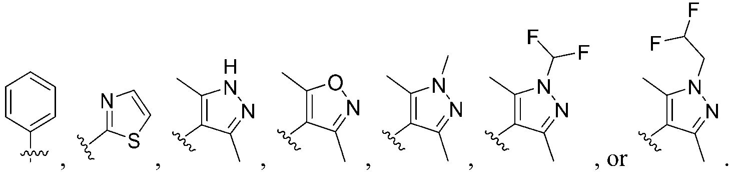

In some embodiments, R^{2g} is selected from H, methyl, tert-butyl, trifluoromethyl, cyclopropyl, cyclopentyl, cyclohexyl, and tetrahydropyran-2-yl.

In some embodiments, X² is NH or O.

In some embodiments, X² is NH.

In some embodiments, the compound has a structure represented by formula (III-0):

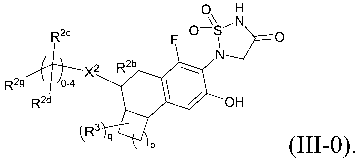

In some embodiments, the compound has a structure represented by formula (III-1) or (III-2): 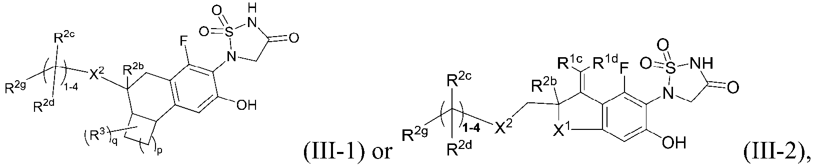 wherein R^{1c}, R^{1d}, R^{2b}, R^{2c}, R^{2d}, R^{2g}, R³, X¹, X², p, and q have the definitions described in the present invention.

In some embodiments, the compound has a structure represented by formula (IV-1) or (IV-2): 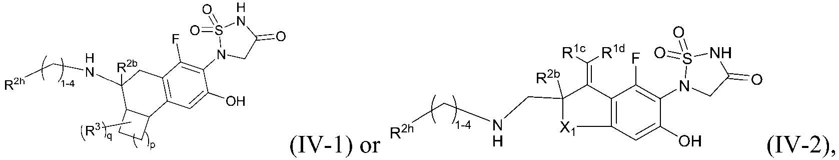 wherein R^{1c}, R^{1d}, R^{2b}, R^{2c}, R^{2h}, R³, X¹, p, and q have the definitions described in the present invention.

In some embodiments, R^{2h} is selected from C₁₋₆ alkyl, C₃₋₆ cycloalkyl, 4-7 membered heterocyclyl, 5-6 membered heteroaryl, phenyl, -C₁₋₄ alkylene-C₃₋₆ cycloalkyl, -C₁₋₄ alkylene-(4-7 membered heterocyclyl), -C₁₋₄ alkylene-(5-6 membered heteroaryl), -C₁₋₄ alkylene-phenyl, -NH(C₁₋₆ alkyl), and -N(C₁₋₆ alkyl)₂; and the C₁₋₆ alkyl, C₃₋₆ cycloalkyl, 4-7 membered heterocyclyl, 5-6 membered heteroaryl, phenyl, -C₁₋₄ alkylene-C₃₋₆ cycloalkyl, -C₁₋₄ alkylene-(4-7 membered heterocyclyl), - C₁₋₄ alkylene-(5-6 membered heteroaryl), -C₁₋₄ alkylene-phenyl, -NH(C₁₋₆ alkyl), and -N(C₁₋₆ alkyl)₂ are each independently and optionally substituted by one or more substituents selected from halogen, -OH, -CN, C₁₋₄ alkyl, and C₁₋₄ haloalkyl.

In some embodiments, R^{2h} is selected from C₁₋₆ alkyl, C₃₋₆ cycloalkyl, 4-7 membered heterocyclyl, 5-6 membered heteroaryl, phenyl, -C₁₋₂ alkylene-C₃₋₆ cycloalkyl, -C₁₋₂ alkylene-(4-7 membered heterocyclyl), -C₁₋₂ alkylene-(5-6 membered heteroaryl), -C₁₋₂ alkylene-phenyl, -NH(C₁₋₆ alkyl), and-N(C₁₋₆ alkyl)₂; and the C₁₋₆ alkyl, C₃₋₆ cycloalkyl, 4-7 membered heterocyclyl, 5-6 membered heteroaryl, phenyl, -C₁₋₂ alkylene-C₃₋₆ cycloalkyl, -C₁₋₂ alkylene-(4-7 membered heterocyclyl), - C₁₋₂ alkylene-(5-6 membered heteroaryl), -C₁₋₂ alkylene-phenyl, -NH(C₁₋₆ alkyl), and -N(C₁₋₆ alkyl)₂ are each independently and optionally substituted by one or more substituents selected from halogen, -OH, -CN, C₁₋₄ alkyl, and C₁₋₄ haloalkyl.

In some embodiments, R^{2h} is selected from C₁₋₆ alkyl, C₃₋₆ cycloalkyl, 4-7 membered heterocyclyl, 5-6 membered heteroaryl, phenyl, -C₁₋₂ alkylene-C₃₋₆ cycloalkyl, and -N(C₁₋₆ alkyl)₂; and the C₁₋₆ alkyl, C₃₋₆ cycloalkyl, 4-7 membered heterocyclyl, 5-6 membered heteroaryl, phenyl, -C₁₋₂ alkylene-C₃₋₆ cycloalkyl, and - N(C₁₋₆ alkyl)₂ are each independently and optionally substituted by one or more substituents selected from halogen, -OH, -CN, C₁₋₄ alkyl, and C₁₋₄ haloalkyl.

In some embodiments, R^{2h} is selected from C₁₋₆ alkyl, C₃₋₆ cycloalkyl, and 4-7 membered heterocyclyl; and the C₁₋₆ alkyl, C₃₋₆ cycloalkyl, and 4-7 membered heterocyclyl are each independently and optionally substituted by one or more substituents selected from halogen.

In some embodiments, R^{2h} is selected from C₁₋₆ alkyl, C₁₋₆ haloalkyl, and C₃₋₆ cycloalkyl.

In some embodiments, R^{2h} is selected from methyl, ethyl, isopropyl, tert-butyl, difluoromethyl, trifluoromethyl, 2-(1,1,1-trifluoromethyl)-propan-2-yl, cyclopropyl, cyclobutyl, cyclopentyl, cyclohexyl, phenyl, 2-hydroxy-propan-2-yl, 2-cyano-propan-2-yl, -N(CH₃)₂,

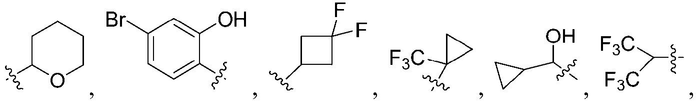

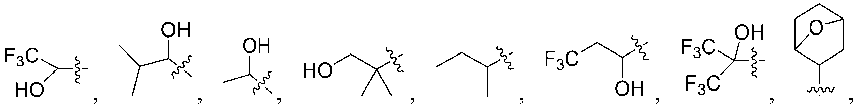

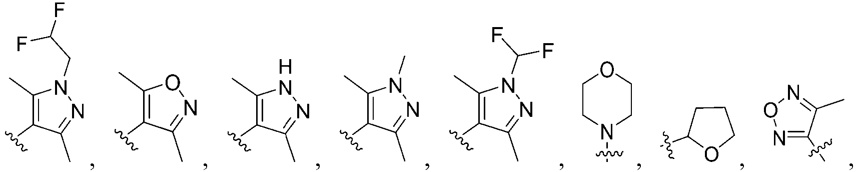

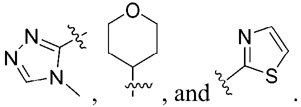

In some embodiments, R^{2h} is selected from methyl, ethyl, isopropyl, tert-butyl, difluoromethyl, trifluoromethyl, 2-(1,1,1-trifluoromethyl)-propan-2-yl, cyclopropyl, cyclobutyl, cyclopentyl, and cyclohexyl.

In some embodiments, R^{2h} is selected from isopropyl, tert-butyl, trifluoromethyl, 2-(1,1,1-trifluoromethyl)-propan-2-yl, cyclopropyl, cyclopentyl, cyclohexyl, and tetrahydropyran-2-yl.

In some embodiments, the compound has a structure represented by formula (V): 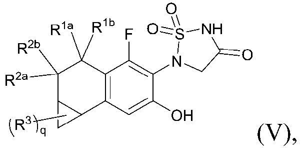 wherein R^{1a}, R^{1b}, R^{2a}, R^{2b}, R³, and q have the definitions described in the present invention.

In some embodiments, R^{1a} and R^{1b} are each independently selected from H, D, F, and -OH.

In some embodiments, R^{1a} and R^{1b} are H.

In some embodiments, R^{2a} is selected from -N(R^{a})-C₁₋₈ alkyl, -N(R^{a})-C₃₋₆ cycloalkyl, -N(R^{a})- (4-8 membered heterocyclyl), -N(R^{a})-C(=O)-C₁₋₈ alkyl, -N(R^{a})-C(=O)-O-C₁₋₈ alkyl, -N(R^{a})-C₁₋₆ alkylene-C₃₋₆ cycloalkyl, -N(R^{a})-C₁₋₆ alkylene-Si(R^{c})₃, -N(R^{a})-(C=N(R^{b}))-C₁₋₈ alkyl, -N(R^{a})-S(=O)_{w}-C₁₋₈ alkyl, -N(R^{a})-C₁₋₆ alkylene-(4-8 membered heterocyclyl), -N(R^{a})-C₁₋₆ alkylene-(5-6 membered heteroaryl), - N(R^{a})-C₁₋₆ alkylene-phenyl, -O-C₁₋₈ alkyl, -O-C₁₋₆ alkylene-C₃₋₆ cycloalkyl, -O-C₁₋₆ alkylene-(4-8 membered heterocyclyl), -O-C(=O)-N(R^{a})-C₁₋₈ alkyl, -O-C(=O)-N(R^{a})-phenyl, wherein the substitutable carbon atoms of R^{2a} are each independently and optionally substituted by one or more substituents each independently selected from R^{g}; R^{g} is independently selected from H, D, halogen, -OH, -CN, nitro, oxo, -NH₂, - NH(C₁₋₆ alkyl), -N(C₁₋₆ alkyl)(C₁₋₆ alkyl), C₁₋₆ alkyl, and C₁₋₆ haloalkyl; w is selected from 1 or 2; R^{a} and R^{b} are each independently selected from H and C₁₋₆ alkyl; and optionally, the C₁₋₆ alkyl is substituted by one or more substituents selected from halogen, -CN, oxo, and -OH.

In some embodiments, R^{2a} is selected from -N(R^{a})-C₁₋₆ alkyl, -N(R^{a})-C₃₋₆ cycloalkyl, -N(R^{a})- (4-8 membered heterocyclyl), -N(R^{a})-C(=O)-C₁₋₆ alkyl, -N(R^{a})-C(=O)-O-C₁₋₈ alkyl, -N(R^{a})-C₁₋₄ alkylene-C₃₋₆ cycloalkyl, -N(R^{a})-C₁₋₄ alkylene-Si(R^{c})₃, -N(R^{a})-(C=N(R^{b}))-C₁₋₆ alkyl, -N(R^{a})-S(=O)_{w}-C₁₋₆ alkyl, -N(R^{a})-C₁₋₄ alkylene-(4-8 membered heterocyclyl), -N(R^{a})-C₁₋₄ alkylene-(5-6 membered heteroaryl), - N(R^{a})-C₁₋₄ alkylene-phenyl, -O-C₁₋₆ alkyl, -O-C₁₋₄ alkylene-C₃₋₆ cycloalkyl, -O-C₁₋₄ alkylene-(4-8 membered heterocyclyl), -O-C(=O)-N(R^{a})-C₁₋₆ alkyl, and -O-C(=O)-N(R^{a})-phenyl, wherein the substitutable carbon atoms of R^{2a} are each independently and optionally substituted by one or more substituents each independently selected from R^{g}; R^{g} is independently selected from H, D, F, Cl, Br, -OH, -CN, nitro, oxo, - NH₂, -NH(C₁₋₄ alkyl), -N(C₁₋₄ alkyl)(C₁₋₄ alkyl), C₁₋₄ alkyl, and C₁₋₄ haloalkyl; w is selected from 1 or 2; R^{a} and R^{b} are each independently selected from H and C₁₋₄ alkyl; and optionally, the C₁₋₄ alkyl is substituted by one or more substituents selected from halogen, -CN, oxo, and -OH.

In some embodiments, R^{2a} is selected from -N(R^{a})-C₁₋₆ alkyl, -N(R^{a})-C₃₋₆ cycloalkyl, -N(R^{a})-C₁₋₄ alkylene-C₃₋₆ cycloalkyl, -N(R^{a})-C₁₋₄ alkylene-(4-8 membered heterocyclyl), -N(R^{a})-C₁₋₄ alkylene-(5-6 membered heteroaryl), -N(R^{a})-C₁₋₄ alkylene-phenyl, -O-C₁₋₆ alkyl, and -O-C₁₋₄ alkylene-(4-8 membered heterocyclyl), wherein the substitutable carbon atoms of R^{2a} are each independently and optionally substituted by one or more substituents each independently selected from R^{g}; and R^{g} is independently selected from H, F, -OH, -CN, -N(CH₃)(CH₃), methyl, ethyl, trifluoromethyl, and difluoromethyl; and R^{a} is H.

In some embodiments, R^{2a} is selected from -N(R^{a})-C₁₋₈ alkyl, -N(R^{a})-C₃₋₆ cycloalkyl, -N(R^{a})-C₁₋₆ alkylene-C₃₋₆ cycloalkyl, -N(R^{a})-C₁₋₆ alkylene-(4-8 membered heterocyclyl), -N(R^{a})-C₁₋₆ alkylene-(5-6 membered heteroaryl), -N(R^{a})-C₁₋₆ alkylene-phenyl, -O-C₁₋₈ alkyl, -O-C₁₋₆ alkylene-(4-8 membered heterocyclyl), -O-C₁₋₆ alkylene-C₃₋₆ cycloalkyl, and -N(R^{a})-(4-8 membered heterocyclyl), wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}.

In some embodiments, R^{2a} is selected from -N(R^{a})-C₁₋₆ alkyl, -N(R^{a})-C₃₋₆ cycloalkyl, -N(R^{a})-C₁₋₄ alkylene-C₃₋₆ cycloalkyl, -N(R^{a})-C₁₋₄ alkylene-(4-7 membered heterocyclyl), -N(R^{a})-C₁₋₄ alkylene-(5-6 membered heteroaryl), -N(R^{a})-C₁₋₄ alkylene-phenyl, -O-C₁₋₆ alkyl, -O-C₁₋₄ alkylene-(4-7 membered heterocyclyl), -O-C₁₋₄ alkylene-C₃₋₆ cycloalkyl, and -N(R^{a})-(4-7 membered heterocyclyl), wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}.

In some embodiments, R^{2a} is selected from -N(R^{a})-C₁₋₈ alkyl, -N(R^{a})-C₃₋₆ cycloalkyl, -N(R^{a})-C₁₋₆ alkylene-C₃₋₆ cycloalkyl, -N(R^{a})-C₁₋₆ alkylene-(4-8 membered heterocyclyl), -N(R^{a})-C₁₋₆ alkylene-(5-6 membered heteroaryl), -N(R^{a})-C₁₋₆ alkylene-phenyl, -O-C₁₋₈ alkyl, and -O-C₁₋₆ alkylene-(4-8 membered heterocyclyl), wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}.

In some embodiments, R^{2a} is selected from -N(R^{a})-C₁₋₆ alkyl, -N(R^{a})-C₃₋₆ cycloalkyl, -N(R^{a})-C₁₋₄ alkylene-C₃₋₆ cycloalkyl, -N(R^{a})-C₁₋₄ alkylene-(4-7 membered heterocyclyl), -N(R^{a})-C₁₋₄ alkylene-(5-6 membered heteroaryl), -N(R^{a})-C₁₋₄ alkylene-phenyl, -O-C₁₋₆ alkyl, and -O-C₁₋₄ alkylene-(4-7 membered heterocyclyl), wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}.

In some embodiments, R^{2a} is selected from -N(R^{a})-C₁₋₈ alkyl, -N(R^{a})-C₁₋₆ alkylene-C₃₋₆ cycloalkyl, and -N(R^{a})-C₁₋₆ alkylene-(4-8 membered heterocyclyl), wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}.

In some embodiments, R^{2a} is selected from -N(R^{a})-C₁₋₆ alkyl, -N(R^{a})-C₁₋₄ alkylene-C₃₋₆ cycloalkyl, and -N(R^{a})-C₁₋₄ alkylene-(4-7 membered heterocyclyl), wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}.

In some embodiments, R^{g} is independently selected from H, D, halogen, -OH, - CN, -NH₂, -NH(C₁₋₆ alkyl), -N(C₁₋₆ alkyl)(C₁₋₆ alkyl), C₁₋₆ alkyl, and C₁₋₆ haloalkyl.

In some embodiments, R^{g} is independently selected from H, D, F, Cl, Br, -OH, - CN, -NH₂, -NH(C₁₋₄ alkyl), -N(C₁₋₄ alkyl)(C₁₋₄ alkyl), C₁₋₄ alkyl, and C₁₋₄ haloalkyl.

In some embodiments, R^{g} is independently selected from H, F, -OH, -CN, - N(CH₃)₂, methyl, ethyl, trifluoromethyl, and difluoromethyl.

In some embodiments, R^{h} is selected from H, D, C₁₋₆ alkyl, and C₁₋₆ haloalkyl.

In some embodiments, R^{h} is selected from H, D, C₁₋₄ alkyl, and C₁₋₄ haloalkyl.

In some embodiments, R^{h} is selected from H, D, methyl, trifluoromethyl, difluoromethyl, and 2,2-difluoroethyl.

In some embodiments, R^{a} is selected from H and C₁₋₆ alkyl; and optionally, the C₁₋₆ alkyl is substituted by one or more substituents selected from halogen, -CN, oxo, and -OH.

In some embodiments, R^{a} is selected from H and C₁₋₄ alkyl; and optionally, the C₁₋₄ alkyl is substituted by one or more substituents selected from halogen, -CN, oxo, and -OH.

In some embodiments, R^{a} is H.

In some embodiments, R^{2b} is selected from H, D, -OH, F, Cl, and Br.

In some embodiments, R^{2b} is H.

In some embodiments, R³ is selected from H, D, halogen, and -OH.

In some embodiments, R³ is selected from H and F.

In some embodiments, q is selected from 0, 1, and 2.

In some embodiments, the compound has a structure represented by formula (V-1): 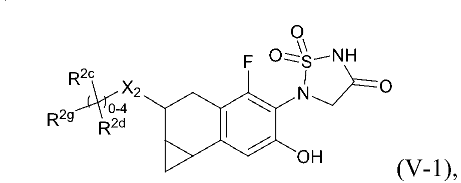
wherein R^{2c} and R^{2d} are each independently selected from H, D, F, Cl, -CN, -OH, C₁₋₆ alkyl, and C₁₋₆ haloalkyl; and optionally, R^{2c} and R^{2d} form an oxo;
R^{2g} is selected from H, C₁₋₄ alkyl, C₃₋₆ cycloalkyl, 4-8 membered heterocyclyl, phenyl, 5-6 membered heteroaryl, -NH₂, -NH(C₁₋₆ alkyl), and -N(C₁₋₆ alkyl)(C₁₋₆ alkyl); and the C₁₋₄ alkyl, C₃₋₆ cycloalkyl, 4-8 membered heterocyclyl, phenyl, 5-6 membered heteroaryl are each independently and optionally substituted by one or more substituents selected from halogen, -OH, -CN, C₁₋₄ alkyl, and C₁₋₄ haloalkyl; and
X² is NH or O.

In some embodiments, X² is NH.

In some embodiments, R^{2c} and R^{2d} are each independently selected from H, F, - CN, -OH, C₁₋₃ alkyl, and C₁₋₃ haloalkyl.

In some embodiments, R^{2c} and R^{2d} are each independently selected from H, F, - CN, -OH, methyl, ethyl, and trifluoromethyl.

In some embodiments, R^{2g} is selected from H, C₁₋₃ alkyl, C₃₋₆ cycloalkyl, 4-8 membered heterocyclyl, phenyl, and 5-6 membered heteroaryl (such as pyrazolyl, isoxazolyl, triazolyl, and oxadiazolyl); and the C₁₋₃ alkyl, C₃₋₆ cycloalkyl, 4-8 membered heterocyclyl, phenyl, and 5-6 membered heteroaryl are each independently and optionally substituted by one or more substituents selected from halogen, -OH, - CN, C₁₋₃ alkyl, and C₁₋₃ haloalkyl.

In some embodiments, R^{2g} is selected from H, C₁₋₃ alkyl, C₃₋₆ cycloalkyl, 4-7 membered heterocyclyl, phenyl, and 5-6 membered heteroaryl (such as pyrazolyl, isoxazolyl, triazolyl, and oxadiazolyl); and the C₁₋₃ alkyl, C₃₋₆ cycloalkyl, 4-7 membered heterocyclyl, phenyl, 5-6 membered heteroaryl are each independently and optionally substituted by one or more substituents selected from halogen, -OH, -CN, C₁₋₃ alkyl, and C₁₋₃ haloalkyl.

In some embodiments, R^{2g} is selected from H, F, methyl, ethyl, -CH(CH₃)₂, - CH₂OH, difluoromethyl, trifluoromethyl, -CH₂-CHF₂, cyclopropyl, 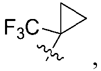 cyclobutyl, 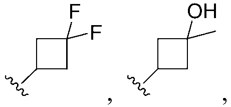 cyclopentyl, cyclohexyl, 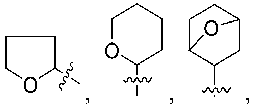 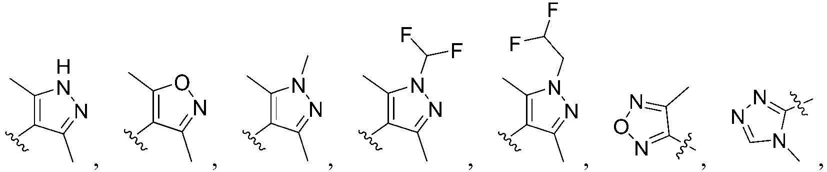 or

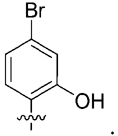

In some embodiments, R^{2c} and R^{2d} are H, and R^{2g} is difluoromethyl.

In some embodiments, the compound has a structure represented by formula (VI-0):

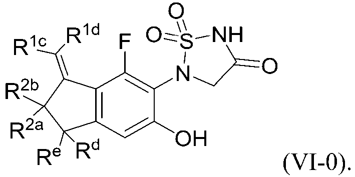

In some embodiments, R^{2a} is selected from -C₁₋₄ alkylene-N(R^{a})-C₁₋₆ alkyl, -C₁₋₄ alkylene-N(R^{a})-C₁₋₄ alkylene-C₃₋₆ cycloalkyl, -C₁₋₄ alkylene-N(R^{a})-C₁₋₄ alkylene-(4-7 membered heterocyclyl), -C₁₋₄ alkylene-N(R^{a})-C₁₋₄ alkylene-(5-6 membered heteroaryl), -C₁₋₄ alkylene-N(R^{a})-C₁₋₄ alkylene-phenyl, -C₁₋₄ alkylene-O-C₁₋₆ alkyl, -C₁₋₄ alkylene-O-C₁₋₄ alkylene-C₃₋₆ cycloalkyl, -C₁₋₄ alkylene-O-C₁₋₄ alkylene-(4-7 membered heterocyclyl), -C(=O)-N(R^{a})-C₁₋₆ alkyl, -C₁₋₄ alkylene-N(R^{a})-C(=O)-C₁₋₆ alkyl, -C₁₋₄ alkylene-N(R^{a})(R^{b}), -C₁₋₄ alkylene-O-C₁₋₄ alkylene-N(R^{a})(R^{b}), and -N(R^{a})-C(=O)-C₁₋₆ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}.

In some embodiments, R^{2a} is selected from -C₁₋₂ alkylene-N(R^{a})-C₁₋₆ alkyl, -C₁₋₂ alkylene-N(R^{a})-C₁₋₄ alkylene-C₃₋₆ cycloalkyl, -C₁₋₂ alkylene-N(R^{a})-C₁₋₄ alkylene-(4-7 membered heterocyclyl), -C₁₋₂ alkylene-N(R^{a})-C₁₋₄ alkylene-(5-6 membered heteroaryl), -C₁₋₂ alkylene-N(R^{a})-C₁₋₄ alkylene-phenyl, -C₁₋₂ alkylene-O-C₁₋₆ alkyl, -C₁₋₂ alkylene-O-C₁₋₄ alkylene-C₃₋₆ cycloalkyl, -C₁₋₂ alkylene-O-C₁₋₄ alkylene-(4-7 membered heterocyclyl), -C(=O)-N(R^{a})-C₁₋₆ alkyl, -C₁₋₂ alkylene-N(R^{a})-C(=O)-C₁₋₆ alkyl, and -C₁₋₂ alkylene-O-C₁₋₄ alkylene-N(R^{a})(R^{b}), wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}.

In some embodiments, R^{2a} is selected from -C₁₋₂ alkylene-N(R^{a})-C₁₋₆ alkyl, -C₁₋₂ alkylene-N(R^{a})-C₁₋₄ alkylene-C₃₋₆ cycloalkyl, -C₁₋₂ alkylene-N(R^{a})-C₁₋₄ alkylene-(4-7 membered heterocyclyl), -C₁₋₂ alkylene-N(R^{a})-C₁₋₄ alkylene-(5-6 membered heteroaryl), and -C₁₋₂ alkylene-N(R^{a})-C₁₋₄ alkylene-phenyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}.

In some embodiments, R^{2a} is selected from -C₁₋₂ alkylene-N(R^{a})-C₁₋₆ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}.

In some embodiments, R^{g} is independently selected from H, D, halogen (such as F, Cl, and Br), -OH, -CN, -NH₂, -NH(C₁₋₄ alkyl), -N(C₁₋₄ alkyl)(C₁₋₄ alkyl), C₁₋₄ alkyl, and C₁₋₄ haloalkyl.

In some embodiments, R^{h} is selected from H, D, C₁₋₄ alkyl, and C₁₋₄ haloalkyl.

In some embodiments, R^{a} and R^{b} are each independently selected from H and C₁₋₄ alkyl; and optionally, the C₁₋₄ alkyl is substituted by one or more substituents selected from halogen.

In some embodiments, the compound has a structure represented by formula (VI-1):

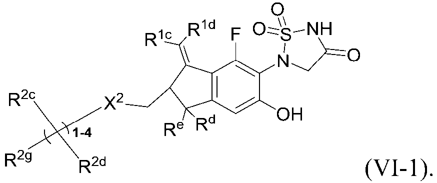

In some embodiments, the compound has a structure represented as follows:

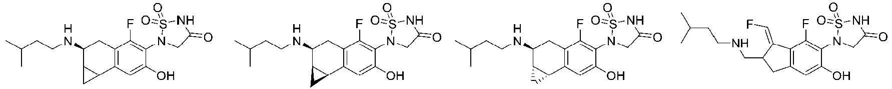

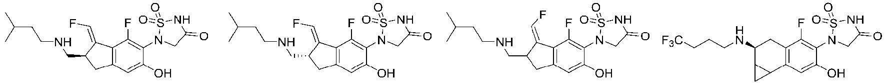

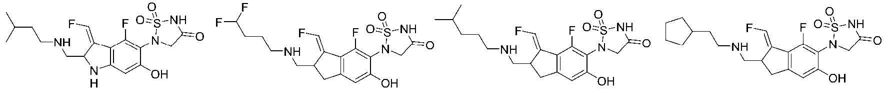

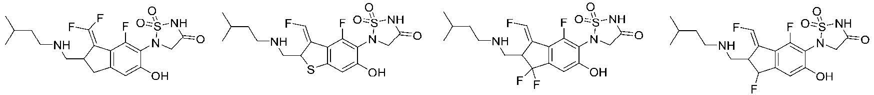

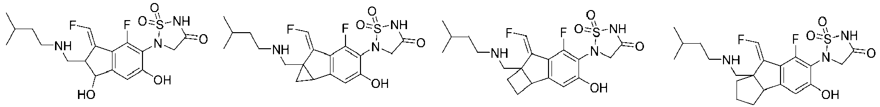

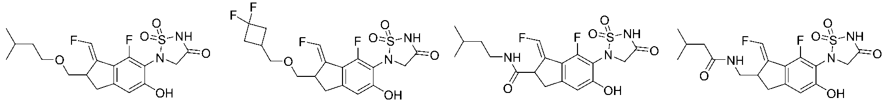

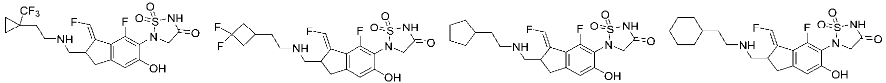

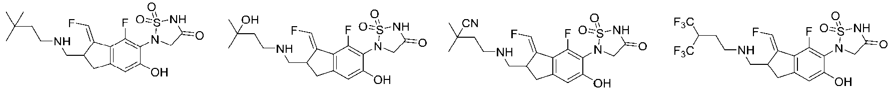

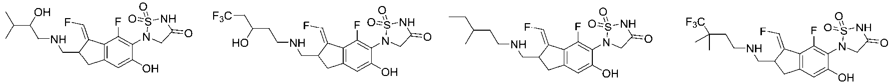

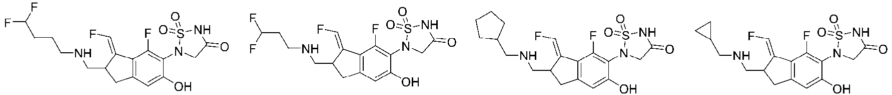

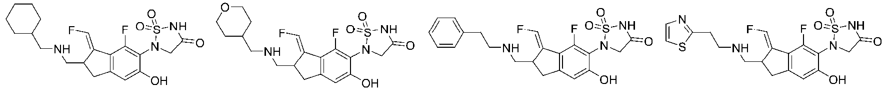

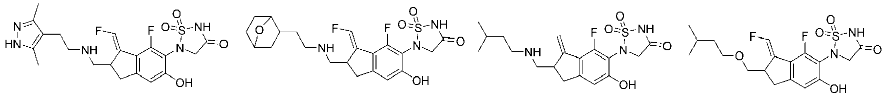

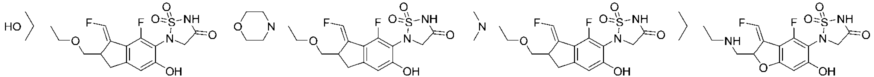

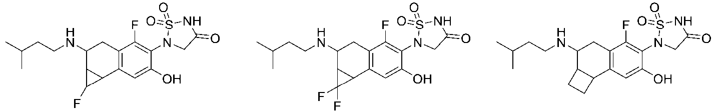

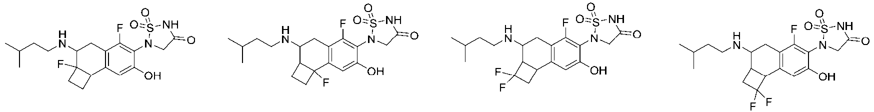

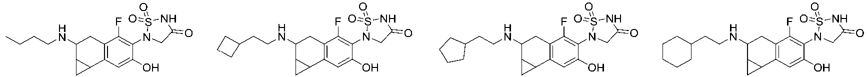

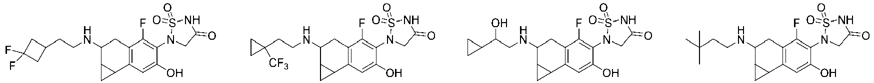

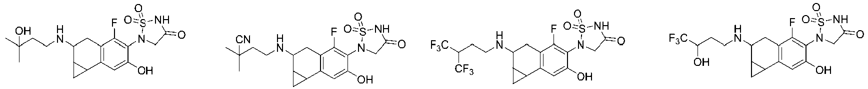

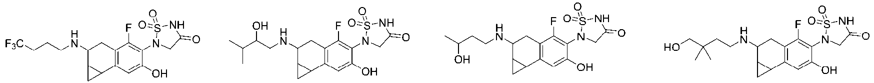

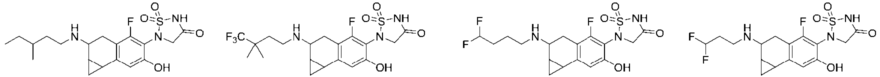

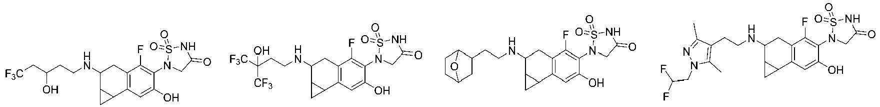

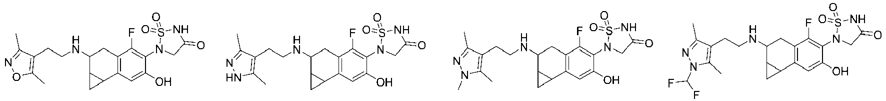

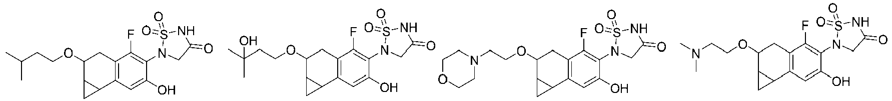

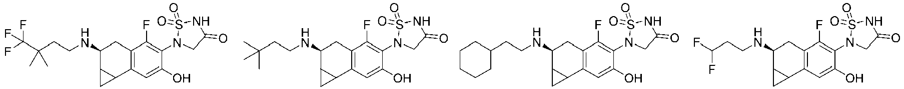

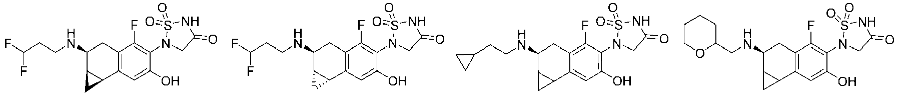

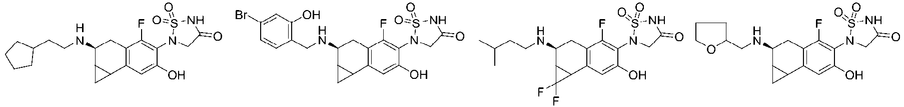

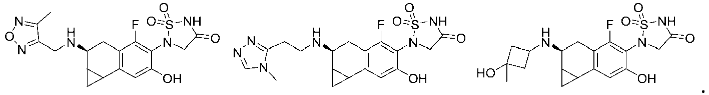

In some embodiments, the compound has a structure represented as follows:

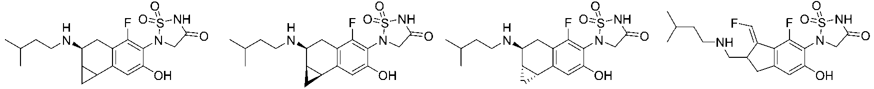

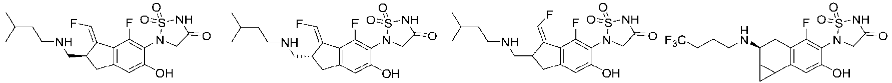

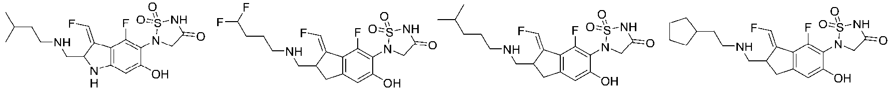

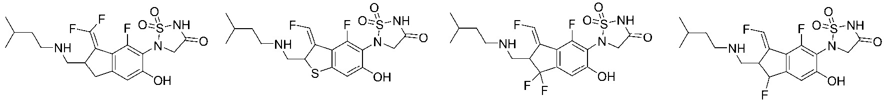

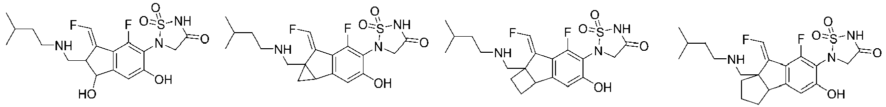

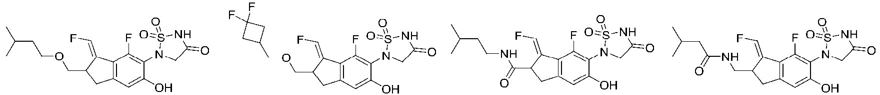

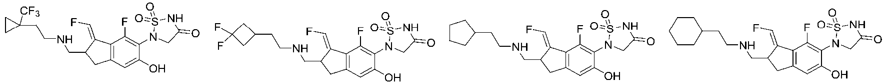

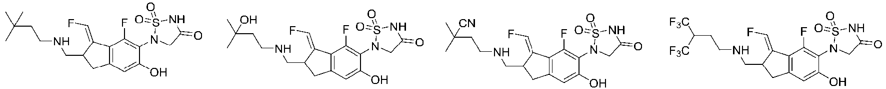

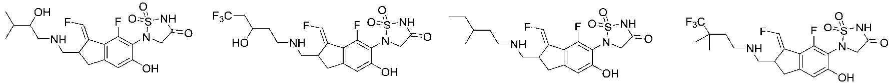

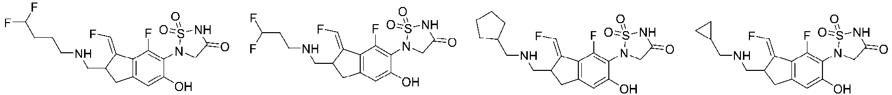

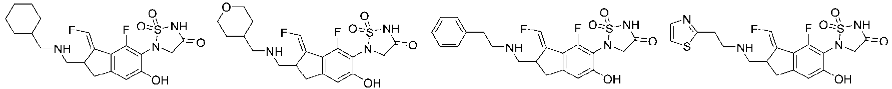

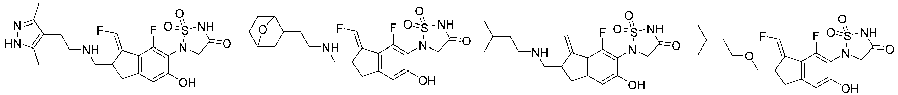

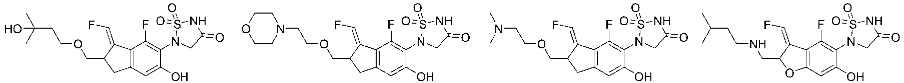

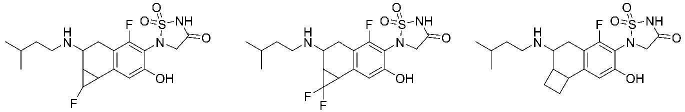

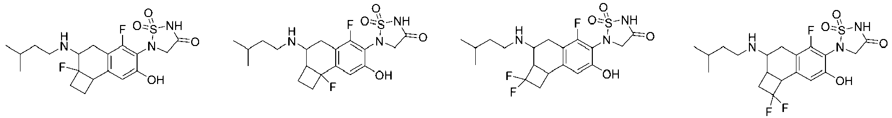

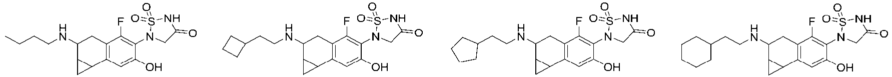

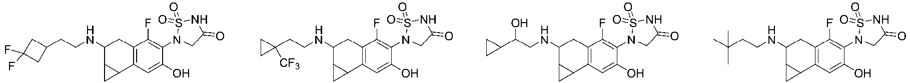

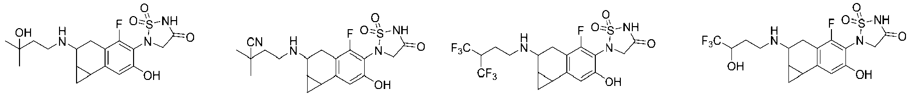

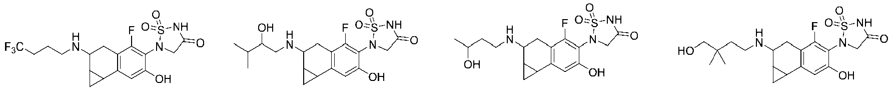

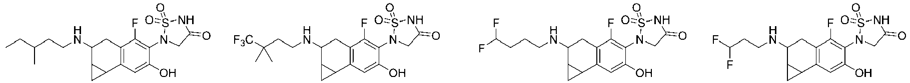

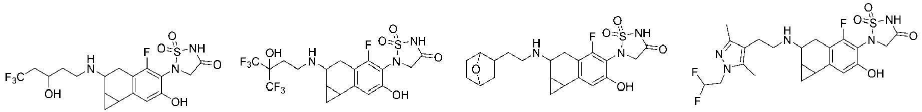

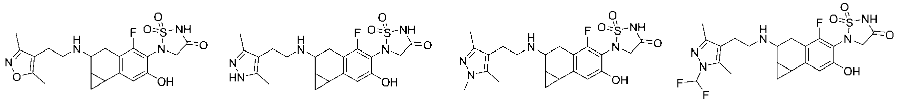

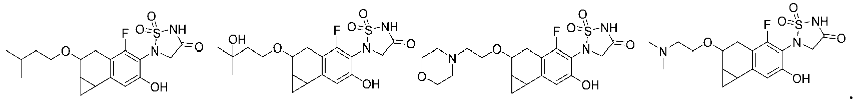

In some embodiments, the compound has a structure represented as follows:

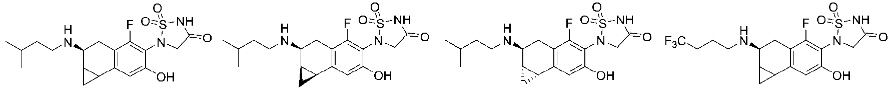

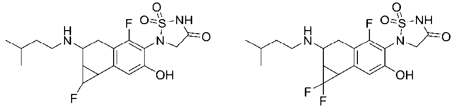

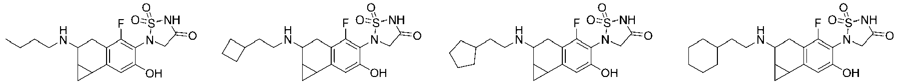

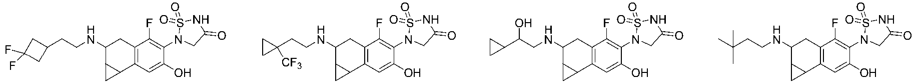

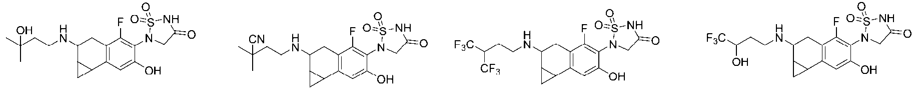

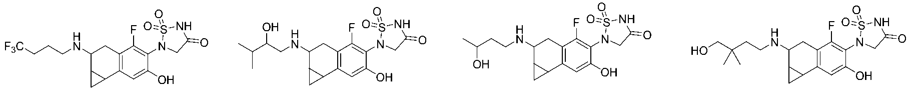

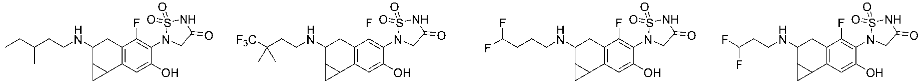

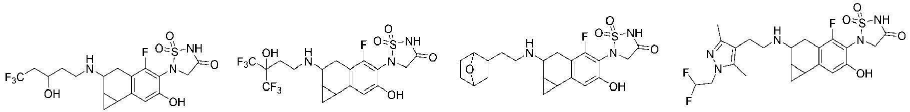

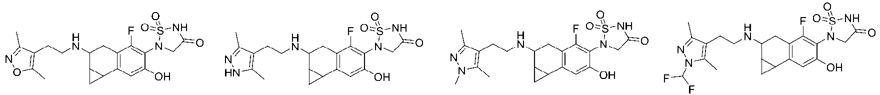

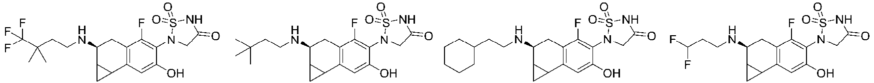

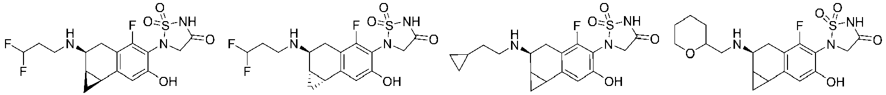

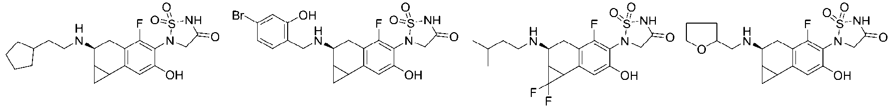

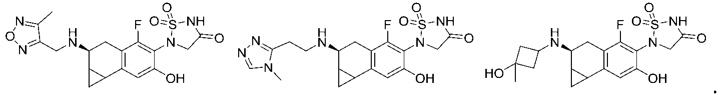

The present invention encompasses compounds obtained by any combination of the various embodiments.

### Pharmaceutical compositions and administration methods

The present invention relates to a pharmaceutical composition, which includes the compound or the enantiomer, diastereomer, racemate, tautomer, stereoisomer, geometric isomer, N-oxide, metabolite or pharmaceutically acceptable salt, ester, solvate, hydrate, isotope-labeled compound, labeled compound or prodrug thereof; and a pharmaceutically acceptable carrier.

The term "pharmaceutical composition" refers to a mixture of one or more compounds described herein, or physiologically/pharmaceutically acceptable salts or prodrugs thereof, with other chemical components, such as physiologically/pharmaceutically acceptable carriers and diluents, furthermore adjuvants such as excipients, binders, and fillers, and additional therapeutic agents such as antidiabetic agents, antihyperglycemic agents, antiobesity agents, antihypertensive agents, antiplatelet agents, antiatherosclerotic agents, or lipid-lowering agents. The purpose of the pharmaceutical composition is to facilitate the administration of the compound to an organism.

As used herein, the term "pharmaceutically acceptable carrier" refers to the substance that can be used to prepare or use a pharmaceutical composition, and includes, for example, suitable diluents, solvents, disperse medium, surfactants, antioxidants, preservatives, isotonic agents, buffers, emulsifiers, absorption retarders, salts, drug stabilizers, binders, excipients, disintegrants, lubricants, wetting agents, sweeteners, flavoring agents, dyes, and combinations thereof, as known to those skilled in the art (see, for example, Remington The Science and Practice of Pharmacy, 22nd ed., Pharmaceutical Press, 2013, pp. 1049-1070).

The present invention also relates to a compound with formula I-1, I-2, II-1, II-2, III-1, III-2, IV-1, or IV-2 as active ingredient or an enantiomer, diastereomer, racemate, tautomer, stereoisomer, geometric isomer, N-oxide, metabolite or pharmaceutically acceptable salt, ester, solvate, hydrate, isotope-labeled compound or prodrug thereof, or a pharmaceutical composition thereof, which may be used, in particular, for the treatment of neoplastic diseases, especially cancer, as described herein. The composition may be formulated for non-parenteral administration, such as nasal, oral, rectal, pulmonary, vaginal, sublingual, topical, transdermal, ophthalmic, or especially for oral administration, for example, in the form of oral solid dosage forms, such as granules, pills, powders, tablets, film-coated tablets or sugar-coated tablets, effervescent tablets, hard and soft capsules or hydroxypropyl methylcellulose (HPMC) capsules (suitably coated), orally disintegrating tablets, oral solutions, lipid emulsions or suspensions, or for parenteral administration, such as intravenous, intramuscular or subcutaneous, intrathecal, intradermal or epidural administration to mammals, especially humans, for example, in the form of solutions, lipid emulsions or suspensions containing microparticles or nanoparticles. These compositions may include the active ingredient alone, or preferably, together with a pharmaceutically acceptable carrier.

The compound with formula I-1, I-2, II-1, II-2, III-1, III-2, IV-1, or IV-2, or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof, can be processed with pharmaceutically inert inorganic or organic excipients to produce oral solid dosage forms, such as granules, pills, powders, tablets, film-coated tablets or sugar-coated tablets, effervescent tablets, hard capsules or HPMC capsules or orally disintegrating tablets. Fillers such as lactose, cellulose, mannitol, sorbitol, calcium phosphate, starch or derivatives thereof, binders such as cellulose, starch, polyvinylpyrrolidone or derivatives thereof, glidants such as talc, stearic acid or salts thereof, flowable agents such as fumed silica, can be used as such excipients for the preparation and manufacture of oral solid dosage forms, such as granules, pills, powders, tablets, film-coated tablets or sugar-coated tablets, effervescent tablets, hard capsules or HPMC capsules or orally disintegrating tablets. Suitable excipients for soft capsules are, for example, vegetable oils, waxes, fats, semisolid, and liquid polyols.

Suitable excipients for the manufacture of oral solutions, lipid emulsions or suspensions are, for example, water, alcohols, polyols, sucrose, invert sugar, and glucose.

Suitable excipients for parenteral formulations are, for example, water, alcohols, polyols, glycerol, vegetable oils, lecithin, and surfactants.

In addition, the pharmaceutical preparations may contain preservatives, solubilizers, stabilizers, wetting agents, emulsifiers, sweeteners, colorants, flavorings, salts for varying the osmotic pressure, buffers, masking agents or antioxidants. The pharmaceutical preparations may also contain other therapeutically valuable substances.

The dosage can vary within wide limits and, of course, meets individual requirements in each particular case. Generally, in the case of oral administration, a daily dosage of about 1 to 1000 mg of a compound with general formula I per person should be appropriate, although the above lower or upper limits may be exceeded if necessary.

The compounds with formula I-1, I-2, II-1, II-2, III-1, III-2, IV-1, or IV-2 can also be used in combination with one or more other pharmacologically active compounds that are also effective against the same disease, preferably using different modes of action, or reducing or preventing possible undesirable side effects of compounds with formula I-1, I-2, II-1, II-2, III-1, III-2, IV-1, or IV-2. Combination chaperones may be administered simultaneously in such treatment, for example by incorporating them into a single pharmaceutical formulation, or sequentially by administering two or more different dosage forms (each containing one or more combination chaperones).

The term "therapeutically effective amount" of a compound of the present invention refers to an amount of a compound of the present invention that will cause a biological or medical response in a subject (e.g., reduction or inhibition of enzyme or protein activity, or improvement of symptoms, alleviation of symptoms, slowing or delaying disease progression, or prevention of disease). In a non-limiting embodiment, the term "therapeutically effective amount" refers to an amount of a compound of the present invention that, when administered to a subject, is effective in at least partially alleviating, inhibiting, preventing and/or making improvements against any disease, disorder or condition related to PTPN2.

The term "treatment" or "treating" as used herein in the context of treating a disease or disorder generally relates to treatment and therapy of humans or animals (e.g., in veterinary applications), wherein some desired therapeutic effects are obtained, e.g., inhibiting the progression of a disease or disorder, and including reducing the rate of progression, stopping the rate of progression, alleviating the symptoms of a disease or disorder, making improvements against a disease or disorder, and curing a disease or disorder. It also includes treatment as a preventive measure (i.e., prevention). For example, patients who have not yet developed the disease or disorder but are at risk of developing the disease or disorder are covered by the term "treatment". For example, treatment includes prevention of cancer, reduction of cancer incidence, alleviation of cancer symptoms, etc.

In some embodiments, the present invention provides the compound with formula I-1, I-2, II-1, II-2, III-1, III-2, IV-1, or IV-2 or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof, or the pharmaceutical composition thereof, for use in treatment or prevention of a disease or disorder related to PTPN2.

In some embodiments, the present invention provides use of the compound with I-1, I-2, II-1, II-2, III-1, III-2, IV-1, or IV-2 or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof, or the pharmaceutical composition thereof, in manufacture of a medicament for treating or preventing a disease or disorder related to PTPN2.

In some embodiments, the present invention provides a method for treating or preventing PTPN2-related diseases or disorders, including administering a therapeutically effective amount of the compound with I-1, I-2, II-1, II-2, III-1, III-2, IV-1, or IV-2, or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof, or the pharmaceutical composition thereof.

In some embodiments, a disease or disorder related to PTPN2 includes cancer, type 2 diabetes, metabolic syndrome, obesity, or a metabolic disease.

In some embodiments, the cancer includes: carcinoma, sarcoma, adenocarcinoma, lymphoma, leukemia, and melanoma.

As used herein, the term "cancer" refers to all types of cancers, neoplasms or malignancies found in mammals, including leukemia, lymphoma, carcinoma, and sarcoma. Exemplary cancers that can be treated with the compounds, pharmaceutical compositions or methods provided herein include lymphoma, sarcoma, bladder cancer, bone cancer, brain tumors, cervical cancer, colon cancer, esophageal cancer, gastric cancer, head and neck cancer, kidney cancer, myeloma, thyroid cancer, leukemia, prostate cancer, breast cancer (e.g., ER positive, ER negative, chemotherapy resistance, herceptin resistance, HER2 positive, doxorubicin resistance, tamoxifen resistance, ductal carcinoma, lobular carcinoma, primary, and metastatic), ovarian cancer, pancreatic cancer, liver cancer (e.g., hepatocellular carcinoma), lung cancer (e.g., non-small cell lung cancer, squamous cell lung cancer, adenocarcinoma, large cell lung cancer, small cell lung cancer, carcinoid tumor, and sarcoma), glioblastoma multiforme, glioma or melanoma. Other examples include thyroid cancer, endocrine system cancer, brain cancer, breast cancer, cervical cancer, colon cancer, head and neck cancer, liver cancer, kidney cancer, lung cancer, non-small cell lung cancer, melanoma, mesothelioma, ovarian cancer, sarcoma, stomach cancer, uterine cancer or medulloblastoma, Hodgkin's disease, non-Hodgkin's lymphoma, multiple myeloma, neuroblastoma, glioma, glioblastoma multiforme, ovarian cancer, rhabdomyosarcoma, primary thrombocythemia, primary macroglobulinemia, primary brain tumor, cancer, malignant pancreatic insulinoma, malignant carcinoid tumor, bladder cancer, premalignant skin lesions, testicular cancer, lymphoma, thyroid cancer, neuroblastoma, esophageal cancer, genitourinary tract cancer, malignant hypercalcemia, endometrial cancer, adrenal cortical cancer, endocrine or exocrine pancreatic neoplasm, medullary thyroid cancer, medullary thyroid carcinoma, melanoma, colorectal cancer, papillary thyroid cancer, hepatocellular carcinoma, paget's disease of the nipple, phyllodes tumors, lobular carcinomas, ductal carcinomas, pancreatic stellate cell carcinomas, hepatic stellate cell carcinomas, or prostate cancer.

The term "carcinoma" refers to a malignant neoplasm composed of epithelial cells that tends to infiltrate surrounding tissues and cause metastases. Exemplary carcinomas that can be treated with the compounds, pharmaceutical compositions, or methods provided herein include, for example, medullary thyroid carcinoma, familial medullary thyroid carcinoma, adenocarcinoma, acinar carcinoma, adenoid cystic carcinoma, adenoma carcinoma, adrenocortical carcinoma, alveolar carcinoma, alveolar cell carcinoma, basal cell carcinoma, basaloid carcinoma, basosquamous cell carcinoma, bronchoalveolar carcinoma, bronchial carcinoma, bronchogenic carcinoma, encephalic carcinoma, cholangiocellular carcinoma, choriocarcinoma, colloid carcinoma, comedo carcinoma, uterine corpus carcinoma, cribriform carcinoma, armor carcinoma, cutaneous carcinoma, columnar carcinoma, columnar cell carcinoma, ductal carcinoma, tubular carcinoma, dural carcinoma, embryonal carcinoma, encephalic carcinoma, epidermoid carcinoma, epithelial adenocarcinoma, exophytic carcinoma, preulcerous carcinoma, fibrous carcinoma, colloid carcinoma, gelatinous carcinoma, giant cell carcinoma, giant cell carcinoma, adenocarcinoma, granulosa cell carcinoma, hair matrix carcinoma, hematogenous carcinoma, hepatocellular carcinoma, Hurthle cell carcinoma, clear carcinoma, adrenal carcinoma, infantile embryonal carcinoma, carcinoma in situ, intraepidermal carcinoma, intraepithelial carcinoma, Krompecher's carcinoma, Kulchitzky-cell carcinoma, large cell carcinoma, lenticular carcinoma, bean-shaped carcinoma, lipomatous carcinoma, lobular carcinoma, lymphoepithelial carcinoma, medullary carcinoma, medullary carcinoma, melanoma, nevus carcinoma, myxoid carcinoma, mucinous carcinoma, mucinous cell carcinoma, mucoepidermoid carcinoma, mucinous carcinoma, mucinous adenocarcinoma, myxomatous carcinoma, nasopharyngeal carcinoma, oat cell carcinoma, ossifying carcinoma, osteoid carcinoma, papillary carcinoma, periportal carcinoma, preinvasive carcinoma, bee-shaped epithelial carcinoma, erosive carcinoma, renal cell carcinoma, reserve cell carcinoma, sarcomatous carcinoma, Schneider's carcinoma, sclerosing carcinoma, scrotal carcinoma, signet ring cell carcinoma, simple carcinoma, small cell carcinoma, potato-shaped carcinoma, spheroid cell carcinoma, spindle cell carcinoma, cavernous carcinoma, squamous carcinoma, squamous cell carcinoma, linear carcinoma, telangiectatic carcinoma, telangiectatic carcinoma, transitional cell carcinoma, nodular carcinoma, tubular carcinoma, tuberous carcinoma, verrucous carcinoma, or villous carcinoma.

The term "sarcoma" generally refers to tumors that consists of embryonic connective tissue-like material and generally consists of tightly packed cells embedded in a fibrillary or homogenous material. Sarcomas that can be treated with the compounds, pharmaceutical compositions, or methods provided herein include chondrosarcoma, fibrosarcoma, lymphosarcoma, melanosarcoma, myxosarcoma, osteosarcoma, Abemethy's sarcoma, liposarcoma, liposarcoma, alveolar soft part sarcoma, ameloblastic sarcoma, botryoid sarcoma, green sarcoma, choriocarcinoma, embryonal sarcoma, Wilms' tumor sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, spontaneous multiple colored hemorrhagic sarcoma, B-cell immunoblastic sarcoma, lymphoma, T-cell immunoblastic sarcoma, Jensen's sarcoma, Kaposi's sarcoma, Kupffer cell sarcoma, angiosarcoma, leukemic sarcoma, malignant mesenchymal sarcoma, extraperiosteal sarcoma, reticular cell sarcoma, Rous sarcoma, serous cystic sarcoma, synovial sarcoma, or telangiectatic sarcoma.

The term "leukemia" refers broadly to progressive malignancies of the blood-forming organs and is generally characterized by abnormal proliferation and development of white blood cells and their precursors in the blood and bone marrow. Leukemia is generally classified clinically based on: (1) the duration and characteristics of the disease: acute or chronic; (2) the cell types involved: bone marrow (myelogenic), lymphoid (lymphogenic), or monocytic; and (3) the increase or absence of an increase in the number of abnormal cells in the blood: leukemic or non-leukemic (subleukemic). Exemplary leukemias that can be treated with the compounds, pharmaceutical compositions, or methods provided herein include, for example, acute non-lymphocytic leukemia, chronic lymphocytic leukemia, acute granulocytic leukemia, chronic granulocytic leukemia, acute promyelocytic leukemia, adult T-cell leukemia, non-leukemic leukemia, leukocytic leukemia, basophilic leukemia, blast cell leukemia, bovine leukemia, chronic myeloid leukemia, cutaneous leukemia, embryonic leukemia, eosinophilic leukemia, Gross'leukemia, hairy cell leukemia, hemocytoblastic leukemia, hemoblastic leukemia, histiocytic leukemia, stem cell leukemia, acute monocytic leukemia, leukopenic leukemia, lymphatic leukemia, lymphoblastic leukemia, lymphocytic leukemia, lymphogenic leukemia, lymphocyte-like leukemia, lymphosarcoma cell leukemia, mast cell leukemia, megakaryocytic leukemia, myeloblastic leukemia, monocytic leukemia, myeloblastic leukemia, myeloid leukemia, myelogranulocytic leukemia, myelomonocytic leukemia, Naegeli leukemia, plasma cell leukemia, multiple myeloma, plasma cell leukemia, promyelocytic leukemia, Rieder cell leukemia, Schilling's leukemia, stem cell leukemia, subleukemic leukemia or undifferentiated cell leukemia.

The term "melanoma" is considered to mean a tumor arising from the melanocyte system of the skin and other organs. Melanomas that can be treated with the compounds, pharmaceutical compositions, or methods provided herein include, for example, acral lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma, subungual melanoma, or superficial spreading melanoma.

In some embodiments, the cancer includes: solid and lymphatic cancers, kidney cancer, breast cancer, lung cancer, bladder cancer, colon cancer, ovarian cancer, prostate cancer, pancreatic cancer, stomach cancer, brain cancer, head and neck cancer, skin cancer, uterine cancer, testicular cancer, glioma, esophageal cancer, liver cancer (including liver tumors), lymphomas including B acute lymphoblastic lymphoma, non-Hodgkin's lymphomas (e.g., Burkitt's lymphomas, small cell lymphomas, and large cell lymphomas), Hodgkin's lymphoma, leukemias (including AML, ALL, and CML), and/or multiple myeloma.

In some embodiments, the cancer includes lung cancer, breast cancer, ovarian cancer, leukemia, lymphoma, melanoma, pancreatic cancer, sarcoma, bladder cancer, bone cancer, brain cancer, cervical cancer, colon cancer, esophageal cancer, stomach cancer, liver cancer, head and neck cancer, kidney cancer, myeloma, thyroid cancer, prostate cancer, metastatic cancer, or carcinoma.

### Synthesis method

Compounds with formula I-1, I-2, II-1, II-2, III-1, III-2, IV-1, or IV-2 can be synthesized by the methods given below, by the methods given in the experimental section below, or by similar methods. The schemes described herein are not intended to present an exhaustive list of methods for preparing compounds with formula I-1, I-2, II-1, II-2, III-1, III-2, IV-1, or IV-2; on the contrary, other techniques known to skilled chemists can also be used for compound synthesis.

The structure of the compound is determined by nuclear magnetic resonance (¹H-NMR, ¹³C-NMR and/or ¹⁹F-NMR). ¹H-NMR, ¹³C-NMR, and ¹⁹F-NMR chemical shift (δ) is given in units of parts per million (ppm). ¹H-NMR, ¹³C-NMR, and ¹⁹F-NMR were measured using the Bruker Ultrashield-400 NMR spectrometer and the Bruker Avance III HD600 NMR spectrometer, and the measurement solvents were deuterated chloroform (CDCl₃), deuterated methanol (CD₃OD or MeOH-d₄) or deuterated dimethyl sulfoxide (DMSO-d₆). TMS (0 ppm) or chloroform (7.25 ppm) was used as the reference standard. When multiple peaks appear, the following abbreviations are used: s (singlet), d (doublet), t (triplet), m (multiplet), br (broadened), dd (doublet of doublets), dt (doublet of triplets), td (triplet of doublets), and brs (broadened singlet). The coupling constant J is expressed in Hertz (Hz).

Liquid chromatography-mass spectrometry (LC-MS) was detected using an Agilent 1260 mass spectrometer, and HPLC was determined using an Agilent 1100 high pressure chromatograph (Microsorb 5 micron C18 100 x 3.0 mm column).

A Qingdao GF254 silica gel plate is used as the thin layer chromatography silica gel plate, 0.15-0.20 mm is used for TLC, and 0.4 mm-0.5 mm is used for preparative thin layer chromatography. For column chromatography, Qingdao silica gel 200-300 mesh silica gel is generally used as a carrier.

The starting materials in the examples of the present invention are all known and commercially available, or can be synthesized using or according to literature data reported in the art.

Unless otherwise specified, all reactions of the present invention are performed under the protection of dry inert gas (such as nitrogen or argon) with continuous magnetic stirring, and the reaction temperatures are all degrees Celsius.

It should be understood by those skilled in the art of organic synthesis that optimal reaction conditions may vary with the specific reactants or solvents used, but these conditions may be determined by conventional optimization procedures. In some cases, the order of the following reaction scheme and/or reaction steps may be changed to facilitate reaction or avoid forming unwanted by-products. In addition, the functional groups present at each position of the molecule must be compatible with the proposed reagent and reaction. This limitation on substituents compatible with reaction conditions is obvious to those skilled in the art, and then alternative methods must be used. Furthermore, in some reactions mentioned herein, it may be necessary or desirable to protect any sensitive groups in the compounds, and it is assumed that such a protecting group (PG) is in the appropriate position if necessary. Conventional protecting groups can be used according to standard practices well known in the art (for explanation, see Greene T.W, Wuts P.G.M, Protective Groups in Organic Synthesis, 5th edition, publisher: John Wiley & Sons, 2014). The protecting groups may be removed at any convenient stage in the synthesis using conventional techniques well known in the art, or may be removed in a subsequent reaction step or work-up.

The following abbreviations are used throughout the present invention.
LCMS: Liquid chromatography-mass spectrometry
M, mol/L: mole per liter
ml, mL: milliliter
g: gram
mmol: millimole
°C: degree Celsius

The following examples are provided to help understand the present invention. However, it should be understood that these examples and drawings are only used to illustrate the present invention, but do not constitute any limitation. The actual protection scope of the present invention is set forth in the claims. It should be understood that any modifications and changes can be made without departing from the spirit of the present invention.

### Preparation Example

### Example 1: Preparation of compounds 1, 2, and 3

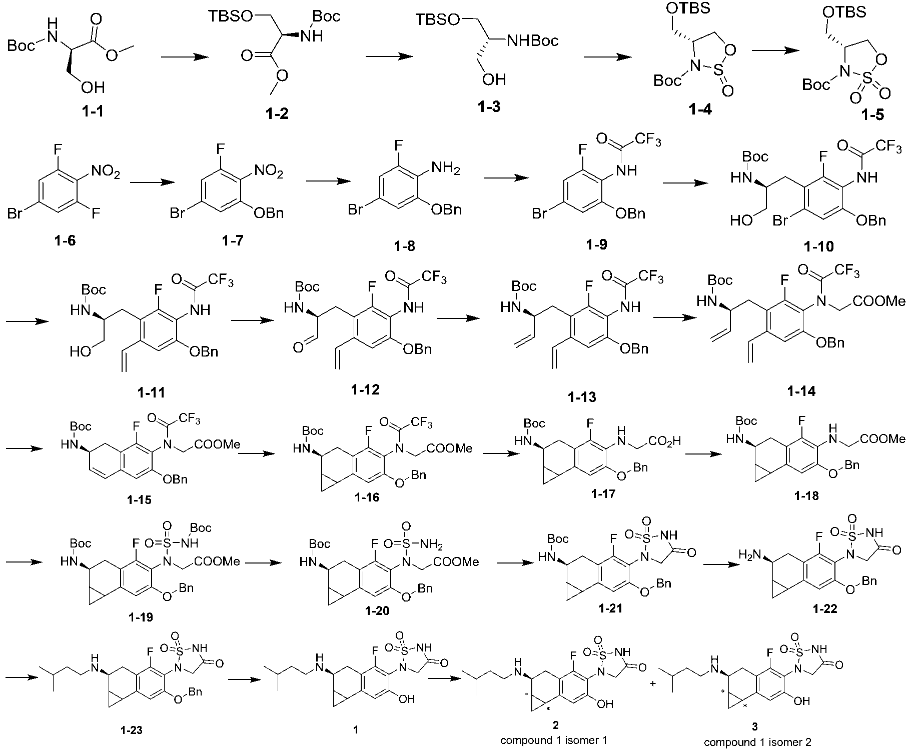

Step 1: Tert-butyldimethylsilyl chloride (11.3 g, 74.97 mmol) was added to the dichloromethane solution (200 mL) of compound 1-1 (15.7 g, 71.61 mmol) and imidazole (14.7 g, 215.92 mmol) at 0°C. The reaction solution was stirred at 0°C for 2 hours, and was diluted with water (200 mL) and extracted with dichloromethane (100 mL ×3). The combined organic phases were washed with 1 M dilute hydrochloric acid (100 mL) and saturated brine (100 mL), respectively, dried over anhydrous sodium sulfate and concentrated to obtain compound **1-2. ¹H NMR** (400 MHz, CDCl₃) δ 5.32 (d, *J* = 8.4 Hz, 1 H), 4.38 - 4.30 (m, 1 H), 4.03 (dd, *J* = 2.8*,* 10.0 Hz, 1 H), 3.81 (dd, *J* = 2.8, 10.0 Hz, 1 H), 3.73 (s, 3 H), 1.45 (s, 9 H), 0.86 (s, 9 H), 0.02 (d, *J* = 5.2 Hz, 6 H).

Step 2: Sodium borohydride (8.3 g, 219.40 mmol) was added to the mixture of tetrahydrofuran (200 mL) and ethanol (100 mL) of compound **1-2** (27 g, 72.86 mmol) and calcium chloride (16.2 g, 145.97 mmol) at 0°C. The mixture was stirred at 15°C for 12 hours. The reaction solution was slowly poured into water (300 mL) and diluted with ethyl acetate (200 mL). The mixture was filtered through celite and the filtrate was extracted with ethyl acetate (200 mL ×3), and the combined organic phases were washed with saturated brine (200 mL), dried over anhydrous sodium sulfate and concentrated to obtain compound **1-3. ¹H NMR** (400 MHz, CDCl₃) δ 5.13 (s, 1 H), 3.91 - 3.73 (m, 3 H), 3.73 - 3.56 (m, 2 H), 2.69 (s, 1 H), 1.45 (s, 9 H), 0.90 (s, 9 H), 0.07 (s, 6 H).

Step 3: Thionyl chloride (6.7 mL, 92.37 mmol) was added dropwise to the dichloromethane solution (100 mL) of imidazole (19.5 g, 286.43 mmol) and triethylamine (21.7 mL, 156.55 mmol) under nitrogen atmosphere at 0°C. The mixture was stirred at 0°C for 30 min and then the dichloromethane solution (100 mL) of compound **1-3** (21.7 g, 71.03 mmol) was added dropwise. The reaction solution was stirred at 0°C for 1 hour. The reaction solution was diluted with water (200 mL) and extracted with ethyl acetate (100 mL ×3). The combined organic phases were washed with brine (100 mL), dried over anhydrous sodium sulfate and concentrated to obtain compound **1-4,** which was directly used in the next step.

Step 4: Ruthenium(III) chloride (0.93 g, 3.56 mmol) and sodium periodate (22.8 g, 106.60 mmol) were added to the dichloromethane (120 mL) and water (120 mL) solution of compound **1-4** (25 g, 71.12 mmol). The reaction solution was stirred at 15°C for 12 hours. The reaction solution was filtered through celite and the filtrate was extracted with dichloromethane (100 mL x3). The organic phases were combined, washed with brine (100 mL), dried over anhydrous sodium sulfate and concentrated. The crude product was purified by silica gel column chromatography (ethyl acetate:petroleum ether = 1:5) to obtain compound **1-5. ¹H NMR** (400 MHz, CDCl₃) δ 4.65 - 4.55 (m, 2 H), 4.30 - 4.23 (m, 1 H), 3.87 (dd, *J* = 4.0*,* 10.0 Hz, 1 H), 3.78 (dd, *J* = 8.0, 10.0 Hz, 1 H), 1.55 (s, 9 H), 0.89 (s, 9 H), 0.09 (d, *J =* 2.8 Hz, 6 H).

Step 5: Potassium tert-butoxide solution (105 mL, 105.00 mmol, 1 M tetrahydrofuran solution) was added dropwise to the tetrahydrofuran (450 mL) solution of compound **1-6** (23.5 g, 98.74 mmol) and benzyl alcohol (10.8 mL, 103.87 mmol) under nitrogen atmosphere at -50°C. The mixture was stirred at -50°C for 20 min under nitrogen atmosphere. The reaction solution was quenched with saturated ammonium chloride solution (300 mL) and extracted with ethyl acetate (200 mL × 3). The organic phases were combined and washed with brine (200 mL), dried over anhydrous sodium sulfate and concentrated. The crude product was purified by silica gel column chromatography (ethyl acetate:petroleum ether = 1:20) to obtain compound **1-7. ¹H NMR** (400 MHz, DMSO-*d*₆) δ 7.63 (t, *J =* 1.6 Hz, 1 H), 7.59 (dd, *J* = 9.2, 1.6 Hz, 1 H), 7.47 - 7.33 (m, 5 H), 5.36 (s, 2 H).

Step 6: Saturated ammonium chloride solution (120 mL) was added to the tetrahydrofuran (230 mL) and methanol (230 mL) solution of compound **1-7** (23 g, 70.53 mmol) and zinc powder (14 g, 214.13 mmol). The reaction solution was stirred at 20°C for 2 hours. The reaction solution was diluted with ethyl acetate (200 mL) and filtered through celite. The filtrate was diluted with water (300 mL) and extracted with ethyl acetate (100 mL × 3). The organic phases were combined and washed with brine (100 mL), dried over anhydrous sodium sulfate and concentrated to obtain compound **1-8,** which was directly used in the next step.

Step 7: Trifluoroacetic anhydride (12.3 mL, 88.43 mmol) was added dropwise to the acetonitrile (200 mL) solution of compound **1-8** (20.2 g, 68.21 mmol) and pyridine (9 mL, 111.50 mmol) at 20°C. The reaction solution was stirred at 20°C for 1 hour. The reaction solution was diluted with water (300 mL) and extracted with ethyl acetate (100 mL × 3). The organic phases were combined and washed with brine (200 mL), dried over anhydrous sodium sulfate and concentrated. The crude product was purified by silica gel column chromatography (ethyl acetate:petroleum ether = 1:10) to obtain compound **1-9. ¹H NMR** (400 MHz, DMSO-*d*₆) δ 11.03 (s, 1 H), 7.48 - 7.27 (m, 7 H), 5.24 (s, 2 H).

Step 8: 2 M tetrahydrofuran solution of Lithium diisopropylamide was dropwise added to the tetrahydrofuran (100 mL) solution of compound **1-9** (18.5 g, 47.18 mmol) under nitrogen atmosphere at -70°C. The reaction solution was stirred at -70°C for 30 min followed by a tetrahydrofuran (100 mL) solution of compound **1-5** (15 g, 42.67 mmol) was added dropwise at -70°C. The reaction solution was stirred at -70°C for 2 hours. After 3 M dilute hydrochloric acid (86 mL) was added dropwise to the reaction solution, the reaction solution was stirred at 15°C for another 12 hours. The reaction solution was diluted with water (200 mL) and extracted with ethyl acetate (100 mL x3). The organic phases were combined and washed with brine (100 mL), dried over anhydrous sodium sulfate and concentrated. The crude product was purified by silica gel column chromatography (ethyl acetate: petroleum ether = 4:5) to obtain compound **1-10. MS m/z (ESI):** 465.2 [M-Boc+H]⁺. **¹H NMR** (400 MHz, DMSO-*d*₆) δ 11.03 (s, 1 H), 7.44 - 7.34 (m, 6 H), 6.44 (d, *J =* 9.2 Hz, 1 H), 5.19 (s, 2 H), 4.83 - 4.66 (m, 1 H), 3.79 - 3.69 (m, 1 H), 3.43 - 3.38 (m, 1 H), 2.90 - 2.73 (m, 2 H), 1.28 (s, 9 H).

Step 9: [1,1'-Bis(diphenylphosphino)ferrocene]dichloropalladium(II) (1 g, 1.37 mmol) was added to the 1,4-dioxane (150 mL) and water (15 mL) mixture of compound **1-10** (15 g, 26.53 mmol), potassium vinyltrifluoroborate (11 g, 82.12 mmol) and potassium carbonate (11 g, 79.59 mmol) under nitrogen atmosphere. The reaction solution was stirred at 100°C for 12 h under nitrogen atmosphere. The reaction solution was diluted with water (200 mL) and extracted with ethyl acetate (100 mL × 3). The organic phases were combined and washed with brine (100 mL), dried over anhydrous sodium sulfate and concentrated. The crude product was purified by silica gel column chromatography (ethyl acetate: petroleum ether = 2:5) to obtain compound **1-11. MS m/z (ESI):** 535.4 [M+Na]⁺. **¹H NMR** (400 MHz, DMSO-*d*₆) δ 10.96 (s, 1 H), 7.47 - 7.41 (m, 2 H), 7.41 - 7.35 (m, 2 H), 7.35 - 7.31 (m, 1 H), 7.19 (s, 1 H), 7.13 (dd, *J =* 17.2, 10.8 Hz, 1 H), 6.51 (d, *J* = 8.4 Hz, 1 H), 6.05 - 5.77 (m, 1 H), 5.42 (d, *J* = 11.2 Hz, 1 H), 5.22 (s, 2 H), 4.70 (t, *J* = 6.0 Hz, 1 H), 3.59 - 3.49 (m, 1 H), 3.31 - 3.26 (m, 2 H), 2.83 - 2.78 (m, 1 H), 2.71 - 2.63 (m, 1 H), 1.31 (s, 9 H).

Step 10: Dimethyl sulfoxide (80 mL) solution of pyridine sulfur trioxide (14.6 g, 91.73 mmol) was added to the dichloromethane (80 mL) and dimethyl sulfoxide (80 mL) solution of compound **1-11** (7.8 g, 15.22 mmol) and triethylamine (12.7 mL, 91.62 mmol) at 0°C under nitrogen atmosphere. The reaction solution was stirred at 0°C for 1 hour under nitrogen atmosphere. The reaction solution was poured into saturated sodium bicarbonate solution (200 mL) and extracted with dichloromethane (100 mL × 3). The organic phases were combined and washed with 1.0 M dilute hydrochloric acid solution (200 mL) and saturated brine (100 mL), dried over anhydrous sodium sulfate and concentrated. The crude product was slurried with a mixed solution of petroleum ether/ethyl acetate (100 mL, v/v ratio 10/1) to obtain compound **1-12. MS m/z (ESI):** 533.2 [M+Na]⁺. **¹H NMR** (400 MHz, DMSO-*d*₆) δ 10.98 (s, 1 H), 9.47 (s, 1 H), 7.53 - 7.26 (m, 6 H), 7.19 (s, 1 H), 6.93 (dd, *J* = 10.8, 17.2 Hz, 1 H), 5.86 (d, *J* = 17.2 Hz, 1 H), 5.42 (d, *J* = 11.2 Hz, 1 H), 5.23 (s, 2 H), 3.87 - 3.73 (m, 1 H), 3.13 - 3.08 (m, 1 H), 2.99 - 2.85 (m, 1 H), 1.34 (s, 9 H).

Step 11: Sodium hydrogen (1.3 g, 32.50 mmol) was added to the tetrahydrofuran (30 mL) solution of methyltriphenylphosphonium bromide (12 g, 33.59 mmol) at 0°C under nitrogen atmosphere. The reaction solution was stirred at 0°C for 30 min and then a tetrahydrofuran (30 mL) solution of compound **1-12** (4.8 g, 9.40 mmol) was added to the reaction solution. The reaction solution was stirred at 60°C for 12 hours. The reaction solution was quenched with water (100 mL) and extracted with ethyl acetate (50 mL × 3). The organic phases were combined and washed with saturated brine (100 mL), dried over anhydrous sodium sulfate and concentrated. The crude product was purified by silica gel column chromatography (ethyl acetate: petroleum ether = 1:10) to obtain compound **1-13. MS m/z (ESI):** 531.2 [M+Na]⁺. **¹H NMR** (400 MHz, DMSO-*d*₆) δ 10.96 (s, 1 H), 7.50 - 7.26 (m, 5 H), 7.18 (s, 1 H), 7.13 - 6.83 (m, 2 H), 5.87 (d, *J* = 17.2 Hz, 1 H), 5.80 - 5.64 (m, 1 H), 5.44 (d, *J =* 11.2 Hz, 1 H), 5.22 (s, 2 H), 5.04 - 4.80 (m, 2 H), 4.17 - 3.95 (m, 1 H), 2.90 - 2.71 (m, 2 H), 1.33 (s, 9 H).

Step 12: Methyl bromoacetate (0.66 mL, 6.95 mmol) was added to the N,N-dimethylformamide (30 mL) solution of compound **1-13** (2.5 g, 4.92 mmol) and potassium carbonate (2.05 g, 14.83 mmol). The reaction solution was stirred at 60°C for 2 hours. The reaction solution was diluted with water (100 mL) and extracted with ethyl acetate (50 mL × 3). The organic phases were combined and washed with saturated brine (100 mL), dried over anhydrous sodium sulfate and concentrated. The crude product was purified by silica gel column chromatography (ethyl acetate:petroleum ether = 1:5) to obtain compound **1-14. MS m/z (ESI):** 603.4 [M+Na]⁺. **¹H NMR** (400 MHz, DMSO-*d*₆) δ 7.52 - 7.29 (m, 5 H), 7.24 (d, *J =* 7.6 Hz, 1H), 7.20 - 6.85 (m, 2 H), 5.94 (dd, *J =* 11.2, 17.2 Hz, 1 H), 5.83 - 5.60 (m, 1 H), 5.51 (dd, *J* = 4.8, 11.2 Hz, 1 H), 5.35 - 5.15 (m, 2 H), 5.00 - 4.81 (m, 2 H), 4.50 (dd, *J =* 2.8, 16.8 Hz, 1 H), 4.29 - 4.13 (m, 1 H), 4.12 - 4.05 (m, 1 H), 3.61 (s, 3 H), 2.86 - 2.72 (m, 2 H), 1.43 - 1.25 (m, 9 H).

Step 13: 1,3-Bis(2,4,6-trimethylphenyl)-4,5-dihydroimidazol-2-ylidene[2-(i-propoxy)-5-(N,N-dimethylaminosulfonyl)phenyl]methyleneruthenium(II) dichloride (0.71 g, 0.96 mmol) was added to the dichloromethane (160 mL) solution of compound **1-14** (2.8 g, 4.82 mmol) under nitrogen atmosphere. The reaction solution was stirred in the dark at 40°C for 6 hours. The reaction solution was diluted with water (200 mL) and extracted with dichloromethane (100 mL × 3). The organic phases were combined and washed with saturated brine (200 mL), dried over anhydrous sodium sulfate and concentrated. The crude product was purified by silica gel column chromatography (ethyl acetate: petroleum ether = 1:3) to obtain compound **1-15. MS m/z (ESI):** 575.2 [M+Na]⁺. **¹H NMR** (400 MHz, DMSO-*d*₆) δ 7.49 - 7.11 (m, 6 H), 7.01 (s, 1 H), 6.53 (d, *J =* 9.6 Hz, 1 H), 6.12 - 5.96 (m, 1 H), 5.28 - 5.07 (m, 2 H), 4.51 (dd, *J =* 5.6, 16.8 Hz, 1 H), 4.35 - 4.26 (m, 2 H), 3.61 (d, *J* = 2.8 Hz, 3 H), 3.00 - 2.85 (m, 1 H), 2.64 - 2.53 (m, 1 H), 1.40 (s, 9 H).

Step 14: The dichloromethane (1 mL) solution of diiodomethane (0.18 mL, 2.23 mmol) was added dropwise to the dichloromethane (1 mL) solution of diethylzinc (1.10 mL, 1.10 mmol, 1 M hexane solution) at 0°C under nitrogen atmosphere. The reaction solution was stirred at 0°C for 15 min and then the dichloromethane (1 mL) solution of compound **1-15** (250 mg, 0.45 mmol) was added dropwise. The reaction solution was stirred at 20°C for 30 minutes. The reaction solution was diluted with saturated ammonium chloride solution (20 mL) and extracted with ethyl acetate (30 mL × 3). The organic phases were combined and washed with saturated brine (50 mL), dried over anhydrous sodium sulfate and concentrated. The crude product was purified by silica gel column chromatography (ethyl acetate: petroleum ether = 1:3) to obtain compound **1-16. MS m/z (ESI):** 589.4 [M+Na]⁺. **¹H NMR** (400 MHz, DMSO-*d*₆) δ 7.44 - 7.38 (m, 4 H), 7.37 - 7.30 (m, 1 H), 7.12 (s, 1 H), 7.08 - 7.02 (m, 1 H), 5.27 - 5.07 (m, 2 H), 4.49 (dd, *J* = 5.2, 16.8 Hz, 1 H), 4.20 (dd, *J* = 8.0, 16.8 Hz, 1 H), 3.91 - 3.71 (m, 1 H), 3.60 (s, 3 H), 2.88 - 2.77 (m, 1 H), 2.20 - 2.00 (m, 2 H), 1.78 - 1.65 (m, 1 H), 1.42 (s, 9 H), 1.04 - 0.89 (m, 2 H).

Step 15: Sodium methoxide (110 mg, 2.04 mmol) was added to the methanol (3 mL) solution of compound **1-16** (280 mg, 0.49 mmol). The reaction solution was stirred at 60°C for 2 hours. The solvent was dried by rotary evaporation to obtain crude compound **1-17,** which was directly used in the next step.

Step 16: Iodomethane (0.04 mL, 0.64 mmol) was added to the N,N-dimethylformamide (4 mL) solution of compound 1-17 (222 mg, 0.46 mmol). The reaction solution was stirred at 15°C for 3 hours. The reaction solution was diluted with water (20 mL) and extracted with ethyl acetate (30 mL × 3). The organic phases were combined and washed with saturated brine (50 mL), dried over anhydrous sodium sulfate and concentrated. The crude product was purified by silica gel column chromatography (ethyl acetate: petroleum ether = 3:20) to obtain compound **1-18. MS m/z (ESI):** 571.4 [M+H]⁺. **¹H NMR** (400 MHz, DMSO-*d*₆) δ 7.47 (d, *J* = 7.2 Hz, 2 H), 7.40 (t, *J =* 7.2 Hz, 2 H), 7.34 (t, *J* = 7.2 Hz, 1 H), 6.97 (d, *J =* 8.4 Hz, 1 H), 6.79 (s, 1 H), 5.11 (s, 2 H), 4.86 - 4.74 (m, 1 H), 4.02 - 3.93 (m, 2 H), 3.81 - 3.67 (m, 1 H), 3.58 (s, 3 H), 2.82 - 2.78 (m, 1 H), 1.99 - 1.86 (m, 2 H), 1.61 - 1.51 (m, 1 H), 1.41 (s, 9 H), 0.84 - 0.71 (m, 2 H).

Step 17: Tert-butanol (73 µL, 0.77 mmol) was added to the dichloromethane (0.2 mL) solution of chlorosulfonyl isocyanate (65 µL, 0.75 mmol). After the reaction solution was stirred at 20°C for 15 min, the dichloromethane (0.2 mL) solution of compound **1-18** (150 mg, 0.32 mmol) and triethylamine (135 µL, 0.97 mmol) was added dropwise. The reaction solution was stirred at 20°C for an additional 1 hour. The reaction solution was diluted with water (30 mL) and extracted with ethyl acetate (20 mL × 3). The organic phases were combined and washed with saturated brine (50 mL), dried over anhydrous sodium sulfate and concentrated. The crude product was purified by silica gel column chromatography (ethyl acetate:petroleum ether = 1:4) to obtain compound **1-19. MS m/z (ESI):** 672.4 [M+Na]⁺. **¹H NMR** (400 MHz, DMSO-*d*₆) δ 11.21 (d, *J =* 5.6 Hz, 1 H), 7.47 (d, *J =* 7.6 Hz, 2 H), 7.39 (t, *J* = 7.6 Hz, 2 H), 7.32 (t, *J* = 7.2 Hz, 1 H), 7.03 (t, *J* = 7.6 Hz, 1 H), 6.88 (s, 1 H), 5.20 (q, *J* = 13.2 Hz, 2 H), 4.67 (dd, *J =* 7.2, 17.6 Hz, 1 H), 4.34 (d, *J =* 18.4 Hz, 1 H), 3.85 - 3.69 (m, 1 H), 3.55 (s, 3 H), 2.87 - 2.74 (m, 1 H), 2.08 - 2.00 (m, 2 H), 1.72 - 1.61 (m, 1 H), 1.42 (s, 9 H), 1.34 - 1.28 (m, 9 H), 0.96 - 0.80 (m, 2 H).

Step 18: Trifluoroacetic acid (500 µL, 6.71 mmol) was added to the dichloromethane (1 mL) solution of compound **1-19** (107 mg, 0.16 mmol). The reaction solution was stirred at 25°C for 1 hour, the solvent was dried by rotary evaporation, and the crude compound was dissolved in dichloromethane (5 mL) with the addition of triethylamine (110 µL, 0.79 mmol) and di-tert-butyl dicarbonate (170 µL, 0.79 mmol). The reaction solution was stirred at 25°C for another 30 min and the solvent was dried by rotary evaporation. The crude product was purified by silica gel column chromatography (ethyl acetate: petroleum ether = 2:3) to obtain compound **1-20. MS m/z (ESI):** 572.4 [M+Na]⁺. **¹H NMR** (400 MHz, DMSO-*d*₆) δ 7.51 (d, *J =* 7.6 Hz, 2 H), 7.39 (t, *J =* 7.6 Hz, 2 H), 7.32 (t, *J* = 7.2 Hz, 1 H), 7.07 - 7.00 (m, 1 H), 6.94 (s, 1 H), 6.88 (s, 2 H), 5.15 (s, 2 H), 4.36 (dd, *J* = 4.0*,* 17.6 Hz, 1 H), 4.18 (dd, *J =* 3.2, 17.6 Hz, 1 H), 3.83 - 3.75 (m, 1 H), 3.55 (s, 3 H), 2.87 - 2.80 (m, 1 H), 2.10 - 1.99 (m, 2 H), 1.74 - 1.59 (m, 1 H), 1.42 (s, 9 H), 0.97 - 0.84 (m, 2 H).

Step 19: Sodium methoxide (25 mg, 0.46 mmol) was added to the methanol (2 mL) solution of compound **1-20** (117 mg, 0.21 mmol). The reaction solution was stirred at 60°C for 20 minutes. The reaction solution was diluted with water (30 mL) and extracted with ethyl acetate (20 mL × 3). The organic phase was washed with saturated brine (20 mL), dried over anhydrous sodium sulfate and concentrated to obtain compound **1-21. MS m/z (ESI):** 540.2 [M+Na]⁺. **¹H NMR** (400 MHz, DMSO-*d*₆) δ 7.50 (d, *J =* 6.8 Hz, 2 H), 7.35 (t, *J =* 6.8 Hz, 2 H), 7.30 (d, *J* = 7.2 Hz, 1 H), 7.02 (d, *J* = 7.6 Hz, 1 H), 6.92 (s, 1 H), 5.14 (s, 2 H), 4.00 - 3.89 (m, 2 H), 3.86 - 3.75 (m, 1 H), 2.88 - 2.75 (m, 1 H), 2.07 - 1.99 (m, 2 H), 1.72 - 1.60 (m, 1 H), 1.42 (s, 9 H), 0.94 - 0.81 (m, 2 H).

Step 20: Trifluoroacetic acid (500 µL, 6.71 mmol) was added to the dichloromethane (2 mL) solution of compound **1-21** (94 mg, 0.18 mmol). The reaction solution was stirred at 25°C for 1 hour. The solvent was dried by rotary evaporation to obtain crude compound **1-22,** which was directly used in the next step. **MS m/z (ESI):** 416.1 [M-H]⁻.

Step 21: Sodium cyanoborohydride (35 mg, 0.56 mmol) was added to the methanol (0.3 mL) solution of compound **1-22** (75.8 mg, 0.18 mmol) and isovaleraldehyde (20 µL, 0.19 mmol). The reaction solution was stirred at 25°C for 1 hour. The reaction solution was concentrated, and the crude product was purified by a reversed-phase column (C18, 5-50% acetonitrile/0.1% aqueous ammonium bicarbonate) to afford compound **1-23. MS m/z (ESI):** 488.2 [M+H]⁺. **¹H NMR** (400 MHz, DMSO-*d*₆) δ 8.61 (s, 1 H), 7.50 (d, *J =* 7.6 Hz, 2 H), 7.35 (t, *J =* 7.6 Hz, 2 H), 7.30 (d, *J =* 7.2 Hz, 1 H), 6.98 (s, 1 H), 5.16 (s, 2 H), 3.93 (s, 2 H), 3.66 (s, 1 H), 3.22 - 3.00 (m, 4 H), 2.29 - 2.15 (m, 2 H), 1.95 - 1.83 (m, 1 H), 1.74 - 1.61 (m, 1 H), 1.58 - 1.48 (m, 2 H), 1.14 - 1.01 (m, 2 H), 0.93 (d, *J =* 6.4 Hz, 6 H).

Step 22: 10% wet palladium on carbon (10 mg) was added to the methanol (2 mL) solution of compound **1-23** (60 mg, 0.12 mmol). The reaction solution was stirred at 25°C for 4 hours under the protection of hydrogen (15 psi). The reaction solution was filtered through celite, the filtrate was dried by rotary evaporation, and the crude product was purified by preparative HPLC (C18, acetonitrile/0.1% aqueous ammonium bicarbonate solution) to obtain compound **1. MS m/z (ESI):** 396.1 [M-H]⁻. **¹H NMR** (400 MHz, DMSO-*d*₆) δ 9.28 (br s, 1 H), 8.51 (br s, 1H), 6.69 (s, 1 H), 3.91 (s, 2 H), 3.65 (s, 1 H), 3.30 - 2.96 (m, 4 H), 2.25 - 2.08 (m, 2 H), 1.90 - 1.78 (m, 1 H), 1.74 - 1.60 (m, 1 H), 1.57 - 1.46 (m, 2 H), 1.11 - 0.98 (m, 2 H), 0.92 (d, *J =* 6.4 Hz, 6 H).

Compound **1** was separated by SFC chiral separation (Column: Chiralpak IC-3 100*4.6mm I.D., 3um; Mobile phase: A: CO2 B: methanol (0.05% DEA) Isocratic: 50% B; Flow Rate (ml/min) : 80; Column temp.: 35°C; ABPR: 1500 psi) to obtain compound **2** (isomer 1, first peak, retention time 1.301 min) and compound **3** (isomer 2, second peak, retention time 1.936 min).

Compound **2: MS m/z(ESI):** 398.1 [M+H]⁺. **¹H NMR** (400 MHz, DMSO-*d*₆) δ 9.27 (br s, 1 H), 8.50 (br s, 1 H), 6.69 (s, 1 H), 3.92 (s, 2 H), 3.67 - 3.60 (m, 1 H), 3.30 - 3.04 (m, 4 H), 2.22 - 2.13 (m, 2 H), 1.89 - 1.82 (m, 1 H), 1.75 - 1.64 (m, 1 H), 1.53 (q, *J =* 7.3 Hz, 2 H), 1.08 - 1.00 (m, 2 H), 0.93 (d, *J =* 6.6 Hz, 6 H).

Compound **3: MS m/z(ESI):** 398.2 [M+H]⁺. **¹H NMR** (400 MHz, DMSO-*d*₆) δ 9.27 (br s, 1 H), 8.50 (br s, 1 H), 6.69 (s, 1 H), 3.92 (s, 2 H), 3.67 - 3.60 (m, 1 H), 3.30 - 3.04 (m, 4 H), 2.22 - 2.13 (m, 2 H), 1.89 - 1.82 (m, 1 H), 1.75 - 1.64 (m, 1 H), 1.53 (q, *J =* 7.3 Hz, 2 H), 1.08 - 1.00 (m, 2 H), 0.93 (d, *J =* 6.6 Hz, 6 H).

### Example 2: Preparation of compounds 4, 5, and 6

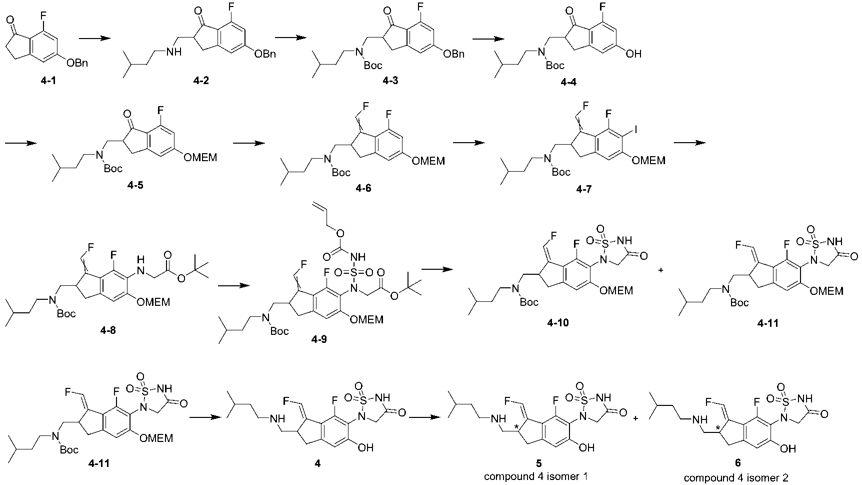

Step 1: Compound **4-1** (15.0 g, 58.53 mmol) was dissolved in ethanol (200 mL), and isopentylamine hydrochloride (15 g, 58.53 mmol) and paraformaldehyde (19.31 g, 585.30 mmol) were added. The reaction solution was stirred at 95 °C for 12 hours. The reaction solution was cooled to room temperature and filtered, and the filter cake was washed with ethanol. The filter cake was dried under reduced pressure to obtain compound **4-2. MS m/z (ESI):** 356.1 [M+H]⁺.

Step 2: Compound **4-2** (14.0 g, 39.39 mmol) was dissolved in tetrahydrofuran (100 mL), and triethylamine (11.96 g, 118.16 mmol) and Boc anhydride (9.46 g, 43.33 mmol) were added. The reaction solution was stirred at 25 °C for 3 hours. The reaction solution was diluted with water (100 mL) and extracted with ethyl acetate (200 mL). The organic phase was washed with saturated brine (100 mL) and dried over anhydrous sodium sulfate, and was filtered. The filtrate was concentrated, and the residue was purified by silica gel column chromatography (tetrahydrofuran:petroleum ether = 1:4) to obtain compound **4-3. ¹H NMR** (400MHz, DMSO-*d₆*) δ 7.50 - 7.34 (m, 5 H), 7.06 (s, 1 H), 6.88 (br d, *J =* 10.4 Hz, 1 H), 5.30 - 5.17 (m, 2 H), 3.52 - 3.35 (m, 2 H), 3.50 - 3.10 (m, 3 H), 2.99 (br s, 1 H), 2.89 (br s, 1 H), 1.55 - 1.44 (m, 1 H), 1.43 - 1.27 (m, 11 H), 0.87 (d, *J =* 6.4 Hz, 6 H).

Step 3: Compound **4-3** (15.0 g, 32.93 mmol) was dissolved in methanol (500 mL) and 10% wet palladium carbon (3.50 g) was slowly added to the reaction solution under nitrogen atmosphere. Hydrogen was replaced three times, and the reaction solution was stirred at room temperature under hydrogen atmosphere (30 psi) for 12 hours. The reaction solution was filtered through celite and the filtrate was washed with methanol. The filtrate was concentrated to obtain compound **4-4,** which was used directly in the next step.

Step 4: Compound **4-4** (8.5 g, 23.26 mmol) was dissolved in N,N-dimethylformamide (100 mL). Cesium carbonate (22.73 g, 69.78 mmol) was added to the reaction solution under nitrogen atmosphere, and 2-methoxyethoxymethyl chloride (5.3 mL, 46.52 mmol) was added dropwise at -30°C. The reaction solution was stirred at -30 °C for 30 min. The reaction solution was quenched with water (300 mL) and extracted with ethyl acetate (300 mL × 3). The organic phase was washed with brine (100 mL) and dried over anhydrous sodium sulfate, and was filtered, and the filtrate was concentrated. The residue was purified by silica gel column chromatography (tetrahydrofuran:petroleum ether = 3:7) to obtain compound **4-5. MS m/z (ESI):** 354.2 [M+H-Boc]⁺. **¹H NMR** (400MHz, DMSO-*d₆*) δ 7.03 (s, 1 H), 6.85 (br d, *J* = 11.2 Hz, 1 H), 5.39 (s, 2 H), 3.78 - 3.70 (m, 2 H), 3.51 - 3.42 (m, 4 H), 3.22 (s, 3 H), 3.14 (br s, 2 H), 2.99 (br s, 1 H), 2.86 (br s, 1 H), 1.48 (td, *J =* 6.4, 13.2 Hz, 1 H), 1.43 - 1.22 (m, 12 H), 0.87 (d, *J =* 6.4 Hz, 6 H).

Step 5: (Fluoromethyl)triphenylphosphonium tetrafluoroborate (12.63 g, 33.07 mmol) was dissolved in tetrahydrofuran (150 mL), and potassium tert-butoxide (3.71 g, 33.07 mmol) was slowly added under nitrogen atmosphere at 0 °C. The reaction solution was stirred at 0 °C for 30 min. Compound **4-5** (5 g, 11.02 mmol) was dissolved in tetrahydrofuran (20 mL) and slowly added dropwise to the reaction solution at 0 °C. The reaction solution was stirred at room temperature for 2 hours. The reaction solution was quenched with saturated aqueous ammonium chloride solution (300 mL), extracted with ethyl acetate (300 mL × 3), and the organic phase was washed with brine (200 mL) and dried over anhydrous sodium sulfate, and was filtered. The filtrate was concentrated to obtain a crude product, and the crude product was purified by silica gel column chromatography (tetrahydrofuran:petroleum ether = 1:10) to obtain compound **4-6. MS m/z (ESI):** 492.2 [M+Na]⁺.

Step 6: Compound **4-6** (4.3 g, 9.16 mmol) was dissolved in tetrahydrofuran (50 mL), 2.5 M n-butyllithium (18.31 mL, 45.79 mmol) was added dropwise under nitrogen atmosphere at -78 °C, and the reaction solution was stirred at -78 °C for 30 min. Iodine (6.97 g, 27.47 mmol) was dissolved in tetrahydrofuran (20 mL) and slowly added dropwise to the reaction solution at -78°C, and the reaction solution was stirred at -78°C for 2 hours. The reaction solution was quenched with saturated ammonium chloride (300 mL) and extracted with ethyl acetate (100 mL × 3). The organic phase was washed with aqueous sodium sulfite (100 mL), washed with brine (100 mL) and dried over anhydrous sodium sulfate. Filtered, and concentrated. The residue was purified by silica gel column chromatography (tetrahydrofuran:petroleum ether = 3:10) to obtain compound **4-7. MS m/z (ESI):** 540.1 [M+H-56]⁺.

Step 7: Compound **4-7** (5.0 g, 8.40 mmol), tert-butyl glycinate (1.65 g, 12.60 mmol), methanesulfonic acid (2-dicyclohexylphosphino-3,6-dimethoxy-2',4',6'-triisopropyl-1,1'-biphenyl)(2'-amino-1,1'-biphenyl-2-yl)palladium(II) (0.76 g, 0.84 mmol), and cesium carbonate (8.21 g, 25.19 mmol) were dissolved in dioxane (50 mL), replaced with nitrogen three times, and the reaction solution was stirred at 95 °C for 4 hours. The reaction solution was extracted with water (300 mL) and ethyl acetate (300 mL × 3), and the organic phase was washed with saturated brine (100 mL) and dried with anhydrous sodium sulfate, and was filtered. The filtrate was concentrated to obtain the crude product, and the crude product was purified by silica gel column chromatography (tetrahydrofuran:petroleum ether = 3:10) to obtain compound **4-8. MS m/z (ESI):** 599.3 [M+H]⁺.

Step 8: Three-necked flask A was taken, chlorosulfonyl isocyanate (1.65 g, 11.69 mmol) was dissolved in dichloromethane (50 mL), allyl alcohol (0.95 g, 16.36 mmol) was added dropwise under nitrogen atmosphere at 0°C, and the reaction solution was stirred at 0°C for 1 hour. Three-necked flask B was taken and compound **4-8** (3.5 g, 5.85 mmol) was dissolved in dichloromethane (50 mL) and triethylamine (2.4 mL, 17.54 mmol) was added dropwise. The solution in three-necked flask A was added to the three-necked flask B at 0°C.The reaction solution was stirred at 0 °C for 2 hours under nitrogen atmosphere. The reaction solution was diluted with water (50 mL), extracted with ethyl acetate (50 mL x3), and the organic phase was washed with brine (30 mL) and dried over anhydrous sodium sulfate, and was filtered. The filtrate was concentrated to obtain compound **4-9. MS m/z(ESI):** 760.3 [M-H]⁻.

Step 9: Compound **4-9** (3.0 g, 3.94 mmol) was dissolved in methanol (60 mL), tetrakis(triphenylphosphine)palladium (0.23 g, 0.20 mmol), sodium methoxide (1.3 mL, 23.63 mmol, 30% methanol solution) and 4A molecular sieves (3.0 g) were added, nitrogen atmosphere was replaced three times, and the reaction solution was stirred at 60°C for 12 hours. The reaction solution was quenched with saturated aqueous ammonium chloride solution (100 mL) and extracted with ethyl acetate (300 mL × 3), and the organic phase was washed with saturated brine (100 mL) and dried over anhydrous sodium sulfate, and was filtered. The filtrate was concentrated and the crude product was prepared by HPLC (column: C18 150×40 mm; flow rate: 60 mL/min, mobile phase: [Water (NH3H2O+NH4HCO3)-ACN]; B%: 20%-60% acetonitrile, 9 min) to obtain compound **4-10** (first peak, retention time 2.64 min) and 4-11 (second peak, retention time 2.76 min). Compound **4-10: MS m/z(ESI):** 602.2 [M-H]⁻. compound 4-11: **MS m/z(ESI):** 602.2 [M-H]⁻.

Step 10: Compound **4-11** (600 mg, 0.99 mmol) was dissolved in dichloromethane (6 mL), trifluoroacetic acid (2.0 mL, 26.84 mmol) was added, and the reaction solution was stirred at 25°C for 12 hours. The reaction solution was concentrated, and the residue was prepared by HPLC (column: Phenomenex Gemini NX 150×30 mm, 5 µm; flow rate: 60 mL/min; mobile phase: [Water (ammonia hydroxide v/v)-ACN]; B%: 9%-49%, 9 min) to obtain compound **4.** Compound **4** was prepared by SFC chiral separation (Column: DAICEL CHIRALPAK IG (250 mm*30 mm, 10 µm); Condition: CO₂-EtOH (0.1%NH₃H₂O) Begin B: 35% End B: 35%; Flow Rate (ml/min): 80) to obtain compound **5** (isomer 1, first peak, retention time 1.062 min) and compound **6** (isomer 2, second peak, retention time 1.486 min).

Compound **4: MS m/z(ESI):** 416.0 [M+H]⁺. **¹H NMR** (400 MHz, DMSO-*d₆*) δ 9.63 (br s, 1 H), 8.23 (s, 1 H), 7.68 - 7.23 (m, 1 H), 6.66 (s, 1 H), 4.01 - 3.88 (m, 2 H), 3.63 (br s, 1 H), 3.30 - 3.11 (m, 2 H), 3.10 - 3.05 (m, 1 H), 3.05 - 2.85 (m, 4 H), 1.68 - 1.56 (m, 1 H), 1.52 - 1.41 (m, 2 H), 0.89 (d, *J =* 6.4 Hz, 6 H).

Compound **5: MS m/z(ESI):** 416.1 [M+H]⁺. **¹H NMR** (400 MHz, DMSO-*d₆*) δ 9.63 (br s, 1 H), 8.23 (s, 1 H), 7.68 - 7.23 (m, 1 H), 6.66 (s, 1 H), 4.01 - 3.88 (m, 2 H), 3.63 (br s, 1 H), 3.30 - 3.11 (m, 2 H), 3.10 - 3.05 (m, 1 H), 3.05 - 2.85 (m, 4 H), 1.68 - 1.56 (m, 1 H), 1.52 - 1.41 (m, 2 H), 0.89 (d, *J =* 6.4 Hz, 6 H).

Compound **6: MS m/z(ESI):** 416.1 [M+H]⁺. **¹H NMR** (400 MHz, DMSO-*d₆*) δ 9.53 (br s, 1 H), 8.18 (br s, 1 H), 7.58 - 7.28 (m, 1 H), 6.66 (s, 1 H), 4.01 - 3.88 (m, 2 H), 3.63 (br s, 1 H), 3.20 - 3.03 (m, 3 H), 3.02 - 2.85 (m, 4 H), 1.68 - 1.56 (m, 1 H), 1.54 - 1.43 (m, 2 H), 0.89 (d, *J* = 6.4 Hz, 6 H).

### Example 3: Preparation of compound 7

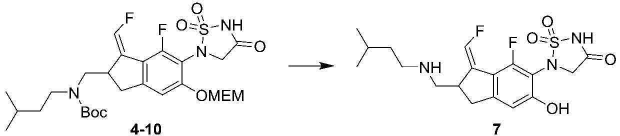

Step 1: Compound **4-10** (400 mg, 0.66 mmol) was dissolved in dichloromethane (4 mL), trifluoroacetic acid (1.3 mL, 17.44 mmol) was added, and the reaction solution was stirred at 25 °C for 12 hours. The reaction solution was concentrated, and the residue was prepared by HPLC (column: Phenomenex Gemini NX 150×30 mm, 5 µm; flow rate: 60 mL/min; mobile phase: [Water (ammonia hydroxide v/v)-ACN]; B%: 6%-46%, 9 min) to obtain compound **7. MS m/z(ESI):** 416.0 [M+H]⁺. **¹H NMR** (400 MHz, DMSO-*d₆*) δ 9.67 (s, 1 H), 8.25 (br s, 1 H), 6.95 (br d, *J* = 82.0 Hz, 1 H), 6.67 (s, 1 H), 3.94 (s, 2 H), 3.22 - 2.96 (m, 4 H), 2.95 - 2.88 (m, 3 H), 2.77 (br d, *J* = 16.0 Hz, 1 H), 1.61 (td, *J =* 6.4, 13.2 Hz, 1 H), 1.54 - 1.44 (m, 2 H), 0.89 (d, *J =* 6.4 Hz, 6 H).

### Example 4: Preparation of compound 8

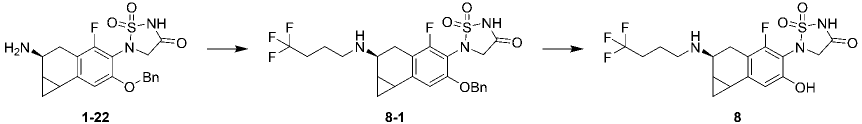

Step 1: Sodium cyanoborohydride (6 mg, 95.48 µmol) was added to the methanol (0.2 mL) solution of compound **1-22** (24 mg, 57.49 µmol) and 4,4,4-trifluorobutyraldehyde (7 µL, 66.62 µmol). The reaction solution was stirred at 25 °C for 2 hours. The reaction solution was concentrated under reduced pressure, and the crude product was purified by silica gel column chromatography (methanol:dichloromethane = 1:10) to obtain compound **8-1. MS m/z (ESI):** 528.2 [M+H]⁺.

Step 2: 10% wet palladium carbon (10 mg) was added to the ethanol (2 mL) solution of compound **8-1** (30 mg, 56.87 µmol) under nitrogen atmosphere. The reaction solution was stirred at 25°C for 12 hours under hydrogen (15 psi) atmosphere. The reaction solution was filtered through celite, the filtrate was dried by rotary evaporation, and the crude product was purified by preparative HPLC (C18, 0.05% ammonium bicarbonate solution/acetonitrile) to obtain compound **8. MS m/z (ESI):** 438.1 [M+H]⁺. **¹H NMR** (400 MHz, DMSO-*d*₆) δ 9.25 (br s, 1 H), 8.63 (br s, 1 H), 6.69 (s, 1 H), 3.90 (s, 2 H), 3.67 (br s, 1 H), 3.26 - 2.92 (m, 4 H), 2.47 - 2.36 (m, 2 H), 2.26 - 2.05 (m, 2 H), 1.95 - 1.71 (m, 3 H), 1.13 - 0.94 (m, 2 H).

### Example 5: Preparation of compound 9

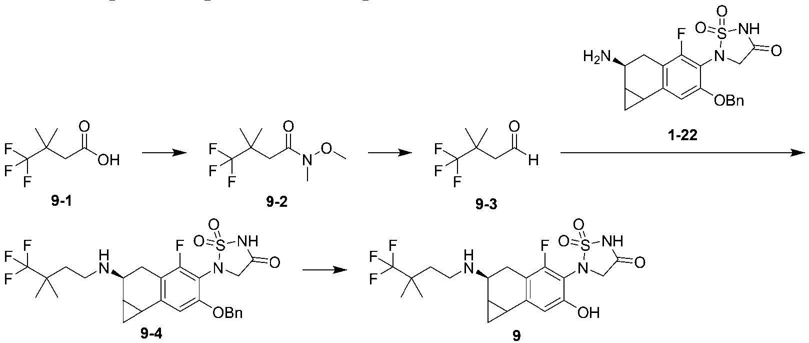

Step 1: 2-(7-azabenzotriazol-1-yl)-N,N,N',N'-tetramethyluronium hexafluorophosphate (8.8 g, 23.14 mmol) and triethylamine (7.4 mL, 53.38 mmol) were added to the N,N-dimethylformamide (30 mL) solution of compound **9-1** (3.0 g, 17.63 mmol) and N,O-dimethylhydroxylamine hydrochloride (3.5 g, 35.88 mmol). The reaction solution was stirred at 25°C for 12 hours. The reaction solution was diluted with water (100 mL) and extracted with ethyl acetate (50 mL × 3). The combined organic phase was washed with saturated brine, dried over anhydrous sodium sulfate and filtered, and the filtrate was concentrated under reduced pressure. The crude product was purified by silica gel column chromatography (ethyl acetate:petroleum ether = 1:10) to obtain compound **9-2. MS m/z (ESI):** 214.0 [M+H]⁺. **¹H NMR** (400 MHz, CDCl₃) δ 3.69 (s, 3 H), 3.19 (s, 3 H), 2.60 (s, 2 H), 1.29 (s, 6 H).

Step 2: Lithium aluminum hydride (0.56 mL, 1.4 mmol, 2.5M tetrahydrofuran solution) was added dropwise to the tetrahydrofuran (5 mL) solution of compound **9-2** (200 mg, 0.94 mmol) at 0°C. After the addition was completed, the reaction solution was stirred at 0°C for 2 hours. The reaction solution was quenched with sodium sulfate decahydrate, the mixture was filtered through celite, and the filtrate was concentrated under reduced pressure to obtain compound **9-3. ¹H NMR** (400 MHz, CDCl₃) δ 9.75 - 9.73 (m, 1 H), 2.47 (d, *J =* 2.4 Hz, 2 H), 1.22 (s, 6 H).

Step 3: Sodium cyanoborohydride (10 mg, 159.13 µmol) was added to the methanol (1 mL) solution of compound **1-22** (22 mg, 52.70 µmol) and compound **9-3** (1.44 mL, 158.1 µmol). The reaction solution was stirred at 25°C for 12 hours. The reaction solution was concentrated under reduced pressure, and the crude product was purified by reversed-phase column chromatography (C18, 0.05% ammonium bicarbonate solution/acetonitrile) to obtain compound **9-4. MS m/z (ESI):** 556.4 [M+H]⁺.

Step 4: 10% wet palladium carbon (5 mg) was added to the tetrahydrofuran (3 mL) solution of compound **9-4** (24 mg, 43.20 µmol) under nitrogen atmosphere. The reaction solution was stirred at 25°C for 12 hours under hydrogen (15 psi) atmosphere. The reaction solution was filtered through celite, the filtrate was dried by rotary evaporation, and the crude product was purified by preparative HPLC (C18, 0.05% ammonium bicarbonate solution/acetonitrile) to obtain compound **9. MS m/z (ESI):** 466.1 [M+H]⁺. **¹H NMR** (400 MHz, DMSO-*d*₆) δ 9.25 (s, 1 H), 8.41 (s, 1 H), 6.69 (s, 1 H), 3.91 (s, 2 H), 3.78 - 3.56 (m, 1 H), 3.47 - 3.38 (m, 1 H), 3.19 - 3.06 (m, 3 H), 2.25 - 2.08 (m, 2 H), 1.96 - 1.70 (m, 3 H), 1.15 (s, 6 H), 1.08 - 0.92 (m, 2 H).

### Example 6: Preparation of compound 10

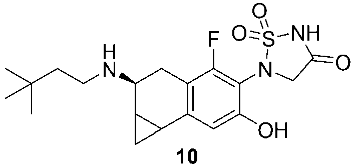

Compound **10** was synthesized with reference to Example 4, except that 3,3-dimethylbutyraldehyde was used as the raw material to replace 4,4,4-trifluorobutyraldehyde in Example 4, and compound 10 was prepared by the same method as Example 4.

Compound **10: MS m/z (ESI):** 412.0 [M+H]⁺. **¹H NMR** (400 MHz, DMSO-*d*₆) δ 9.24 (s, 1 H), 8.29 (s, 1 H), 6.75 - 6.62 (m, 1 H), 3.94 - 3.88 (m, 2 H), 3.70 - 3.59 (m, 1 H), 3.27 - 2.80 (m, 4 H), 2.25 - 1.98 (m, 2 H), 1.92 - 1.79 (m, 1 H), 1.59 - 1.51 (m, 1 H), 1.45 (t, *J =* 8.8 Hz, 1 H), 1.17 - 0.97 (m, 2 H), 0.96 - 0.86 (m, 9 H).

### Example 7: Preparation of compound 11

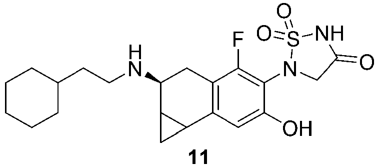

Compound **11** was synthesized with reference to Example 4, except that 2-cyclohexyl acetaldehyde was used as the raw material to replace 4,4,4-trifluorobutyraldehyde in Example 4, and compound **11** was prepared by the same method as Example 4.

Compound **11: MS m/z (ESI):** 438.0 [M+H]⁺. **¹H NMR** (400 MHz, DMSO-*d*₆) δ 9.25 (brs, 1 H), 8.33 (brs, 1 H), 6.76 - 6.63 (m, 1 H), 3.94 - 3.87 (m, 2 H), 3.68 - 3.62 (m, 1 H), 3.20 - 2.84 (m, 4 H), 2.25 - 1.94 (m, 2 H), 1.90 - 1.78 (m, 1 H), 1.78 - 1.57 (m, 5 H), 1.56 - 1.49 (m, 1 H), 1.48 - 1.39 (m, 1 H), 1.28 - 0.78 (m, 8 H).

### Example 8: Preparation of compounds 12, 13, and 14

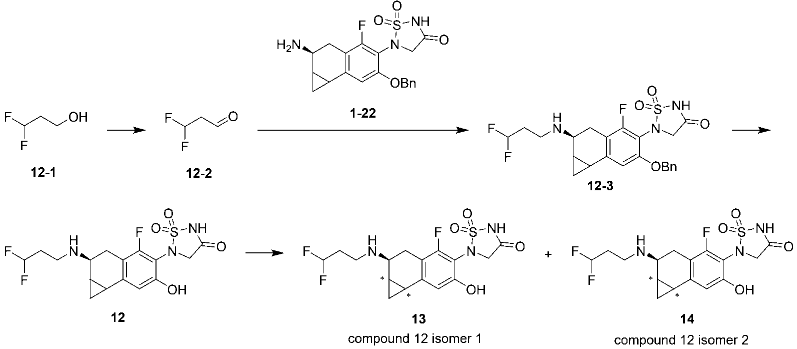

Step 1: Dess-Martin reagent (980 mg, 2.31 mmol) was added to the dichloromethane (4 mL) solution of compound **12-1** (200 mg, 2.08 mmol). The reaction solution was stirred at 25°C for 12 hours. The reaction solution was filtered through celite and the filtrate was diluted with dichloromethane (10 mL) to obtain a solution of compound **12-2,** which was directly used in the next step. **¹H NMR** (400 MHz, CDCl₃) δ 9.79 (s, 1 H), 6.24 (t, *J* = 50.4 Hz, 1 H), 3.10 - 2.96 (m, 2 H).

Step 2: Sodium cyanoborohydride (75 mg, 1.19 mmol) was added to the methanol (2 mL) solution of compound **1-22** (400 mg, 0.60 mmol) and compound **12-2** (4.0 mL, 0.84 mmol, dichloromethane solution). The reaction solution was stirred at 25°C for 3 hours. The reaction solution was concentrated under reduced pressure, and the crude product was purified by reversed-phase column chromatography (C18, 0.05% ammonium bicarbonate solution/acetonitrile) to obtain compound **12-3. MS m/z (ESI):** 496.2 [M+H]⁺. **¹H NMR** (400 MHz, DMSO-*d*₆) δ 8.80 (brs, 1 H), 7.53 - 7.47 (m, 2 H), 7.40 - 7.32 (m, 2 H), 7.32 - 7.26 (m, 1 H), 6.98 (s, 1 H), 6.26 (tt, *J =* 4.0, 56.0 Hz, 1 H), 5.16 (s, 2 H), 3.94 (d, *J* = 2.0 Hz, 2 H), 3.82 - 3.66 (m, 1 H), 3.30 - 3.24 (m, 2 H), 3.20 - 3.08 (m, 2 H), 2.39 - 2.14 (m, 4 H), 1.95 - 1.81 (m, 1 H), 1.16 - 1.01 (m, 2 H).

Step 3: Boron trichloride (1.5 mL, 1.5 mmol, 1M n-hexane solution) was added to the dichloromethane (5 mL) solution of compound **12-3** (160 mg, 0.32 mmol) and pentamethylbenzene (96 mg, 0.65 mmol) under nitrogen atmosphere at -70°C. The reaction solution was stirred at -70°C for 1 hour. The reaction solution was quenched with methanol (2 mL) at -70°C and the mixture was concentrated under reduced pressure and the crude was purified by preparative HPLC (C18, 0.05% ammonium bicarbonate solution/acetonitrile) to obtain compound **12. MS m/z (ESI):** 406.2 [M+H]⁺. **¹H NMR** (400 MHz, DMSO-*d*₆) δ 9.31 (s, 1 H), 8.76 (brs, 1 H), 6.69 (s, 1 H), 6.25 (tt, *J* = 4.0, 56.0 Hz, 1 H), 3.91 (s, 2 H), 3.82 - 3.66 (m, 1 H), 3.30 - 3.24 (m, 2 H), 3.20 - 3.08 (m, 2 H), 2.39 - 2.16 (m, 4 H), 1.87 - 1.79 (m, 1 H), 1.07 - 1.02 (m, 2 H).

Compound **12** was separated by SFC (Instrument: WATERS 150 preparative SFC (SFC-26); Column: ChiralPak IC, 250×30mm I.D., 10µm; Mobile phase: A for CO2 and B for Methanol (0.1% NH3H2O); Gradient: B 40%; Flow rate: 150 mL /min; Back pressure: 100 bar; Column temperature: 38°C; Wavelength: 220 nm; Cycle time: ~8 min). The first peak (retention time: 1.543 min) was compound **13, MS m/z (ESI):** 405.9 [M+H]⁺. **¹H NMR** (400 MHz, DMSO-*d*₆) δ 9.31 (s, 1 H), 8.76 (brs, 1 H), 6.69 (s, 1 H), 6.25 (tt, *J =* 4.0, 56.0 Hz, 1 H), 3.91 (s, 2 H), 3.82 - 3.66 (m, 1 H), 3.30 - 3.24 (m, 2 H), 3.20 - 3.08 (m, 2 H), 2.39 - 2.16 (m, 4 H), 1.87 - 1.79 (m, 1 H), 1.07 - 1.02 (m, 2 H). The second peak (retention time: 1.978 min) was compound **14, MS m/z (ESI):** 405.9 [M+H]⁺. **¹H NMR** (400 MHz, DMSO-*d*₆) δ 9.31 (s, 1 H), 8.76 (brs, 1 H), 6.69 (s, 1 H), 6.25 (tt, *J =* 4.0, 56.0 Hz, 1 H), 3.91 (s, 2 H), 3.82 - 3.66 (m, 1 H), 3.30 - 3.24 (m, 2 H), 3.20 - 3.08 (m, 2 H), 2.39 - 2.16 (m, 4 H), 1.87 - 1.79 (m, 1 H), 1.07 - 1.02 (m, 2 H).

### Example 9: Preparation of compound 15

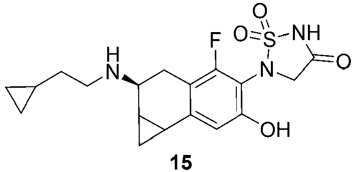

Compound **15** was synthesized with reference to Example 8, except that 2-cyclopropylethanol was used as the raw material to replace compound **12-1** in Example 8, and compound **15** was prepared by the same method as Example 8.

Compound **15: MS m/z (ESI):** 495.9 [M+H]⁺. **¹H NMR** (400 MHz, DMSO-*d*₆) δ 9.25 (brs, 1 H), 8.50 (brs, 1 H), 6.76 - 6.62 (m, 1 H), 3.97 - 3.85 (m, 2 H), 3.69 - 3.62 (m, 1 H), 3.20 - 2.87 (m, 4 H), 2.28 - 1.96 (m, 2 H), 1.89 - 1.75 (m, 1 H), 1.61 - 1.38 (m, 2 H), 1.16 - 0.89 (m, 2 H), 0.87 - 0.64 (m, 1 H), 0.53 - 0.37 (m, 2 H), 0.19 - 0.00 (m, 2 H).

### Example 10: Preparation of compound 16

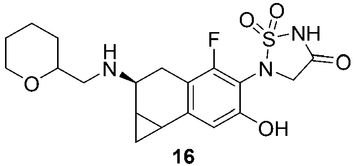

Compound **16** was synthesized with reference to Example 4, except that tetrahydropyran-2-carboxaldehyde was used as the raw material to replace 4,4,4-trifluorobutyraldehyde in Example 4, and compound **16** was prepared by the same method as Example 4.

Compound **16: MS m/z (ESI):** 426.2 [M+H]⁺. **¹H NMR** (400 MHz, DMSO-*d*₆) δ 9.23 (s, 1 H), 8.73 (brs, 1 H), 6.68 (s, 1 H), 4.02 - 3.94 (m, 1 H), 3.90 (s, 2 H), 3.72 - 3.53 (m, 2 H), 3.47 - 3.40 (m, 1 H), 3.26 - 3.17 (m, 1 H), 3.14 - 3.00 (m, 2 H), 2.29 - 2.11 (m, 2 H), 1.92 - 1.77 (m, 2 H), 1.68 - 1.59 (m, 1 H), 1.56 - 1.45 (m, 3 H), 1.29 - 1.21 (m, 2 H), 1.12 - 0.98 (m, 2 H).

### Example 11: Preparation of compound 17

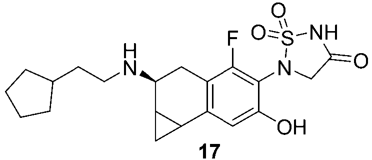

Compound **17** was synthesized with reference to Example 8, except that 2-cyclopentylethanol was used as the raw material to replace compound 12-1 in Example 8, and compound **17** was prepared by the same method as Example 8.

Compound **17: MS m/z (ESI):** 424.6 [M+H]⁺. **¹H NMR** (400 MHz, DMSO-*d*₆) δ 9.25 (brs, 1 H), 8.49 (brs, 1 H), 6.69 (s, 1 H), 3.91 (s, 2 H), 3.73 - 3.60 (m, 1 H), 3.16 - 3.00 (m, 3 H), 2.24 - 2.12 (m, 2 H), 1.89 - 1.72 (m, 4 H), 1.70 - 1.47 (m, 6 H), 1.18 - 1.08 (m, 2 H), 1.08 - 1.01 (m, 2 H).

### Example 12: Preparation of compound 18

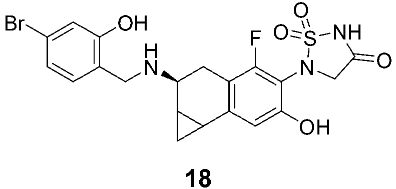

Compound **18** was synthesized with reference to Example 8, except that 3-bromo-5-hydroxybenzyl alcohol was used as the raw material to replace compound **12-1** in Example 8, and compound **18** was prepared by the same method as Example 8.

Compound **18: MS m/z (ESI):** 511.8 [M+H]⁺. **¹H NMR** (400 MHz, DMSO-*d*₆) δ 9.18 (s, 1 H), 7.28 (d, *J =* 8.0 Hz, 1 H), 7.08 - 6.99 (m, 2 H), 6.67 (s, 1 H), 4.17 (q, *J =* 13.6 Hz, 2 H), 3.90 (d, *J* = 2.0 Hz, 2 H), 3.57 - 3.50 (m, 1 H), 3.14 - 3.02 (m, 1 H), 2.24 - 2.09 (m, 2 H), 1.87 - 1.78 (m, 1 H), 1.11 - 0.95 (m, 2 H).

### Experimental Example 1: PTPN2 inhibitory activity assay

The present invention employs HTRF assay to characterize the inhibitory activity of compounds against PTPN2. The specific experimental process was as follows: compounds were transferred to a 384-well plate (PE# 6007290) using ECHO, and enzymatic reaction buffer (50 mM HEPES pH 7.5, 10 mM EDTA,0.01% Tween 20, 2 mM DTT) was prepared. PTPN2 (Sino biological #10570-H20B) was diluted in the reaction buffer, PTPN2 (final concentration 1 nM) was added and incubated with the compound at room temperature for 10 min, then the substrate (final concentration 1 µM) (biotin-(NH-CH₂-CH₂-O-CH₂-CH₂-O-CH₂-CO)-T-R-D-I-(PY)-E-T-D-Y-Y-R-K-K-NH₂) (Genscript) was added, and the reaction was terminated by adding a quencher after incubation at room temperature for 40 min. A final concentration of 10 nM Eu-anti-P (PY20) antibody (Cisbio#AD0066) and 15 nM APC-Streptavidin (Cisbio#AD0201) was added and incubated at room temperature for 1 hour. The fluorescence values of the reacted 384-well plates were read at 620 nm and 665 nm using Envision, and the IC50 value of inhibiting PTPN2 enzyme activity (PTPN 2 IC50) was fitted by nonlinear regression analysis of a four-parameter logistic equation.

Positive reference material:

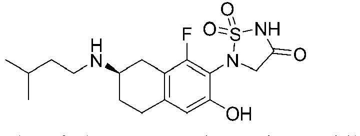

Upon testing, the compounds of the present invention exhibit better PTPN2 inhibitory activity, and the test results of some representative compounds are shown in Table 1.

**Table 1**

| Cpd. NO. | **PTPN2 IC₅₀** | Cpd. NO. | **PTPN2 IC₅₀** | Cpd. NO. | **PTPN2 IC₅₀** |
|---|---|---|---|---|---|
| **1** | A | **6** | A | **11** | A |
| **2** | C | **7** | B | **15** | A |
| **3** | A | **8** | A | 16 | C |
| **4** | A | **9** | A | **17** | A |
| **5** | C | **10** | A | | |

| | | | | | |
|---|---|---|---|---|---|
| A: IC₅₀ ≤ 20 nM, B: 20 nM < IC₅₀ ≤ 100 nM, C: 100 nM < IC₅₀ ≤ 500 nM, D: 500 nM < IC₅₀. | | | | | |

### Experimental Example 2: B16F10 mIFNγ-induced cell growth inhibition assay

40 µL (150 cells/well) of B16F10 cells (source ATCC Cat#CRL-6475) were inoculated into a 384-well plate, and the compounds were transferred to the 384-well plate containing cells using EHCO. One group of cells was added with 10 µL of complete medium (RPMI 1640 medium+10% FBS) without mIFNγ, one group was added with 10 µL of complete medium containing mIFNγ (source R&D Cat#485-MI/CF, final concentration 25 ng/ml), and different concentrations of compounds (DMSO content 0.5%) were added to the reaction system. The cell culture plates were incubated at 37 °C in 5% CO₂ for 96 h. 25 µL of CTG was added, Envision was used for reading, the cell growth inhibition IC50 value (Cell IC50) was fitted by nonlinear regression analysis using a four-parameter logistic equation with DMSO/mIFNγ as the negative control and 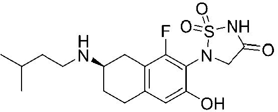 as the positive control.

Upon testing, the compounds of the present invention exhibit better inhibitory effects on B16F10 mIFNγ-induced cell growth, and the results of representative compounds are shown in Table 2.

**Table 2**

| Cpd. NO. | **B16F10 GI₅₀** | Cpd. NO. | **B16F10 GI₅₀** | Cpd. NO. | **B16F10 GI₅₀** |
|---|---|---|---|---|---|
| **1** | B | **6** | A | **11** | C |
| **2** | C | **7** | C | **15** | A |
| **3** | A | **8** | A | **16** | B |
| **4** | B | **9** | B | **17** | B |
| **5** | C | **10** | B | | |

| | | | | | |
|---|---|---|---|---|---|
| A: IC₅₀ ≤ 1 µM, B: 1 µM< IC₅₀ ≤ 10 µM, C: 10 µM < IC₅₀ ≤ 100 µM, D: 100 µM < IC₅₀. | | | | | |

The technical solution of the present invention is not limited to the above-mentioned specific embodiments. All technical variations made according to the technical solution of the present invention fall within the protection scope of the present invention.

## Claims

1. A compound having a structure represented by formula (I-1) or (I-2) or an enantiomer, diastereomer, racemate, tautomer, stereoisomer, geometric isomer, N-oxide, metabolite or pharmaceutically acceptable salt, ester, solvate, hydrate, isotope-labeled compound or prodrug thereof, 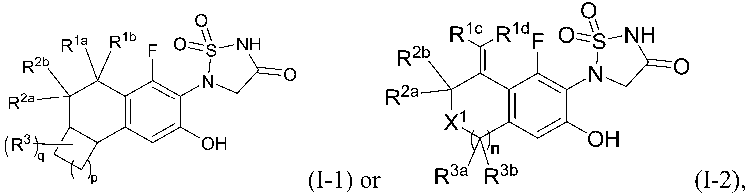 wherein
R^{1a} and R^{1b} are each independently selected from H, D, halogen, -OH, C₁₋₆ alkyl, C₁₋₆ alkoxy, C₁₋₆ haloalkyl, and C₁₋₆ haloalkoxy;
preferably, R^{1a} and R^{1b} are each independently selected from H, D, halogen, -OH, C₁₋₄ alkyl, C₁₋₄ alkoxy, C₁₋₄ haloalkyl, and C₁₋₄ haloalkoxy;
preferably, R^{1a} and R^{1b} are each independently selected from H, D, F, Cl, Br, -OH, methyl, ethyl, methoxy, ethoxy, trifluoromethyl, difluoromethyl, 2,2-difluoroethyl, trifluoromethoxy, and 2,2-difluoroethoxy;
preferably, R^{1a} and R^{1b} are each independently selected from H, D, F, and -OH; R^{1c} and R^{1d} are each independently selected from H, D, halogen, C₁₋₆ alkyl, and C₁₋₆ haloalkyl;
preferably, R^{1c} and R^{1d} are each independently selected from H, D, halogen, C₁₋₄ alkyl, and C₁₋₄ haloalkyl;
preferably, R^{1c} and R^{1d} are each independently selected from H, D, F, Cl, Br, methyl, ethyl, trifluoromethyl, and difluoromethyl;
preferably, R^{1c} and R^{1d} are each independently selected from H, D, and F;
R^{2a} is selected from H, D, -OH, -NH₂, C₁₋₈ alkyl, C₂₋₈ alkenyl, C₃₋₆ cycloalkyl, 5-6 membered heteroaryl, 4-8 membered heterocyclyl, -O-C₁₋₈ alkyl, -O-C₁₋₆ alkylene-C₃₋₆ cycloalkyl, -O-C₁₋₆ alkylene-(4-8 membered heterocyclyl), -O-C(=O)-N(R^{a})-C₁₋₈ alkyl, -O-C(=O)-N(R^{a})-phenyl, -N(R^{a})-C₁₋₈ alkyl, -N(R^{a})-C₃₋₆ cycloalkyl, -N(R^{a})-(4-8 membered heterocyclyl), -N(R^{a})-C(=O)-C₁₋₈ alkyl, -N(R^{a})-C(=O)-O-C₁₋₈ alkyl, -N(R^{a})-C₁₋₆ alkylene-C₃₋₆ cycloalkyl, -N(R^{a})-C₁₋₆ alkylene-Si(R^{c})₃, -N(R^{a})-(C=N(R^{b}))-C₁₋₈ alkyl, -N(R^{a})-S(=O)_{w}-C₁₋₈ alkyl, -N(R^{a})-C₁₋₆ alkylene-(4-8 membered heterocyclyl), - N(R^{a})-C₁₋₆ alkylene-(5-6 membered heteroaryl), -N(R^{a})-C₁₋₆ alkylene-phenyl, -C₁₋₆ alkylene-C₃₋₆ cycloalkyl, -C₁₋₆ alkylene-(4-8 membered heterocyclyl), -C₁₋₆ alkylene-N(R^{a})(R^{b}), -C₁₋₆ alkylene-N(R^{a})-C(=O)-O-C₁₋₈ alkyl, -C₁₋₆ alkylene-N(R^{a})-C₁₋₈ alkyl, - C₁₋₆ alkylene-N(R^{a})-C₁₋₆ alkylene-C₃₋₆ cycloalkyl, -C₁₋₆ alkylene-N(R^{a})-C(=O)-C₁₋₈ alkyl, -C₁₋₆ alkylene-N(R^{a})-C₁₋₆ alkylene-(4-8 membered heterocyclyl), -C₁₋₆ alkylene-N(R^{a})-C₁₋₆ alkylene-(5-6 membered heteroaryl), -C₁₋₆ alkylene-N(R^{a})-C₁₋₆ alkylene-phenyl, -C₁₋₆ alkylene-O-C₁₋₈ alkyl, -C₁₋₆ alkylene-O-C₁₋₆ alkylene-C₃₋₆ cycloalkyl, -C₁₋₆ alkylene-O-C₁₋₆ alkylene-(4-8 membered heterocyclyl), -C₁₋₆ alkylene-O-C₁₋₆ alkylene-N(R^{a})(R^{b}), -S(=O)_{w}-C₁₋₈ alkyl, and -C(=O)-N(R^{a})-C₁₋₈ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h};
preferably, R^{2a} is selected from H, D, -OH, -NH₂, C₁₋₆ alkyl, C₂₋₆ alkenyl, C₃₋₆ cycloalkyl, 5-6 membered heteroaryl, 4-8 membered heterocyclyl, -O-C₁₋₆ alkyl, -O-C₁₋₄ alkylene-C₃₋₆ cycloalkyl, -O-C₁₋₄ alkylene-(4-8 membered heterocyclyl), -O-C(=O)-N(R^{a})-C₁₋₆ alkyl, -O-C(=O)-N(R^{a})-phenyl, -N(R^{a})-C₁₋₆ alkyl, -N(R^{a})-C₃₋₆ cycloalkyl, -N(R^{a})-(4-8 membered heterocyclyl), -N(R^{a})-C(=O)-C₁₋₆ alkyl, -N(R^{a})-C(=O)-O-C₁₋₈ alkyl, -N(R^{a})-C₁₋₄ alkylene-C₃₋₆ cycloalkyl, -N(R^{a})-C₁₋₄ alkylene-Si(R^{c})₃, -N(R^{a})-(C=N(R^{b}))-C₁₋₆ alkyl, -N(R^{a})-S(=O)_{w}-C₁₋₆ alkyl, -N(R^{a})-C₁₋₄ alkylene-(4-8 membered heterocyclyl), -N(R^{a})-C₁₋₄ alkylene-(5-6 membered heteroaryl), -N(R^{a})-C₁₋₄ alkylene-phenyl, -C₁₋₄ alkylene-C₃₋₆ cycloalkyl, -C₁₋₄ alkylene-(4-8 membered heterocyclyl), -C₁₋₄ alkylene-N(R^{a})(R^{b}), -C₁₋₄ alkylene-N(R^{a})-C(=O)-O-C₁₋₆ alkyl, -C₁₋₄ alkylene-N(R^{a})-C₁₋₆ alkyl, -C₁₋₄ alkylene-N(R^{a})-C₁₋₄ alkylene-C₃₋₆ cycloalkyl, -C₁₋₄ alkylene-N(R^{a})-C(=O)-C₁₋₆ alkyl, -C₁₋₄ alkylene-N(R^{a})-C₁₋₄ alkylene-(4-8 membered heterocyclyl), -C₁₋₄ alkylene-N(R^{a})-C₁₋₄ alkylene-(5-6 membered heteroaryl), -C₁₋₄ alkylene-N(R^{a})-C₁₋₄ alkylene-phenyl, -C₁₋₄ alkylene-O-C₁₋₆ alkyl, -C₁₋₄ alkylene-O-C₁₋₄ alkylene-C₃₋₆ cycloalkyl, -C₁₋₄ alkylene-O-C₁₋₄ alkylene-(4-8 membered heterocyclyl), -C₁₋₄ alkylene-O-C₁₋₄ alkylene-N(R^{a})(R^{b}), -S(=O)_{w}-C₁₋₆ alkyl, and -C(=O)-N(R^{a})-C₁₋₆ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h};
preferably, R^{2a} is selected from -O-C₁₋₆ alkyl, -O-C₁₋₄ alkylene-C₃₋₆ cycloalkyl, - O-C₁₋₄ alkylene-(4-8 membered heterocyclyl), -N(R^{a})-C₁₋₆ alkyl, -N(R^{a})-C₃₋₆ cycloalkyl, -N(R^{a})-(4-8 membered heterocyclyl), -N(R^{a})-C(=O)-C₁₋₆ alkyl, -N(R^{a})-C₁₋₄ alkylene-C₃₋₆ cycloalkyl, -N(R^{a})-C₁₋₄ alkylene-(4-8 membered heterocyclyl), -N(R^{a})-C₁₋₄ alkylene-(5-6 membered heteroaryl), -N(R^{a})-C₁₋₄ alkylene-phenyl, -C₁₋₄ alkylene-C₃₋₆ cycloalkyl, -C₁₋₄ alkylene-(4-8 membered heterocyclyl), -C₁₋₄ alkylene-N(R^{a})(R^{b}), -C₁₋₄ alkylene-N(R^{a})-C₁₋₆ alkyl, -C₁₋₄ alkylene-N(R^{a})-C₁₋₄ alkylene-C₃₋₆ cycloalkyl, -C₁₋₄ alkylene-N(R^{a})-C(=O)-C₁₋₆ alkyl, -C₁₋₄ alkylene-N(R^{a})-C₁₋₄ alkylene-(4-8 membered heterocyclyl), -C₁₋₄ alkylene-N(R^{a})-C₁₋₄ alkylene-(5-6 membered heteroaryl), -C₁₋₄ alkylene-N(R^{a})-C₁₋₄ alkylene-phenyl, -C₁₋₄ alkylene-O-C₁₋₆ alkyl, -C₁₋₄ alkylene-O-C₁₋₄ alkylene-C₃₋₆ cycloalkyl, -C₁₋₄ alkylene-O-C₁₋₄ alkylene-(4-8 membered heterocyclyl), -C₁₋₄ alkylene-O-C₁₋₄ alkylene-N(R^{a})(R^{b}), -S(=O)_{w}-C₁₋₆ alkyl, and -C(=O)-N(R^{a})-C₁₋₆ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h};
preferably, R^{2a} is selected from -N(R^{a})-C₁₋₆ alkyl, -N(R^{a})-C₃₋₆ cycloalkyl, -N(R^{a})-(4-8 membered heterocyclyl), -N(R^{a})-C(=O)-C₁₋₆ alkyl, -N(R^{a})-C₁₋₄ alkylene-C₃₋₆ cycloalkyl, -N(R^{a})-C₁₋₄ alkylene-(4-8 membered heterocyclyl), -N(R^{a})-C₁₋₄ alkylene-(5-6 membered heteroaryl), and -N(R^{a})-C₁₋₄ alkylene-phenyl, wherein the substitutable carbon atoms of R^{2a} are each independently and optionally substituted by one or more substituents each independently selected from R^{g};
preferably, R^{2a} is selected from -N(R^{a})-C₁₋₆ alkyl, -N(R^{a})-C₁₋₆ haloalkyl, and - N(R^{a})-C₁₋₄ alkylene-C₃₋₆ cycloalkyl;
preferably, R^{2a} is selected from the following groups: 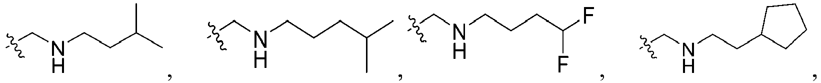 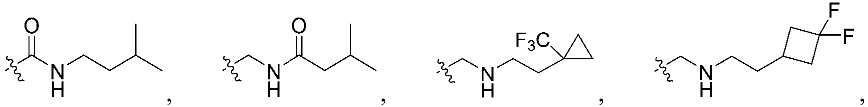 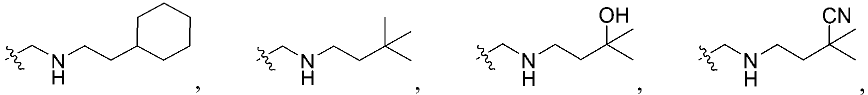 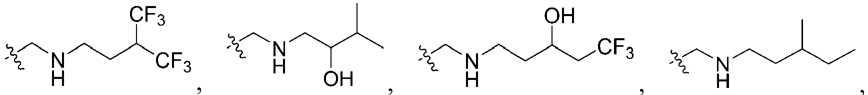 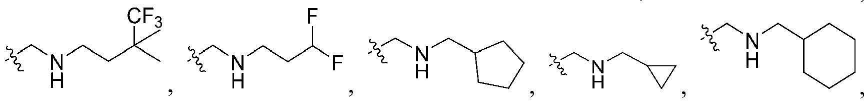 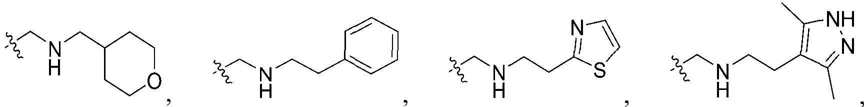 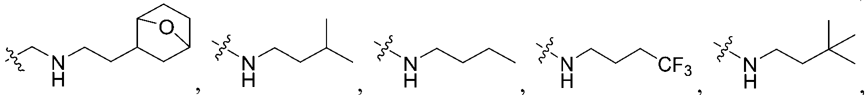 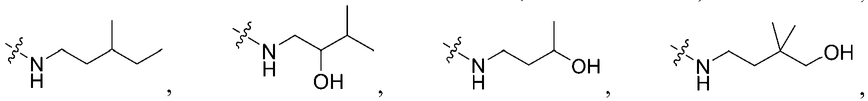 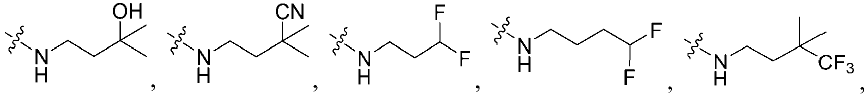 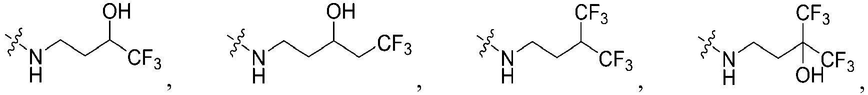 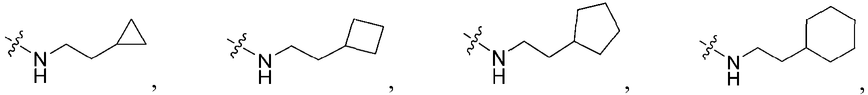 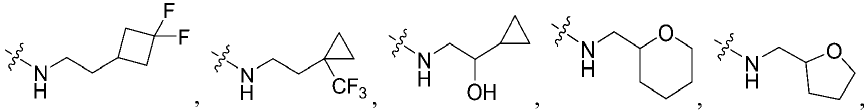 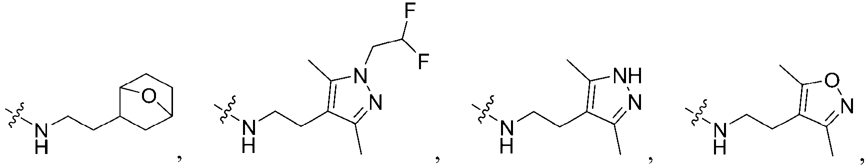 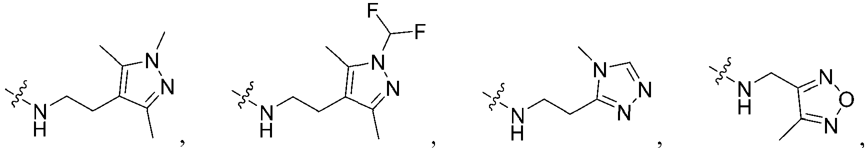 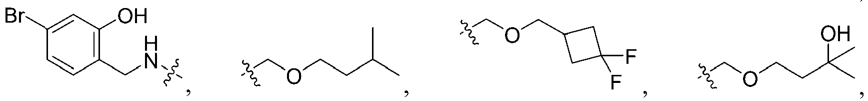 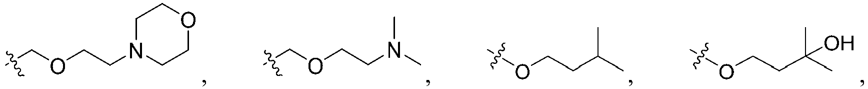 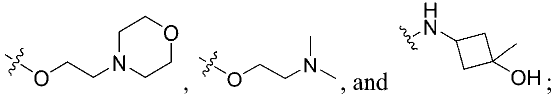
preferably, R^{2a} is selected from the following groups: 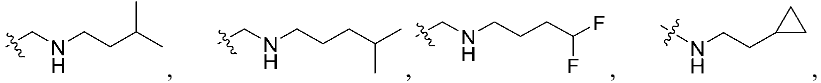 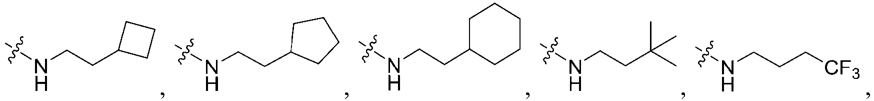 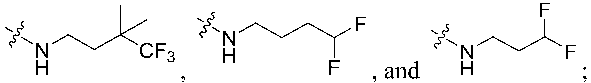
preferably, R^{2a} is selected from the following groups: 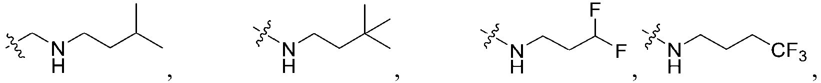 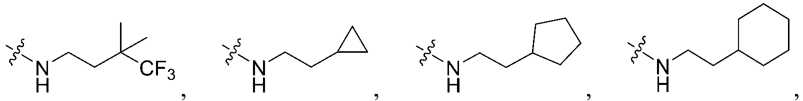 and 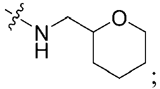
preferably, R^{2a} is selected from the following groups: 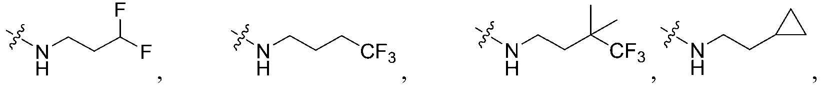 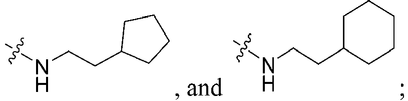
preferably, R^{2a} is 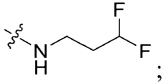
R^{2b} is selected from H, D, -OH, halogen, -N(R^{a})(R^{b}), and -N(R^{a})-N(R^{b})-C(O)-phenyl;
preferably, R^{2b} is selected from H, D, -OH, F, Cl, and Br;
preferably, R^{2b} is selected from H and D; R³, R^{3a}, and R^{3b} are each independently selected from H, D, halogen, -OH, C₁₋₆ alkyl, C₁₋₆ haloalkyl, C₁₋₆ alkoxy, C₁₋₆ haloalkoxy, C₃₋₆ cycloalkyl, C₃₋₆ halocycloalkyl, -NH₂, -NH(C₁₋₆ alkyl), and -N(C₁₋₆ alkyl)(C₁₋₆ alkyl);
preferably, R³, R^{3a}, and R^{3b} are each independently selected from H, D, halogen, - OH, C₁₋₄ alkyl, C₁₋₄ haloalkyl, C₁₋₄ alkoxy, C₁₋₄ haloalkoxy, C₃₋₆ cycloalkyl, C₃₋₆ halocycloalkyl, -NH₂, -NH(C₁₋₄ alkyl), and -N(C₁₋₄ alkyl)(C₁₋₄ alkyl);
preferably, R³, R^{3a}, and R^{3b} are each independently selected from H, D, F, Cl, Br, -OH, C₁₋₄ alkyl, and C₁₋₄ haloalky;
preferably, R³, R^{3a}, and R^{3b} are each independently selected from H, D, F, Cl, Br, -OH, methyl, ethyl, trifluoromethyl, and difluoromethyl;
preferably, R³, R^{3a}, and R^{3b} are each independently selected from H, D, F, and - OH; X¹ is selected from O, S, NR^{f}, and C(R^{e})(R^{d});
R^{a} and R^{b} are each independently selected from H and C₁₋₆ alkyl; optionally, the C₁₋₆ alkyl is substituted by one or more substituents selected from halogen, -CN, oxo, and -OH;
preferably, R^{a} and R^{b} are each independently selected from H and C₁₋₄ alkyl; optionally, the C₁₋₄ alkyl is substituted by one or more substituents selected from halogen, -CN, oxo, and -OH;
preferably, R^{a} and R^{b} are each independently selected from H and methyl;
R^{c} is selected from -OH, C₁₋₆ alkyl, and phenyl;
preferably, R^{c} is selected from -OH, methyl, ethyl, n-propyl, isopropyl, n-butyl, tert-butyl, and phenyl;
R^{e} and R^{d} are each independently selected from H, D, halogen, -OH, C₁₋₆ alkyl, C₁₋₆ haloalkyl, C₁₋₆ alkoxy, C₁₋₆ haloalkoxy, C₃₋₆ cycloalkyl, C₃₋₆ halocycloalkyl, -NH₂, -NH(C₁₋₆ alkyl), and -N(C₁₋₆ alkyl)(C₁₋₆ alkyl); optionally, R^{e} and R^{3a} together with the atoms to which they connect form a 3-7 membered carbocyclic ring; or R^{e} and R^{2b} together with the atoms to which they connect form a 3-7 membered carbocyclic ring;
preferably, R^{e} and R^{d} are each independently selected from H, D, F, Cl, Br, -OH, C₁₋₄ alkyl, C₁₋₄ haloalkyl, C₁₋₄ alkoxy, C₁₋₄ haloalkoxy, C₃₋₆ cycloalkyl, C₃₋₆ halocycloalkyl, -NH₂, -NH(C₁₋₄ alkyl), and -N(C₁₋₄ alkyl)(C₁₋₄ alkyl); optionally, R^{e} and R^{3a} together with the atoms to which they connect form a 3-6 membered carbocyclic ring; or R^{e} and R^{2b} together with the atoms to which they connect form a 3-6 membered carbocyclic ring;
R^{f} is selected from H, C₁₋₆ alkyl, and C₁₋₆ haloalkyl; optionally, R^{f} and R^{3a} together with the atoms to which they connect form a 3-7 membered heterocyclic ring; or R^{f} and R^{2b} together with the atoms to which they connect form a 3-7 membered heterocyclic ring;
preferably, R^{f} is selected from H, C₁₋₄ alkyl, and C₁₋₄ haloalkyl; optionally, R^{f} and R^{3a} together with the atoms to which they connect form a 3-6 membered heterocyclic ring; or R^{f} and R^{2b} together with the atoms to which they connect form a 3-6 membered heterocyclic ring;
each R^{g} is independently selected from H, D, halogen, -OH, -CN, nitro, oxo, -NH₂, -NH(C₁₋₆ alkyl), -N(C₁₋₆ alkyl)(C₁₋₆ alkyl), C₁₋₆ alkyl, and C₁₋₆ haloalkyl;
preferably, each R^{g} is independently selected from H, D, F, Cl, Br, -OH, -CN, nitro, oxo, -NH₂, -NH(C₁₋₄ alkyl), -N(C₁₋₄ alkyl)(C₁₋₄ alkyl), C₁₋₄ alkyl, and C₁₋₄ haloalkyl;
preferably, each R^{g} is independently selected from H, D, F, Cl, Br, -OH, -CN, nitro, oxo, -NH₂, -NH-CH₃, -N(CH₃)(CH₃), methyl, ethyl, trifluoromethyl, and difluoromethyl;
R^{h} is selected from H, D, C₁₋₆ alkyl, and C₁₋₆ haloalkyl;
preferably, R^{h} is selected from H, D, C₁₋₄ alkyl, and C₁₋₄ haloalkyl;
preferably, R^{h} is selected from H, D, methyl, trifluoromethyl, difluoromethyl, and 2,2-difluoroethyl;
n is selected from 0 or 1;
p is selected from 0, 1, 2, or 3;
q is selected from 0, 1, 2, 3, or 4; and
w is selected from 1 or 2,
provided that the compound is not

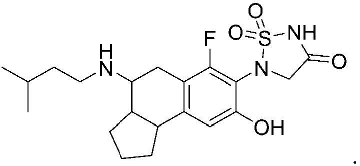

2. The compound or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof according to claim 1, wherein
R^{1a} and R^{1b} are each independently selected from H and D, preferably, R^{1a} and R^{1b} are selected from H; and/or
R^{1c} and R^{1d} are each independently selected from H, D, and halogen, preferably, R^{1c} and R^{1d} are each independently selected from H and halogen, more preferably, one of R^{1c} and R^{1d} is selected from H, and the other is selected from halogen; and/or
p is selected from 0 or 1, preferably, p is selected from 0; and/or
n is selected from 0; and/or
R³ is selected from H, D, and halogen, preferably, R³ is selected from H and halogen, more preferably, R³ is selected from H; and/or
q is selected from 0, 1, or 2, preferably, q is selected from 0; and/or
X¹ is selected from O, S, NR^{f}, and C(R^{e})(R^{d}), preferably, X¹ is selected from C(R^{e})(R^{d}); and/or
R^{e} and R^{d} are each independently selected from H, D, halogen, and -OH, or R^{e} and R^{2b} together with the atoms to which they connect form a 3-7 membered carbocyclic ring, preferably, R^{e} and R^{d} are each independently selected from H, halogen, and -OH, or R^{e} and R^{2b} together with the atoms to which they connect form a 3-6 membered carbocyclic ring (such as C₃₋₆ cycloalkyl), more preferably, R^{e} and R^{d} are selected from H; and
R^{2b} is selected from H, or R^{2b} and R^{e} together with the atoms to which they connect form a 3-6 membered carbocyclic ring (such as C₃₋₆ cycloalkyl), and preferably, R^{2b} is selected from H.

3. The compound or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof according to claim 1 or 2, wherein
R^{2a} is selected from -N(R^{a})-C₁₋₈ alkyl, -N(R^{a})-C₃₋₆ cycloalkyl, -N(R^{a})-C₁₋₆ alkylene-C₃₋₆ cycloalkyl, -N(R^{a})-C₁₋₆ alkylene-(4-8 membered heterocyclyl), -N(R^{a})-C₁₋₆ alkylene-(5-6 membered heteroaryl), -N(R^{a})-C₁₋₆ alkylene-phenyl, -O-C₁₋₈ alkyl, - O-C₁₋₆ alkylene-(4-8 membered heterocyclyl), -O-C₁₋₆ alkylene-C₃₋₆ cycloalkyl, - N(R^{a})-(4-8 membered heterocyclyl), -C₁₋₆ alkylene-N(R^{a})-C₁₋₈ alkyl, -C₁₋₆ alkylene-N(R^{a})-C₁₋₆ alkylene-C₃₋₆ cycloalkyl, -C₁₋₆ alkylene-N(R^{a})-C₁₋₆ alkylene-(4-8 membered heterocyclyl), -C₁₋₆ alkylene-N(R^{a})-C₁₋₆ alkylene-(5-6 membered heteroaryl), -C₁₋₆ alkylene-N(R^{a})-C₁₋₆ alkylene-phenyl, -C₁₋₆ alkylene-O-C₁₋₈ alkyl, -C₁₋₆ alkylene-O-C₁₋₆ alkylene-C₃₋₆ cycloalkyl, -C₁₋₆ alkylene-O-C₁₋₆ alkylene-(4-8 membered heterocyclyl), -C(=O)-N(R^{a})-C₁₋₈ alkyl, -C₁₋₆ alkylene-N(R^{a})-C(=O)-C₁₋₈ alkyl, -C₁₋₆ alkylene-N(R^{a})(R^{b}), -C₁₋₆ alkylene-O-C₁₋₆ alkylene-N(R^{a})(R^{b}), and -N(R^{a})-C(=O)-C₁₋₈ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h};
preferably, R^{2a} is selected from -N(R^{a})-C₁₋₆ alkyl, -N(R^{a})-C₃₋₆ cycloalkyl, -N(R^{a})-C₁₋₄ alkylene-C₃₋₆ cycloalkyl, -N(R^{a})-C₁₋₄ alkylene-(4-7 membered heterocyclyl), - N(R^{a})-C₁₋₄ alkylene-(5-6 membered heteroaryl), -N(R^{a})-C₁₋₄ alkylene-phenyl, -O-C₁₋₆ alkyl, -O-C₁₋₄ alkylene-(4-7 membered heterocyclyl), -O-C₁₋₄ alkylene-C₃₋₆ cycloalkyl, -N(R^{a})-(4-7 membered heterocyclyl), -C₁₋₄ alkylene-N(R^{a})-C₁₋₆ alkyl, -C₁₋₄ alkylene-N(R^{a})-C₁₋₄ alkylene-C₃₋₆ cycloalkyl, -C₁₋₄ alkylene-N(R^{a})-C₁₋₄ alkylene-(4-7 membered heterocyclyl), -C₁₋₄ alkylene-N(R^{a})-C₁₋₄ alkylene-(5-6 membered heteroaryl), -C₁₋₄ alkylene-N(R^{a})-C₁₋₄ alkylene-phenyl, -C₁₋₄ alkylene-O-C₁₋₆ alkyl, -C₁₋₄ alkylene-O-C₁₋₄ alkylene-C₃₋₆ cycloalkyl, -C₁₋₄ alkylene-O-C₁₋₄ alkylene-(4-7 membered heterocyclyl), -C(=O)-N(R^{a})-C₁₋₆ alkyl, -C₁₋₄ alkylene-N(R^{a})-C(=O)-C₁₋₆ alkyl, -C₁₋₄ alkylene-N(R^{a})(R^{b}), -C₁₋₄ alkylene-O-C₁₋₄ alkylene-N(R^{a})(R^{b}), and -N(R^{a})-C(=O)-C₁₋₆ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h};
preferably, R^{2a} is selected from -N(R^{a})-C₁₋₈ alkyl, -N(R^{a})-C₃₋₆ cycloalkyl, -N(R^{a})-C₁₋₆ alkylene-C₃₋₆ cycloalkyl, -N(R^{a})-C₁₋₆ alkylene-(4-8 membered heterocyclyl), - N(R^{a})-C₁₋₆ alkylene-(5-6 membered heteroaryl), -N(R^{a})-C₁₋₆ alkylene-phenyl, -O-C₁₋₈ alkyl, -O-C₁₋₆ alkylene-(4-8 membered heterocyclyl), -C₁₋₆ alkylene-N(R^{a})-C₁₋₈ alkyl, - C₁₋₆ alkylene-N(R^{a})-C₁₋₆ alkylene-C₃₋₆ cycloalkyl, -C₁₋₆ alkylene-N(R^{a})-C₁₋₆ alkylene-(4-8 membered heterocyclyl), -C₁₋₆ alkylene-N(R^{a})-C₁₋₆ alkylene-(5-6 membered heteroaryl), -C₁₋₆ alkylene-N(R^{a})-C₁₋₆ alkylene-phenyl, -C₁₋₆ alkylene-O-C₁₋₈ alkyl, -C₁₋₆ alkylene-O-C₁₋₆ alkylene-C₃₋₆ cycloalkyl, -C₁₋₆ alkylene-O-C₁₋₆ alkylene-(4-8 membered heterocyclyl), -C(=O)-N(R^{a})-C₁₋₈ alkyl, and -C₁₋₆ alkylene-N(R^{a})-C(=O)-C₁₋₈ alkyl, wherein the substitutable carbon atoms of R^{2a} are each independently and optionally substituted by one or more substituents each independently selected from R^{g}, and/or the substitutable nitrogen atoms on the ring of R^{2a} are each independently and optionally substituted by one or more substituents each independently selected from R^{h};
preferably, R^{2a} is selected from -N(R^{a})-C₁₋₆ alkyl, -N(R^{a})-C₃₋₆ cycloalkyl, -N(R^{a})-C₁₋₄ alkylene-C₃₋₆ cycloalkyl, -N(R^{a})-C₁₋₄ alkylene-(4-7 membered heterocyclyl), - N(R^{a})-C₁₋₄ alkylene-(5-6 membered heteroaryl), -N(R^{a})-C₁₋₄ alkylene-phenyl, -O-C₁₋₆ alkyl, -O-C₁₋₄ alkylene-(4-7 membered heterocyclyl), -C₁₋₄ alkylene-N(R^{a})-C₁₋₆ alkyl, - C₁₋₄ alkylene-N(R^{a})-C₁₋₄ alkylene-C₃₋₆ cycloalkyl, -C₁₋₄ alkylene-N(R^{a})-C₁₋₄ alkylene-(4-7 membered heterocyclyl), -C₁₋₄ alkylene-N(R^{a})-C₁₋₄ alkylene-(5-6 membered heteroaryl), -C₁₋₄ alkylene-N(R^{a})-C₁₋₄ alkylene-phenyl, -C₁₋₄ alkylene-O-C₁₋₆ alkyl, -C₁₋₄ alkylene-O-C₁₋₄ alkylene-C₃₋₆ cycloalkyl, -C₁₋₄ alkylene-O-C₁₋₄ alkylene-(4-7 membered heterocyclyl), -C(=O)-N(R^{a})-C₁₋₆ alkyl, and -C₁₋₄ alkylene-N(R^{a})-C(=O)-C₁₋₆ alkyl, wherein the substitutable carbon atoms of R^{2a} are each independently and optionally substituted by one or more substituents each independently selected from R^{g}, and/or the substitutable nitrogen atoms on the ring of R^{2a} are each independently and optionally substituted by one or more substituents each independently selected from R^{h};
preferably, R^{2a} is selected from -N(R^{a})-C₁₋₈ alkyl, -N(R^{a})-C₁₋₆ alkylene-C₃₋₆ cycloalkyl, -N(R^{a})-C₁₋₆ alkylene-(4-8 membered heterocyclyl), -N(R^{a})-C₁₋₆ alkylene-phenyl, and -C₁₋₆ alkylene-N(R^{a})-C₁₋₈ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h};
preferably, R^{2a} is selected from -N(R^{a})-C₁₋₆ alkyl, -N(R^{a})-C₁₋₄ alkylene-C₃₋₆ cycloalkyl, -N(R^{a})-C₁₋₄ alkylene-(4-7 membered heterocyclyl), -N(R^{a})-C₁₋₄ alkylene-phenyl, and -C₁₋₄ alkylene-N(R^{a})-C₁₋₆ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h};
more preferably, R^{2a} is selected from -N(R^{a})-C₁₋₈ alkyl, -N(R^{a})-C₁₋₆ alkylene-C₃₋₆ cycloalkyl, -N(R^{a})-C₁₋₆ alkylene-(4-8 membered heterocyclyl), and -C₁₋₆ alkylene-N(R^{a})-C₁₋₈ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h};
more preferably, R^{2a} is selected from -N(R^{a})-C₁₋₆ alkyl, -N(R^{a})-C₁₋₄ alkylene-C₃₋₆ cycloalkyl, -N(R^{a})-C₁₋₄ alkylene-(4-7 membered heterocyclyl), and -C₁₋₄ alkylene-N(R^{a})-C₁₋₆ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}; and/or
each R^{g} is independently selected from H, D, halogen, -OH, -CN, -NH₂, -NH(C₁₋₆ alkyl), -N(C₁₋₆ alkyl)(C₁₋₆ alkyl), C₁₋₆ alkyl, and C₁₋₆ haloalkyl;
preferably, each R^{g} is independently selected from H, D, halogen, -OH, -CN, -NH₂, -NH(C₁₋₄ alkyl), -N(C₁₋₄ alkyl)(C₁₋₄ alkyl), C₁₋₄ alkyl, and C₁₋₄ haloalkyl; and/or R^{h} is selected from H, D, C₁₋₆ alkyl, and C₁₋₆ haloalkyl;
preferably, R^{h} is selected from H, D, C₁₋₄ alkyl, and C₁₋₄ haloalkyl; and/or
R^{a} and R^{b} are each independently selected from H and C₁₋₆ alkyl; optionally, the C₁₋₆ alkyl is substituted by one or more substituents selected from halogen, preferably, R^{a} and R^{b} are each independently selected from H and C₁₋₄ alkyl; and optionally, the C₁₋₄ alkyl is substituted by one or more substituents selected from halogen.

4. The compound or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof according to any one of claims 1-3, wherein the compound has a structure represented by formula (II-0) or (II-2): 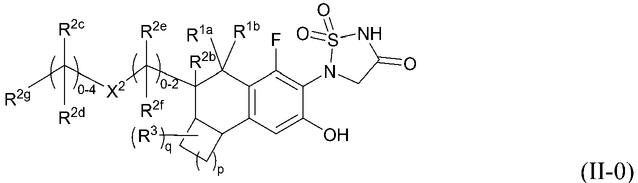 or 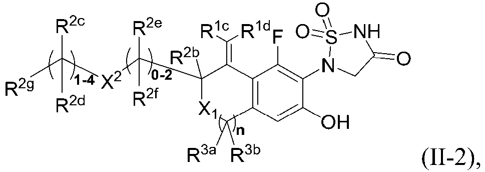 Wherein,
R^{2c}, R^{2d}, R^{2e}, and R^{2f} are each independently selected from H, D, F, Cl, -CN, -OH, C₁₋₆ alkyl, and C₁₋₆ haloalkyl; optionally R^{2c} and R^{2d} form an oxo, or R^{2e} and R^{2f} form an oxo;
preferably, R^{2c}, R^{2d}, R^{2e}, and R^{2f} are each independently selected from H, D, F, Cl, -CN, -OH, C₁₋₄ alkyl, and C₁₋₄ haloalkyl; optionally R^{2c} and R^{2d} form an oxo, or R^{2e} and R^{2f} form an oxo;
preferably, R^{2c}, R^{2d}, R^{2e} and R^{2f} are each independently selected from H, D, F, Cl, -CN, -OH, methyl, ethyl, and trifluoromethyl; optionally, R^{2c} and R^{2d} form an oxo, or R^{2e} and R^{2f} form an oxo;
R^{2g} is selected from H, C₁₋₄ alkyl, C₃₋₆ cycloalkyl, 4-8 membered heterocyclyl, 5-6 membered heteroaryl, phenyl, -NH₂, -NH(C₁₋₆ alkyl), and -N(C₁₋₆ alkyl)(C₁₋₆ alkyl); the C₁₋₄ alkyl, C₃₋₆ cycloalkyl, 4-8 membered heterocyclyl, 5-6 membered heteroaryl, and phenyl are each independently and optionally substituted by one or more substituents selected from halogen, -OH, -CN, C₁₋₄ alkyl, and C₁₋₄ haloalkyl;
preferably, R^{2g} is selected from H, C₁₋₄ alkyl, C₃₋₆ cycloalkyl, 4-8 membered heterocyclyl, 5-6 membered heteroaryl, phenyl, -NH₂, -NH(C₁₋₆ alkyl), and -N(C₁₋₆ alkyl)(C₁₋₆ alkyl); the C₁₋₄ alkyl, C₃₋₆ cycloalkyl, 4-8 membered heterocyclyl, 5-6 membered heteroaryl, and phenyl are each independently and optionally substituted by one or more substituents selected from F, Cl, Br, -OH, -CN, methyl, trifluoromethyl, difluoromethyl, and 2,2-difluoroethyl;
preferably, R^{2g} is selected from H, C₁₋₄ alkyl, C₃₋₆ cycloalkyl, 4-7 membered heterocyclyl, 5-6 membered heteroaryl, and phenyl; the C₁₋₄ alkyl, C₃₋₆ cycloalkyl, 4-7 membered heterocyclyl, 5-6 membered heteroaryl, and phenyl are each independently and optionally substituted by one or more substituents selected from halogen, -OH, - CN, C₁₋₄ alkyl, and C₁₋₄ haloalkyl;
preferably, R^{2g} is selected from H, C₁₋₄ alkyl, C₃₋₆ cycloalkyl, and 4-7 membered heterocyclyl; the C₁₋₄ alkyl, C₃₋₆ cycloalkyl, and 4-7 membered heterocyclyl are each independently and optionally substituted by one or more substituents selected from halogen;
preferably, R^{2g} is selected from H, methyl, trifluoromethyl, difluoromethyl, tert-butyl, -CH₂OH, -N(CH₃)₂, or the following groups: 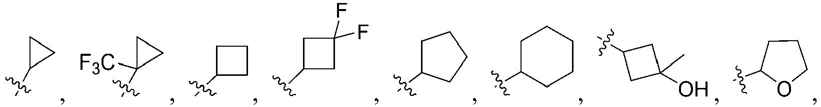 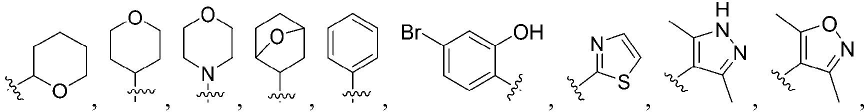 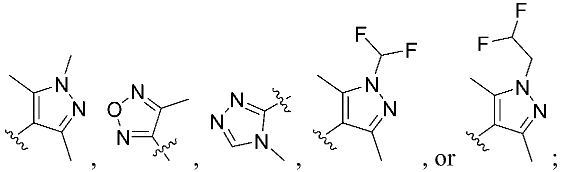
preferably, R^{2g} is selected from H, methyl, tert-butyl, trifluoromethyl, cyclopropyl, cyclopentyl, cyclohexyl, and tetrahydropyran-2-yl;
X² is NH or O, preferably, X² is NH;
preferably, formula (II-0) has a structure represented by formula (II-1):

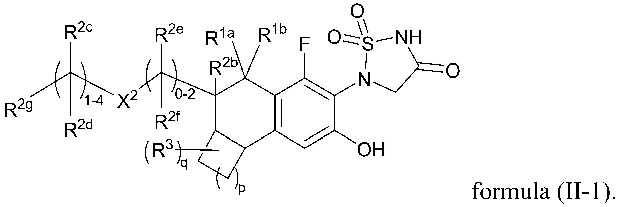

5. The compound or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof according to any one of claims 1-4, wherein the compound has a structure represented by formula (III-0) or (III-2): 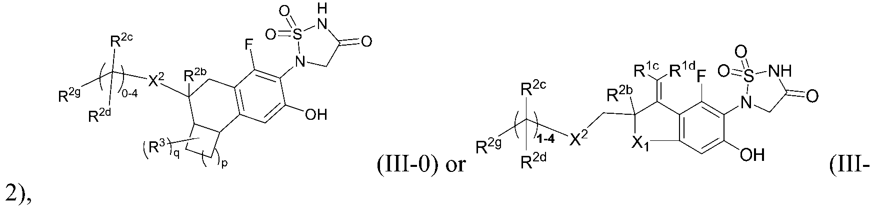 preferably, formula (III-0) has a structure represented by formula (III-1):

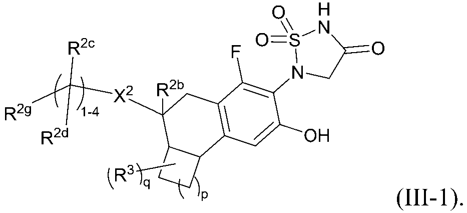

6. The compound or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof according to any one of claims 1-5, wherein the compound has a structure represented by formula (IV-1) or (IV-2): 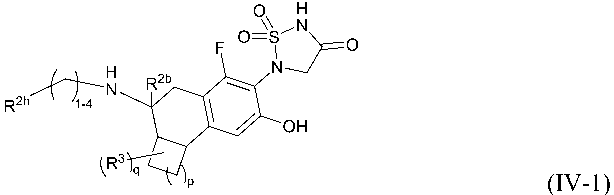 or 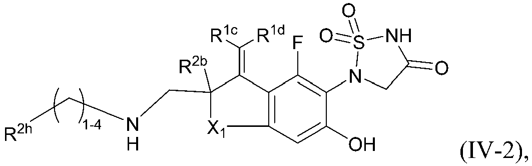
wherein R^{2h} is selected from C₁₋₆ alkyl, C₃₋₆ cycloalkyl, 4-7 membered heterocyclyl, 5-6 membered heteroaryl, phenyl, -C₁₋₄ alkylene-C₃₋₆ cycloalkyl, -C₁₋₄ alkylene-(4-7 membered heterocyclyl), -C₁₋₄ alkylene-(5-6 membered heteroaryl), -C₁₋₄ alkylene-phenyl, -NH(C₁₋₆ alkyl), and -N(C₁₋₆ alkyl)₂; the C₁₋₆ alkyl, C₃₋₆ cycloalkyl, 4-7 membered heterocyclyl, 5-6 membered heteroaryl, phenyl, -C₁₋₄ alkylene-C₃₋₆ cycloalkyl, -C₁₋₄ alkylene-(4-7 membered heterocyclyl), -C₁₋₄ alkylene-(5-6 membered heteroaryl), -C₁₋₄ alkylene-phenyl, -NH(C₁₋₆ alkyl), and -N(C₁₋₆ alkyl)₂ are each independently and optionally substituted by one or more substituents selected from halogen, -OH, -CN, C₁₋₄ alkyl, and C₁₋₄ haloalkyl;
preferably, R^{2h} is selected from C₁₋₆ alkyl, C₃₋₆ cycloalkyl, 4-7 membered heterocyclyl, 5-6 membered heteroaryl, phenyl, -C₁₋₂ alkylene-C₃₋₆ cycloalkyl, -C₁₋₂ alkylene-(4-7 membered heterocyclyl), -C₁₋₂ alkylene-(5-6 membered heteroaryl), -C₁₋₂ alkylene-phenyl, -NH(C₁₋₆ alkyl), and -N(C₁₋₆ alkyl)₂; the C₁₋₆ alkyl, C₃₋₆ cycloalkyl, 4-7 membered heterocyclyl, 5-6 membered heteroaryl, phenyl, -C₁₋₂ alkylene-C₃₋₆ cycloalkyl, -C₁₋₂ alkylene-(4-7 membered heterocyclyl), -C₁₋₂ alkylene-(5-6 membered heteroaryl), -C₁₋₂ alkylene-phenyl, -NH(C₁₋₆ alkyl), and -N(C₁₋₆ alkyl)₂ are each independently and optionally substituted by one or more substituents selected from halogen, -OH, -CN, C₁₋₄ alkyl, and C₁₋₄ haloalkyl;
preferably, R^{2h} is selected from C₁₋₆ alkyl, C₃₋₆ cycloalkyl, 4-7 membered heterocyclyl, 5-6 membered heteroaryl, phenyl, -C₁₋₂ alkylene-C₃₋₆ cycloalkyl, and - N(C₁₋₆ alkyl)₂; the C₁₋₆ alkyl, C₃₋₆ cycloalkyl, 4-7 membered heterocyclyl, 5-6 membered heteroaryl, phenyl, -C₁₋₂ alkylene-C₃₋₆ cycloalkyl, and -N(C₁₋₆ alkyl)₂ are each independently and optionally substituted by one or more substituents selected from halogen, -OH, -CN, C₁₋₄ alkyl, and C₁₋₄ haloalkyl;
preferably, R^{2h} is selected from C₁₋₆ alkyl, C₃₋₆ cycloalkyl, and 4-7 membered heterocyclyl; the C₁₋₆ alkyl, C₃₋₆ cycloalkyl, and 4-7 membered heterocyclyl are each independently and optionally substituted by one or more substituents selected from halogen;
preferably, R^{2h} is selected from methyl, ethyl, isopropyl, tert-butyl, difluoromethyl, trifluoromethyl, 2-(1,1,1-trifluoromethyl)-propan-2-yl, cyclopropyl, cyclobutyl, cyclopentyl, cyclohexyl, phenyl, 2-hydroxy-propan-2-yl, 2-cyano-propan-2-yl, - N(CH₃)₂, 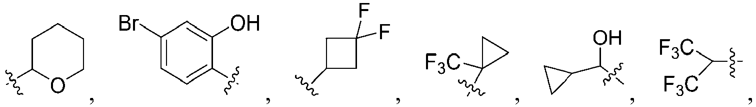 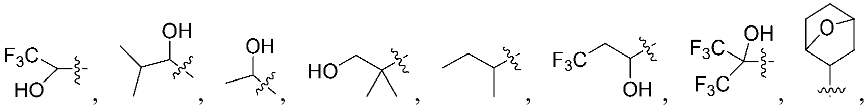 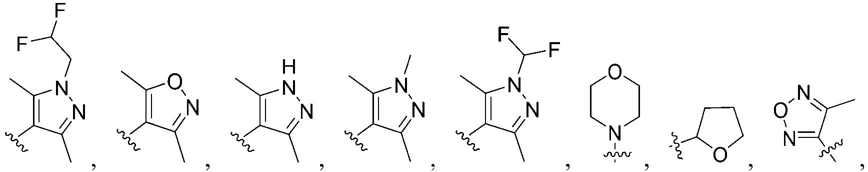 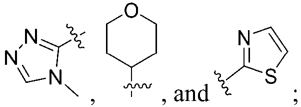 and
preferably, R^{2h} is selected from isopropyl, tert-butyl, trifluoromethyl, 2-(1,1,1-trifluoromethyl)-propan-2-yl, cyclopropyl, cyclopentyl, cyclohexyl, and tetrahydropyran-2-yl.

7. The compound or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof according to any one of claims 1-3, wherein the compound has a structure represented by formula (V):

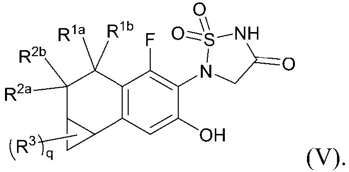

8. The compound or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof according to claim 7, wherein
R^{2a} is selected from -N(R^{a})-C₁₋₈ alkyl, -N(R^{a})-C₃₋₆ cycloalkyl, -N(R^{a})-C₁₋₆ alkylene-C₃₋₆ cycloalkyl, -N(R^{a})-C₁₋₆ alkylene-(4-8 membered heterocyclyl), -N(R^{a})-C₁₋₆ alkylene-(5-6 membered heteroaryl), -N(R^{a})-C₁₋₆ alkylene-phenyl, -O-C₁₋₈ alkyl, - O-C₁₋₆ alkylene-(4-8 membered heterocyclyl), -O-C₁₋₆ alkylene-C₃₋₆ cycloalkyl, and - N(R^{a})-(4-8 membered heterocyclyl), wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h};
preferably, R^{2a} is selected from -N(R^{a})-C₁₋₆ alkyl, -N(R^{a})-C₃₋₆ cycloalkyl, -N(R^{a})-C₁₋₄ alkylene-C₃₋₆ cycloalkyl, -N(R^{a})-C₁₋₄ alkylene-(4-7 membered heterocyclyl), - N(R^{a})-C₁₋₄ alkylene-(5-6 membered heteroaryl), -N(R^{a})-C₁₋₄ alkylene-phenyl, -O-C₁₋₆ alkyl, -O-C₁₋₄ alkylene-(4-7 membered heterocyclyl), -O-C₁₋₄ alkylene-C₃₋₆ cycloalkyl, and -N(R^{a})-(4-7 membered heterocyclyl), wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h};
preferably, R^{2a} is selected from -N(R^{a})-C₁₋₈ alkyl, -N(R^{a})-C₃₋₆ cycloalkyl, -N(R^{a})-C₁₋₆ alkylene-C₃₋₆ cycloalkyl, -N(R^{a})-C₁₋₆ alkylene-(4-8 membered heterocyclyl), - N(R^{a})-C₁₋₆ alkylene-(5-6 membered heteroaryl), -N(R^{a})-C₁₋₆ alkylene-phenyl, -O-C₁₋₈ alkyl, and -O-C₁₋₆ alkylene-(4-8 membered heterocyclyl), wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h};
preferably, R^{2a} is selected from -N(R^{a})-C₁₋₆ alkyl, -N(R^{a})-C₃₋₆ cycloalkyl, -N(R^{a})-C₁₋₄ alkylene-C₃₋₆ cycloalkyl, -N(R^{a})-C₁₋₄ alkylene-(4-7 membered heterocyclyl), - N(R^{a})-C₁₋₄ alkylene-(5-6 membered heteroaryl), -N(R^{a})-C₁₋₄ alkylene-phenyl, -O-C₁₋₆ alkyl, and -O-C₁₋₄ alkylene-(4-7 membered heterocyclyl), wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h};
preferably, R^{2a} is selected from -N(R^{a})-C₁₋₈ alkyl, -N(R^{a})-C₁₋₆ alkylene-C₃₋₆ cycloalkyl, and -N(R^{a})-C₁₋₆ alkylene-(4-8 membered heterocyclyl), wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h};
preferably, R^{2a} is selected from -N(R^{a})-C₁₋₆ alkyl, -N(R^{a})-C₁₋₄ alkylene-C₃₋₆ cycloalkyl, and -N(R^{a})-C₁₋₄ alkylene-(4-7 membered heterocyclyl), wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}; and/or
each R^{g} is independently selected from H, D, halogen, -OH, -CN, -NH₂, -NH(C₁₋₆ alkyl), -N(C₁₋₆ alkyl)(C₁₋₆ alkyl), C₁₋₆ alkyl, and C₁₋₆ haloalkyl;
preferably, each R^{g} is independently selected from H, D, F, Cl, Br, -OH, -CN, - NH₂, -NH(C₁₋₄ alkyl), -N(C₁₋₄ alkyl)(C₁₋₄ alkyl), C₁₋₄ alkyl, and C₁₋₄ haloalkyl;
preferably, each R^{g} is independently selected from H, F, -OH, -CN, -N(CH₃)₂, methyl, ethyl, trifluoromethyl, and difluoromethyl; and/or
R^{h} is selected from H, D, C₁₋₆ alkyl, and C₁₋₆ haloalkyl;
preferably, R^{h} is selected from H, D, C₁₋₄ alkyl, and C₁₋₄ haloalkyl;
preferably, R^{h} is selected from H, D, methyl, trifluoromethyl, difluoromethyl, and 2,2-difluoroethyl; and/or
R^{a} is independently selected from H and C₁₋₆ alkyl; optionally, the C₁₋₆ alkyl is substituted by one or more substituents selected from halogen, -CN, oxo, and -OH;
preferably, R^{a} is independently selected from H and C₁₋₄ alkyl; optionally, the C₁₋₄ alkyl is substituted by one or more substituents selected from halogen, -CN, oxo, and -OH; and
preferably, R^{a} is H.

9. The compound or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof according to any one of claims 1-5 and 7-8, wherein the compound has a structure represented by formula (V-1):

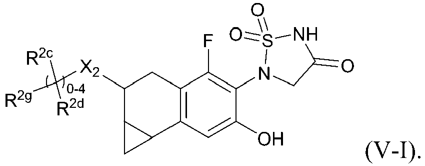

10. The compound or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof according to claim 9, wherein X² is NH;
preferably, R^{2c} and R^{2d} are each independently selected from H, F, -CN, -OH, C₁₋₃ alkyl, and C₁₋₃ haloalkyl;
more preferably, R^{2c} and R^{2d} are each independently selected from H, F, -CN, - OH, methyl, ethyl, and trifluoromethyl; and
preferably, R^{2g} is selected from H, C₁₋₃ alkyl, C₃₋₆ cycloalkyl, 4-7 membered heterocyclyl, phenyl, 5-6 membered heteroaryl (such as pyrazolyl, isoxazolyl, triazolyl, and oxadiazolyl); and the C₁₋₃ alkyl, C₃₋₆ cycloalkyl, 4-7 membered heterocyclyl, phenyl, 5-6 membered heteroaryl are each independently and optionally substituted by one or more substituents selected from halogen, -OH, -CN, C₁₋₃ alkyl, and C₁₋₃ haloalkyl.

11. The compound or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof according to any one of claims 1-3, wherein the compound has a structure represented by formula (VI-0):

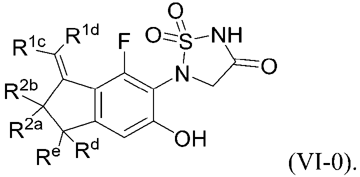

12. The compound or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof according to claim 11, wherein
R^{2a} is selected from -C₁₋₄ alkylene-N(R^{a})-C₁₋₆ alkyl, -C₁₋₄ alkylene-N(R^{a})-C₁₋₄ alkylene-C₃₋₆ cycloalkyl, C₁₋₄ alkylene-N(R^{a})-C₁₋₄ alkylene-(4-7 membered heterocyclyl), -C₁₋₄ alkylene-N(R^{a})-C₁₋₄ alkylene-(5-6 membered heteroaryl), -C₁₋₄ alkylene-N(R^{a})-C₁₋₄ alkylene-phenyl, -C₁₋₄ alkylene-O-C₁₋₆ alkyl, -C₁₋₄ alkylene-O-C₁. ₄ alkylene-C₃₋₆ cycloalkyl, -C₁₋₄ alkylene-O-C₁₋₄ alkylene-(4-7 membered heterocyclyl), -C(=O)-N(R^{a})-C₁₋₆ alkyl, -C₁₋₄ alkylene-N(R^{a})-C(=O)-C₁₋₆ alkyl, -C₁₋₄ alkylene-N(R^{a})(R^{b}), -C₁₋₄ alkylene-O-C₁₋₄ alkylene-N(R^{a})(R^{b}), and -N(R^{a})-C(=O)-C₁₋₆ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h};
preferably, R^{2a} is selected from -C₁₋₂ alkylene-N(R^{a})-C₁₋₆ alkyl, -C₁₋₂ alkylene-N(R^{a})-C₁₋₄ alkylene-C₃₋₆ cycloalkyl, -C₁₋₂ alkylene-N(R^{a})-C₁₋₄ alkylene-(4-7 membered heterocyclyl), -C₁₋₂ alkylene-N(R^{a})-C₁₋₄ alkylene-(5-6 membered heteroaryl), -C₁₋₂ alkylene-N(R^{a})-C₁₋₄ alkylene-phenyl, -C₁₋₂ alkylene-O-C₁₋₆ alkyl, -C₁₋₂ alkylene-O-C₁₋₄ alkylene-C₃₋₆ cycloalkyl, -C₁₋₂ alkylene-O-C₁₋₄ alkylene-(4-7 membered heterocyclyl), -C(=O)-N(R^{a})-C₁₋₆ alkyl, -C₁₋₂ alkylene-N(R^{a})-C(=O)-C₁₋₆ alkyl, and -C₁₋₂ alkylene-O-C₁₋₄ alkylene-N(R^{a})(R^{b}), wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h};
preferably, R^{2a} is selected from -C₁₋₂ alkylene-N(R^{a})-C₁₋₆ alkyl, -C₁₋₂ alkylene-N(R^{a})-C₁₋₄ alkylene-C₃₋₆ cycloalkyl, -C₁₋₂ alkylene-N(R^{a})-C₁₋₄ alkylene-(4-7 membered heterocyclyl), -C₁₋₂ alkylene-N(R^{a})-C₁₋₄ alkylene-(5-6 membered heteroaryl), and -C₁₋₂ alkylene-N(R^{a})-C₁₋₄ alkylene-phenyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h};
more preferably, R^{2a} is selected from -C₁₋₂ alkylene-N(R^{a})-C₁₋₆ alkyl, wherein the substitutable carbon atoms of R^{2a} are each independently and optionally substituted by one or more substituents each independently selected from R^{g}; and/or the substitutable nitrogen atoms on the ring of R^{2a} are each independently and optionally substituted by one or more substituents each independently selected from R^{h}; and/or
each R^{B} is independently selected from H, D, halogen (such as F, Cl, Br), -OH, - CN, -NH₂, -NH(C₁₋₄ alkyl), -N(C₁₋₄ alkyl)(C₁₋₄ alkyl), C₁₋₄ alkyl, and C₁₋₄ haloalkyl; and/or
R^{h} is selected from H, D, C₁₋₄ alkyl, and C₁₋₄ haloalkyl; and/or
R^{a} and R^{b} are each independently selected from H and C₁₋₄ alkyl; and optionally, the C₁₋₄ alkyl is substituted by one or more substituents selected from halogen.

13. The compound or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof according to any one of claims 1-5, wherein the compound has a structure represented by formula (VI-1):

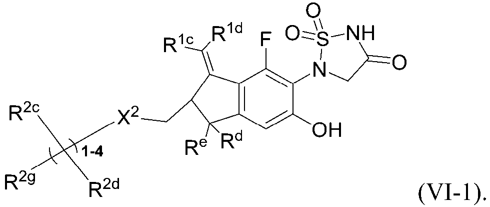

14. The compound or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof according to any one of claims 1-13, wherein the compound has a structure represented as follows:

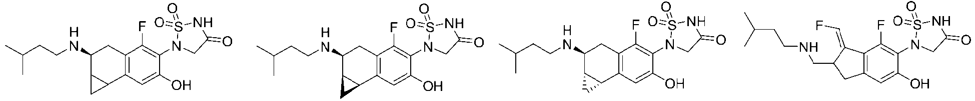

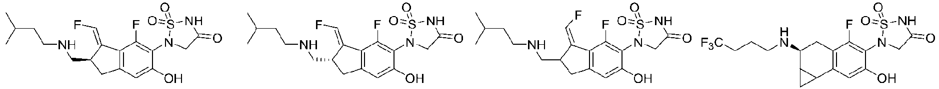

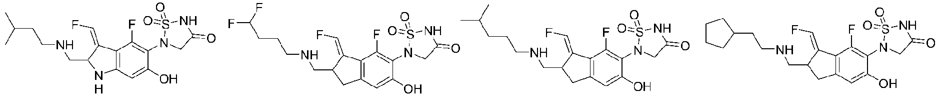

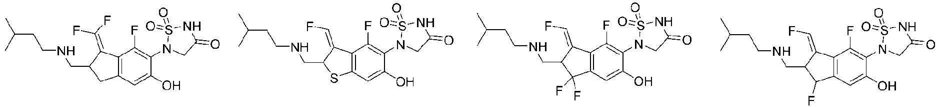

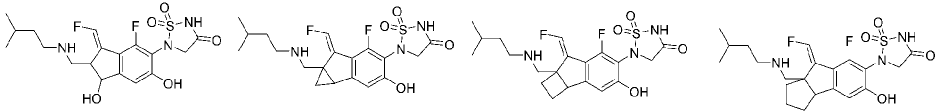

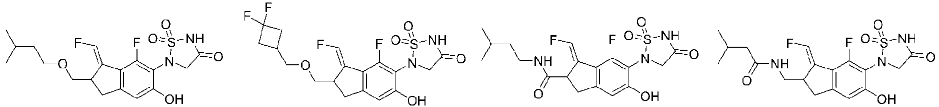

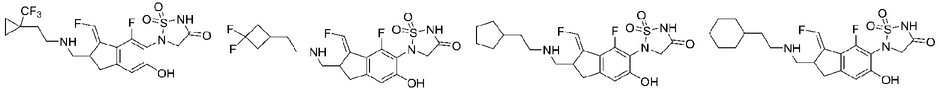

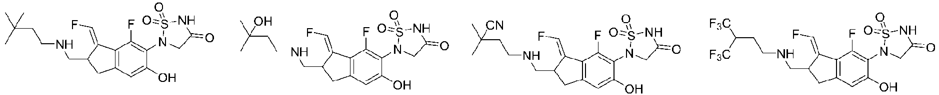

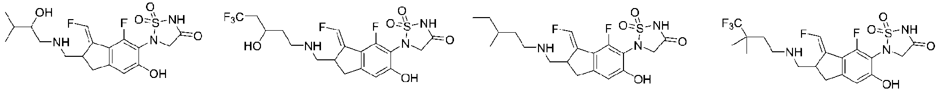

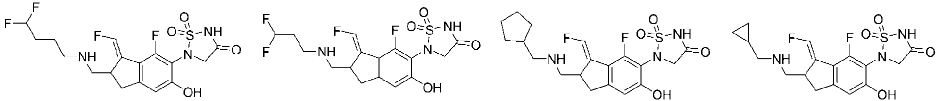

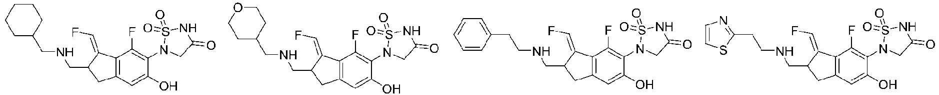

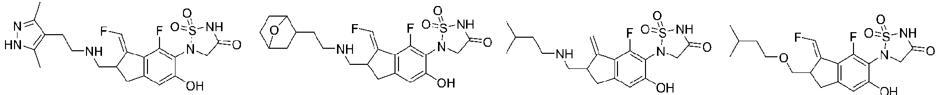

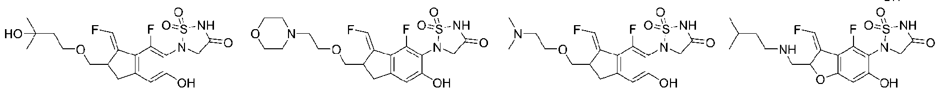

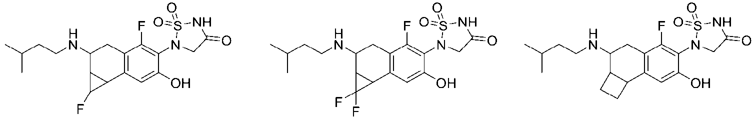

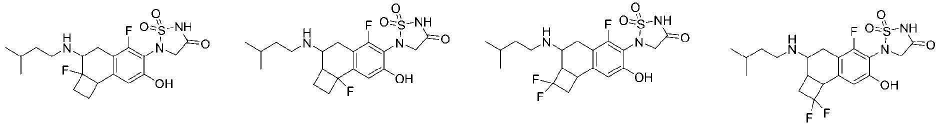

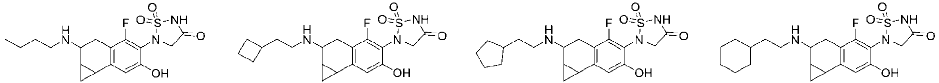

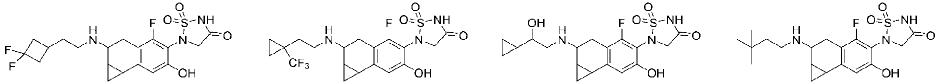

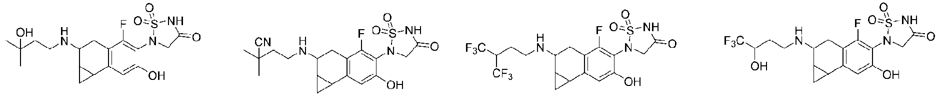

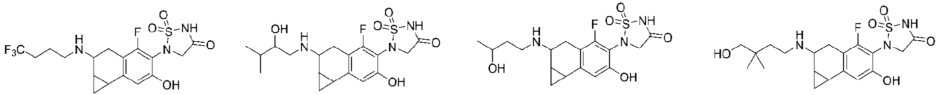

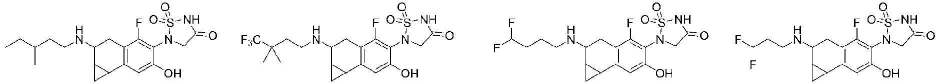

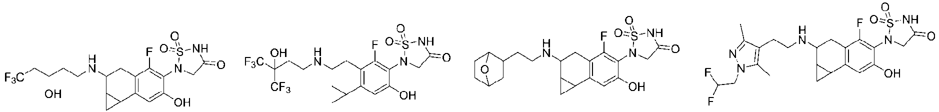

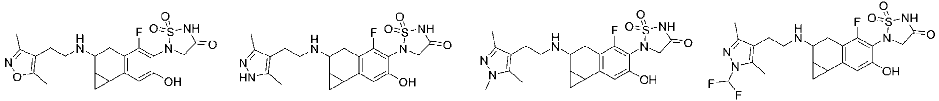

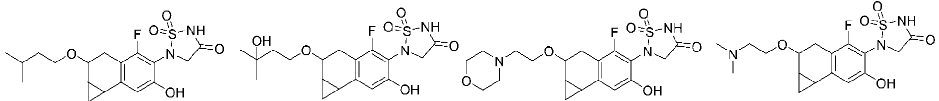

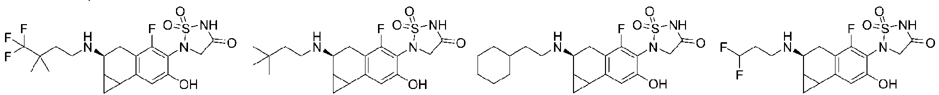

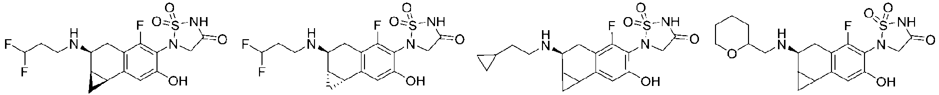

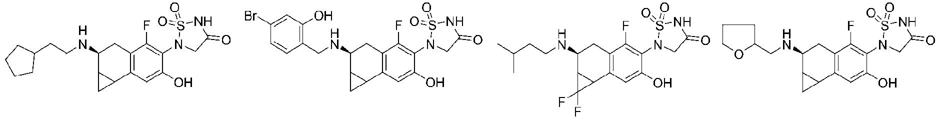

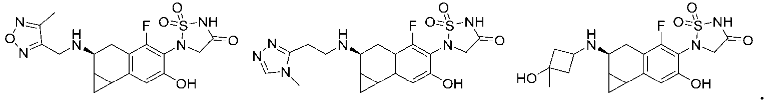

15. A pharmaceutical composition, comprising the compound or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof according to any one of claims 1-14, and at least one pharmaceutically acceptable carrier.

16. The compound or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof according to any one of claims 1-14, or the pharmaceutical composition according to claim 15, wherein it is used for use in treatment or prevention of a disease or disorder related to PTPN2.

17. Use of the compound or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof according to any one of claims 1-14, or the pharmaceutical composition according to claim 15, in manufacture of a medicament, wherein the medicament is used for treatment or prevention of a disease or disorder related to PTPN2.

18. A method of treating or preventing a disease or disorder related to PTPN2, comprising administering to a person in need a therapeutically effective amount of the compound or the enantiomer, diastereomer, racemate, tautomer, stereoisomer, geometric isomer, N-oxide, metabolite or pharmaceutically acceptable salt, ester, solvate, hydrate, isotope-labeled compound or prodrug thereof according to any one of claims 1-14, or the pharmaceutical composition according to claim 15.

19. The use according to claim 17, or the method according to claim 18, wherein the disease or disorder related to PTPN2 comprises cancer, type 2 diabetes, metabolic syndrome, obesity or metabolic diseases; preferably, the cancer comprises carcinoma, sarcoma, adenocarcinoma, lymphoma, leukemia, and melanoma; preferably, the cancer comprises solid and lymphatic cancer, kidney cancer, breast cancer, lung cancer, bladder cancer, colon cancer, ovarian cancer, prostate cancer, pancreatic cancer, stomach cancer, brain cancer, head and neck cancer, skin cancer, uterine cancer, testicular cancer, glioma, esophageal cancer, liver cancer (comprising liver tumors), lymphoma (comprising B-cell acute lymphoblastic lymphoma, non-Hodgkin's lymphoma (non-Hodgkin's lymphomas, such as Burkitt's lymphoma, small cell lymphoma, and large cell lymphoma), Hodgkin's lymphoma), leukemia (comprising AML, ALL, and CML) and/or multiple myeloma; and preferably, the cancer comprises lung cancer, breast cancer, ovarian cancer, leukemia, lymphoma, melanoma, pancreatic cancer, sarcoma, bladder cancer, bone cancer, brain cancer, cervical cancer, colon cancer, esophageal cancer, stomach cancer, liver cancer, head and neck cancer, kidney cancer, myeloma, thyroid cancer, prostate cancer, metastatic cancer or carcinoma.
